(19) Europäisches Patentamt
European Patent Office
Office européen des brevets

(11) Veröffentlichungsnummer: **0 019 078**
**B1**

# (12) EUROPÄISCHE PATENTSCHRIFT

(45) Veröffentlichungstag der Patentschrift: **11.06.86**

(21) Anmeldenummer: **80101864.9**

(22) Anmeldetag: **08.04.80**

(51) Int. Cl.⁴: **C 07 C 93/06**, C 07 C 149/24, C 07 C 103/44, C 07 C 103/82, C 07 C 93/187, C 07 C 93/20, C 07 C 101/12, C 07 C 103/54, C 07 C 143/78, D 06 L 3/12, C 11 D 3/42 // C07D295/08, C07D295/12, C07F9/40, C11D1/62, D06M13/46

(54) **Distyrylbenzole, Verfahren zu deren Herstellung, deren Verwendung beim optischen Aufhellen organischer Materialien, sowie Waschmittel, Textilbehandlungsmittel und Wäschenachbehandlungsmittel, die diese Distyrylbenzole enthalten.**

(30) Priorität: **11.04.79 CH 3479/79**
**26.06.79 CH 5951/79**

(43) Veröffentlichungstag der Anmeldung:
**26.11.80 Patentblatt 80/24**

(45) Bekanntmachung des Hinweises auf die Patenterteilung:
**11.06.86 Patentblatt 86/24**

(84) Benannte Vertragsstaaten:
**CH DE FR GB LI**

(56) Entgegenhaltungen:
**GB - A - 913 735**
**GB - A - 1 351 489**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Weber, Kurt, Dr., Rennweg 98, CH-4052 Basel (CH)**
Erfinder: **Meyer, Hans Rudolf, Dr., Bollwerkstrasse 102, CH-4102 Binningen (CH)**

(74) Vertreter: **Zumstein, Fritz jun., Dr. et al, Dr. F. Zumstein sen. Dr. E. Assmann Dr. R. Koenigsberger Dipl.-Ing. F. Klingseisen Dr. F. Zumstein jun. Bräuhausstrasse 4, D-8000 München 2 (DE)**

EP 0 019 078 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue Distyrylbenzole, Verfahren zu deren Herstellung, deren Verwendung zum optischen Aufhellen von organischen Materialien, sowie Waschmittel, Textilbehandlungsmittel und Wäschenachbehandlungsmittel, die diese Distyrylbenzole enthalten.

Es sind aus der Literatur z.B. aus den DE-OS 19 23 267 und 27 21 730, GB-PS 913 735 und GB-PS 1 351 489 in den Benzolkernen anionisch substituierte wasserlösliche 1,4-Distyrylbenzole bekannt, welche aber mit kationaktiven Textilhilfsmitteln nicht verträglich sind. Ferner sind aus der DE-OS 23 64 396 und der GB-PS 1 351 489 wasserlösliche kationisch substituierte 1,4-Distyrylbenzole bekannt, die aber trotz ihres kationischen Charakters zu unbefriedigenden Ergebnissen führen, wenn sie in kationischem Milieu zur Anwendung gelangen.

Es wurden nun Distyrylbenzole gefunden, welche diese Nachteile nicht aufweisen.

Die Erfindung betrifft daher neue Distyrylbenzole der Formel

(1) 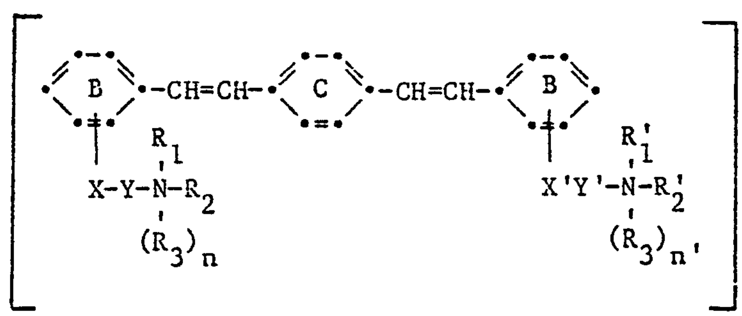 $(n+n')^{\oplus}$ $(n+n')A^{\ominus}$

worin X und X' unabhängig voneinander die direkte Bindung, Sauerstoff, Schwefel -0-C -Alkylen-CON($R_4$)-, -0-C $_3$-Alkylen-COO- oder -OCO-, Y und Y' unabhängig voneinander $C_{1-20}$-Alkylen oder durch eine Hydroxylgruppe substituiertes $C_{1-20}$-Alkylen, $R_1$ und $R'_1$ unabhängig voneinander unsubstituiertes oder durch Halogen, Cyano, Hydroxy, Alkoxy, Phenyl oder Alkoxycarbonyl mit 1 bis 4 C-Atomen substituiertes $C_{1-8}$-Alkyl, $C_{3-4}$-Alkenyl oder $R_1$ bzw. $R'_1$ zusammen mit $R_2$ bzw. $R_2'$ und dem N-Atom, an das sie gebunden sind, einen 5- bis 7-gliedrigen heterocyclischen Ring, $R_2$ und $R'_2$ unabhängig voneinander unsubstituiertes oder durch Halogen,Cyano, Hydroxy, Alkoxy, Phenyl oder Alkoxycarbonyl mit 1 bis 4 C-Atomen substituiertes $C_{1-8}$-Alkyl, $C_{3-4}$-Alkenyloder $R_2$ bzw. $R_2'$ zusammenmitR bzw. $R_1'$ und dem N-Atom, andas sie gebundensind,einen 5-bis7-gliedrigen heterocyclischen Ring, $R_3$ Wasserstoff, unsubstituiertes oder substituiertes $C_{1-4}$-Alkyl oder $C_{3-4}$-Alkenyl, $R_1$, $R_2$ und $R_3$ zusammen mit dem N-Atom,an das sie gebunden sind,bzw. $R_1'$, $R_2'$ und $R_3$ zusammen mit dem N-Atom, an das sie gebunden sind, auch den Pyridin-oder Picolinring, $R_4$ Wasserstoff oder unsubstituiertes oder durch Cyano, Carbamoyl, Carbalkoxy, Hydroxy, Halogen oder Alkoxy mit 1 bis 4 C-Atomen substituiertes $C_{1-6}$-Alkyl, $A^{\ominus}$ ein farbloses Anion und n und n' unabhängig voneinander die Zahl 0 oder 1 bedeuten, wobei die Benzolkarne B und C auch nicht-chromophor substituiert sein können, und worin die Gruppierungen

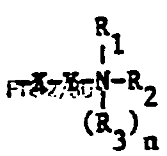

sowie

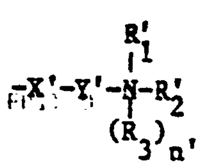

im Falle, dass die Ringe
B und C keine nicht-chromophoren Substituenten tragen, auch beide jeweils

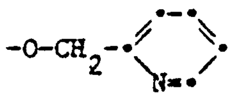

oder beide jeweils

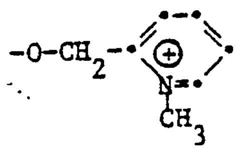

in den ortho-Stellungen zu den beiden Gruppen -CH=CH- sein können.

Die Alkylenreste Y und Y' können verschiedenartig, geradkettig oder verzweigt sein und vorzugsweise 1 bis 12 und besonders 1 bis 6 C-Atome aufweisen.

Geeignete Alkylreste $R_1$, $R_1$, $R_2$ und $R'_2$ sind vorzugsweise solche mit 1 bis 4 C-Atomen.

Als 5- bis 7-gliedriger heterocyclischer Ring, gebildet durch die Reste $R_1$ und $R_2$ sowie $R'_1$ und $R'_2$ zusammen mit dem N-Atom, an das sie gebunden sind, kommt z.B. ein Piperidin-, Pyrrolidin-, Hexamethylenimin-. Pyridin-, Triazol-, Imidazol- oder Morpholinring, die durch Alkylgruppen mit 1 bis 4 C-Atomen substituiert sein können, in Betracht.

Ein Alkylrest $R_4$ weist vorzugsweise 1 bis 4 C-Atomen auf.

Als nicht-chromophore Substituenten der Benzolringe B und C seien beispielsweise erwähnt: Halogenatome; Alkylgruppen; Cycloalkylgruppen; Alkenylgruppen, Alkoxygruppen, Alkenyloxygruppen oder Sulfonylgruppen, z.B. Alkyl- oder Phenylsulfonylgruppen, Carbalkoxygruppen, Carbamoylgruppen und Sulfamoylgrupoen, sowie die Ergänzung zu einem carbocyclischen 5- oder 6-gliedrigen Ring.

Im Rahmen der erfindungsgemässen Distyrylbenzole verdienen eine besondere Erwähnung solche der Formel

(2)

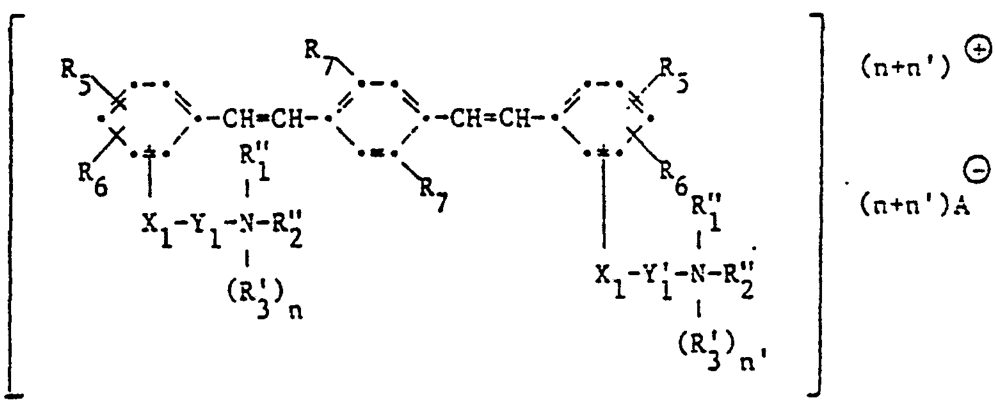

worin $X_1$ die direkte Bindung, Sauerstoff, Schwefel, $-0-C_{1-3}$-Alkylen-CONH-, $-0-C_{1-3}$-Alkylen-COO- oder -OCO-, $Y_1$ und $Y_1'$ unabhängig voneinander $C_{1-4}$-Alkylen oder Hydroxypropylen, $R_1''$ und $R'_2'$ unabhängig voneinander $C_{1-4}$-Alkyl, oder $R_1''$ und $R'_2'$ zusammen mit dem N-Atom, an das sie gebunden sind, einen Pyrrolidin-, Piperidin-, Hexamethylenimin- oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_{2-4}$-Hydroxyalkyl oder $C_{2-4}$-Cyanoalkyl, $R'_1'$, $R_2''$ und $R_3'$ zusammen mit dem N-Atom, an das sie gebunden sind, auch den Pyridin- oder Picolinring, $R_5$ Wasserstoff, Chlor, $C_{1-4}$-Alkyl, $C_{3-4}$-Alkenyl, $C_{1-3}$-Alkoxy oder zusammen mit $R_6$ eine Trimethylen- oder Tetramethylengruppe, $R_6$ Wasserstoff, Chlor, $C_{1-4}$-Alkyl, $C_{1-3}$-Alkoxy oder zusammen mit $R_5$ eineTrimethylen- oder Tetramethylengruppe, $R_7$ Wasserstoff, Chlor oder Methyl, n und n' unabhängig voneinander die Zahl 0 oder 1 und $A^{\ominus}$ ein farbloses Anion bedeuten.

Bevorzugte Distyrylbenzole der Formeln (1), (2) bzw. (5) sind solche,

- welche symmetrisch sind, d.h. jene worin X=X'; Y=Y'; Y =Y'; $R_1=R_1'$, $R_2=R'_2$ und n=n' ist
- welche quaterniert sind, d.h. jene, worin n und n' gleich 1 sind, und - worin $R_3$ und $R_3'$ $C_{1-3}$-Alkyl bedeutet.

Im Rahmen der bevorzugten Distyrylbenzole der Formeln (1) und (2) sind von Interesse solche der Formel

(3) 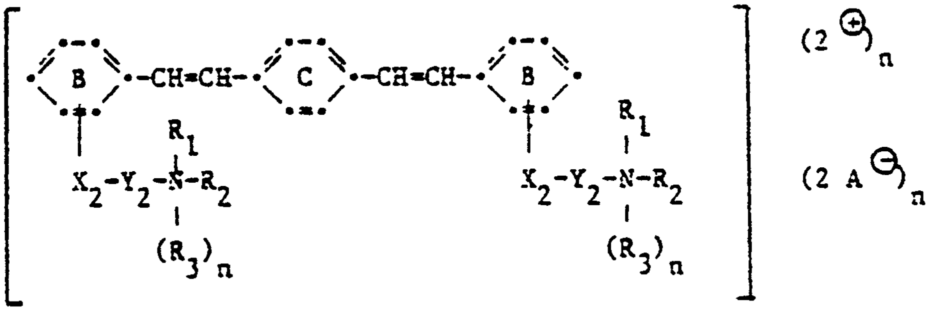

worin $X_2$ Sauerstoff, Schwefel oder -OCO-, $Y_2$$C_{1-20}$-Alkylen oder durch eine Hydroxylgruppe substituiertes $C_{1-20}$-Alkylen, $R_1$ unsubstituiertes oder durch Halogen, Cyano, Hydroxy, Alkoxy, Phenyl oder Alkoxycarbonyl mit 1 bis 4 C-Atomen substituiertes $C_{1-8}$-Alkyl, $C_{3-4}$-Alkanyl oder $R_1$ zusammen mit $R_2$ und dem N-Atom, an das sie gebunden sind, einen 5- bis 7-gliedrigen heterocyclischen Ring, $R_2$ unsubstituiertes oder durch Halogen, Cyano, Hydroxy, Alkoxy, Phenyl oder Alkoxycarbomyl mit 1 bis 4 C-Atomen substituiertes $C_{1-8}$-Alkyl, $C_{3-4}$-Alkenyl oder $R_2$ zusammenmit heterocyclischen Ring, $R_3$ Wasserstoff, unsubstituiertes oder substituiertes $C_{1-4}$-AlkyloderC$_{3-4}$-Alkenyl, $R_1$, $R_2$und $R_3$ zusammen mitdem N-Atom, an das sie gebunden sind, auch den Pyridin- oder Picolinring, $R_4$ Wasserstoff oder unsubstituiertes oder durch Cyano, Carbamoyl, Carbalkoxy, Hydroxy, Halogen oder Alkoxy mit 1 bis 4 C-Atomen substituiertes $C_{1-6}$-Alkyl, n die Zahl 0 oder 1 und A⊖ ein farbloses Anion bedeuten, wobei die Benzolkerne B und C auch nicht-chromophor substituiert sein können, solche der Formel

(4) 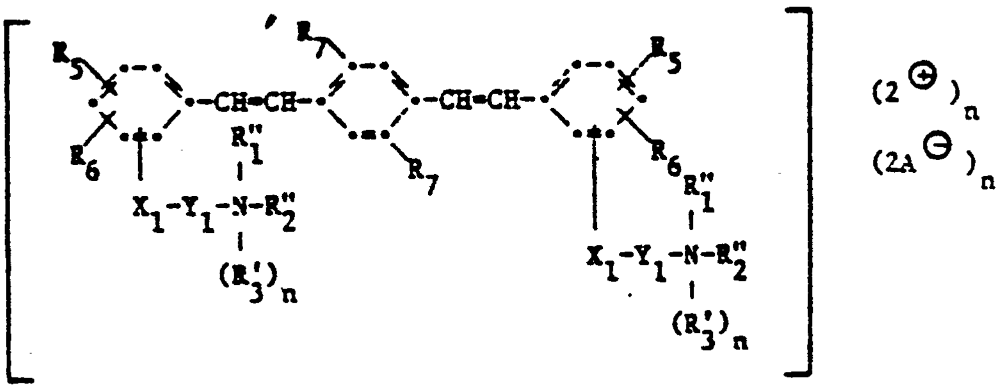

worin $X_1$ die direkte Bindung, Sauerstoff, Schwefel, -O-$C_{1-3}$-Alkylen-CONH-, -O-$C_{1-3}$-Alkylen-COO- oder -OCO-, $Y_1$ $C_{1-4}$-Alkylen oder Hydroxypropylen, $R''_1$ und $R'_2$' unabhängig voneinander $C_{1-4}$-Alkyl oder $R'_1$' und $R_2$'' mit dem N-Atom, an das sie gebunden sind, einen Pyrrolidin-, Piperidin-, Hexamethylenimin- oderMorpholinring, $R_3$' Wasserstoff, $C_{1-4}$-Alkyl, $C_{3-4}$-Al-kenyl, $C_{1-3}$-Alkoxycarbonylmethyl, Benzyl, $C_{2-4}$-Hydroxyalkyl oder $C_{2-4}$-Cyanoalkyl, $R'_1$', $R''_2$ und $R_3$' zusammen mit dem N-Atom,an das sie gebunden sind, auch den Pyridin-oder Picolinring, $R_5$ Wasserstoff, Chlor, $C_{1-4}$-Alkyl, $C_{3-4}$-Alkenyl, $C_{1-3}$-Alkoxy oder zusammen mit $R_6$ eine Trimethylen- oder Tetramethylengruppe, $R_6$ Wasserstoff, Chlor, $C_{1-4}$-Alkyl, $C_{1-3}$-Alkoxy oder zusammen mit $R_5$ eine Trimethylen- oder Tetrammethylengruppe, $R_7$ Wasserstoff, Chlor oder Methyl, n die Zahl 0 oder 1 und A⊖ ein farbloses Anion bedeuten, solche der Formel

(5)

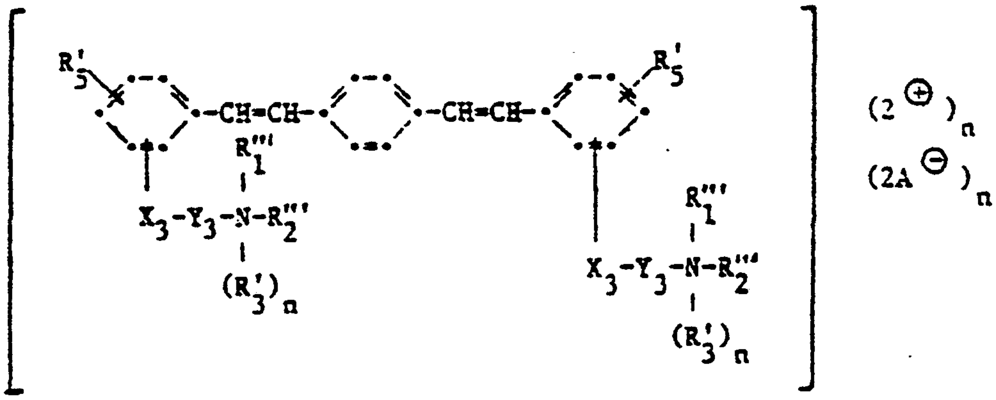

worin $X_3$ die direkte Bindung, Sauerstoff, Schwefel oder -OCO-, $Y_3$ $C_{1-4}$-Alkylen, $R_1''$ und $R_2''$ unabhängig voneinander $C_{1-4}$-Alkyloder $R_1''$ und $R_2''$ zusammen mit dem N-Atom, an das sie gebunden sind einen Pyrrolidin-, Piperidin- oder Morpholinring, $R_3'$ Wasserstoff, $C_{1-4}$-Alkyl, $C_{3-4}$-Alkenyl, $C_{1-3}$ Alkoxycarbonylmethyl, Benzyl, $C_{2-4}$-Hydroxyalkyloder $C_{2-4}$-Cyanoalkyl $R_1''$, $R_2''$ und $R'_3$ zusammen mit dem N-Atom, andas sie gebunden sind, auch den Pyridinring $R_5'$ Wasserstoff Chlor, $C_{1-4}$-Alkyl oder $C_{1-3}$-Alkoxy, n die Zahl 0 oder 1 und A ⊖ ein farbloses Anion bedeuten, und solche der Formel

(6)

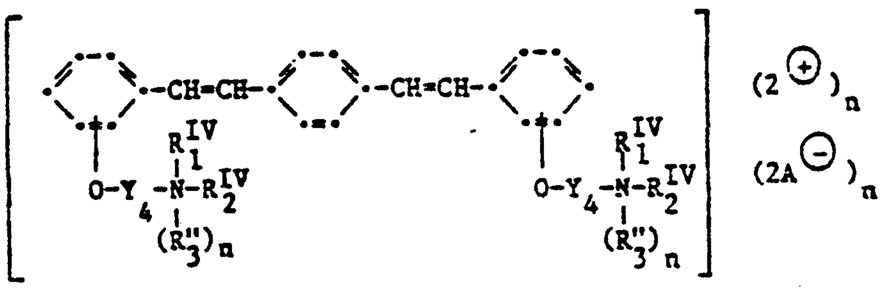

worin $Y_4$ $C_{2-4}$-Alkylen, R $C_{1-3}$-Alkyl oder $R^{IV}_1$ zusammen mit$R^{IV}_2$ und dem N-Atom, am das sie gebunden sind, einen Pyrrolidin-, Piperidin-, Hexamethylenimin- oder Morpholinring, $R^{IV}_2$ $C_{1-3}$-Alkyl oder $R^{IV}_2$ zusammen mit $R^{IV}_1$ und dem N-Atom, an das sie gebunden sind, einen Pyrrolidin-, Piperidin-, Hexamethylenimin- oder Morpholinring, $R_3''$ Wasserstoff, oder $C_{1-3}$-Alkyl, n die Zahl 0 oder 1 und A ⊖ ein farbloses Anion bedeuten.

Von Interesse sind die Distyrylbenzole der Formel

(7)

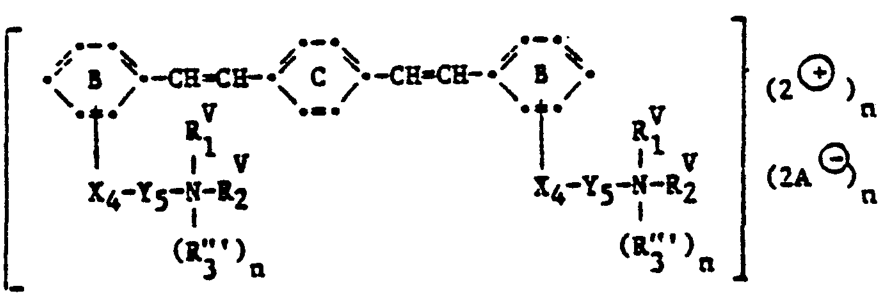

worin $X_4$ Sauerstoff, Schwefel oder-OCO-, $Y_5C_{2\ 20}$-Alkylen, $R_1$ unsubstituiertes oder durch Halogen, Cyano, Hydroxy, Alkoxy, Phenyl oder Alkoxycarbonyl mit 1 bis 4 C-Atomen substituiertes $C_{1-8}$-Alkyl, $C_{3-4}$-Alkenyl oder $R^V_1$

zusammen mit $R^V_2$ und dem N-Atom, an das sie gebundensind, einen 5- bis 7-gliedrigen heterocyclischen Ring, $R^V_2$ unsubstituiertes oder durch Halogen, Cyano, Hydroxy, Alkoxy, Phenyl oder Alkoxycarbonyl mit 1 bis 4 C-Atomen substituiertes $C_{1-8}$-Alkyl, $C_{3-4}$-Alkenyl oder $R^V_2$ zusammen mit $R_1$ und dem N-Atom, an das sie gebunden sind, einen 5- bis 7-gliedrigen heterocyclischen Ring, $R'_3$'' Wasserstoff oder unsubstituiertes oder substituierres $C_{1-4}$-Alkyl, $R_4$ Wasserstoff oder unsubstituiertes oder durch Cyano, Carbamoyl, Carbalkoxy, Hydroxy, Halogen oder Alkoxy mit 1 bis 4 C-Atomen substituiertes $C_{1-6}$-Alkyl, A ⊖ ein farbloses Anion und n die Zahl 0 oder 1 bedeuten, wobei die Benzolkerne B und C auch nicht-chromophor substituiert sein können, und solche der Formel

(3) 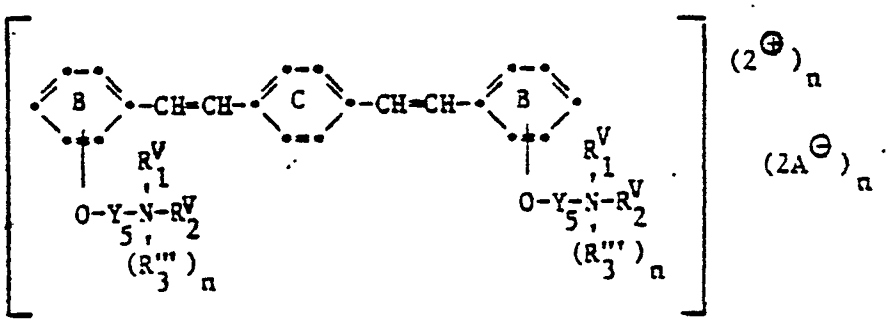

worin $Y_5$ $C_{2-20}$-Alkylen, $R^V_1$ umsubstituiertes oder durch Halogen, Cyano, Hydroxy, Alkoxy, Phenyl oder Alkoxycarbonyl mit 1 bis 4 C-Atomen substituiertes $C_{1-8}$-Alkyl, $C_{3-4}$-Alkenyl oder zusammen mit $R^V_2$ und dem N-Atom, an das sie gebunden sind, einen 5- bis 7-gliedrigen heterocyclischen Ring, $R^V_2$ unsubstituiertes oder durch Halogen, Cyano, Hydroxy, Alkoxy, Phenyl oder Alkoxycarbonyl mit 1 bis 4 C-Atomen substituiertes $C_{1-8}$-Alkyl, $C_{3-4}$-Alkenyl oder zusammen mit $R^V_1$ und dem N-Atom, an das sie gebunden sind, einen 5- bis 7-gliedrigen heterocvclischen Ring, $R'_3$'' Wasserstoff oder unsubstituiertes oder substituiertes $C_{1-4}$-Alkyl, A ⊖ ein farbloses Anion und n die Zahl 0 oder 1 bedeuten, Wobei die Benzolkerne B und C auch nicht-chromophor substituiert sein können.

Von besonderem Interesse sind die Distyrylbenzole der Formel

(9) 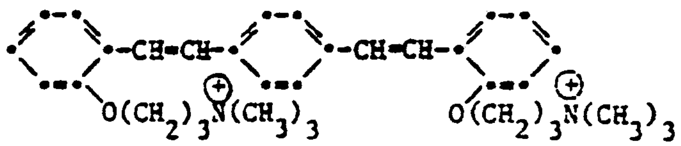 $2\ CH_3OSO_3^{\ominus}$

(10) 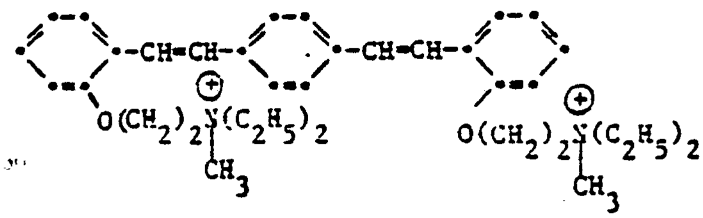 $2\ CH_3OSO_3^{\ominus}$

und

(11) 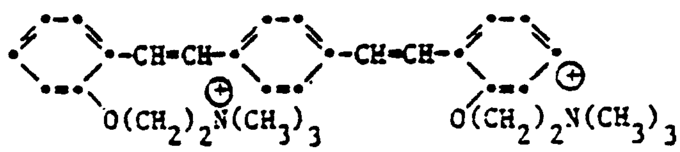 $2\ CH_3OSO_3^{\ominus}$

Die Herstellung der Distyrylbenzole der Formel (1) worin X die direkte Bildung Sauerstoff oder Schwefel, und i und n' die Zahl 0 bedeuten, erfolgt in an sich bekannter Weise, indem man im Molekularverhältnis 1:2 eine Verbindung der Formel

(12) 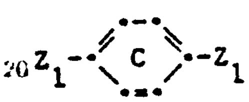

mit einer der beiden oder einer Mischung der beiden Verbindungen der Formeln

(13) 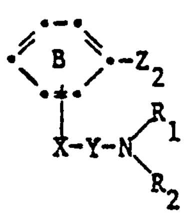 und (14) 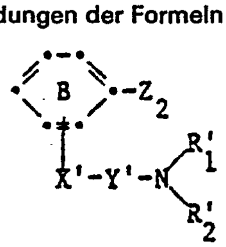

in Gegenwart einer starken Base umsetzt, in welchen Formeln die Benzolkerne B und C auch nicht-chromophor substituiert sein können, X und X' die direkte Bindung, Sauerstoff oder Schwefel und Y, Y', $R_1$, $R'_1$, $R_2$ und $R_2'$ die oben angegebene Bedeutung haben und eines der Symbole $Z_1$ und $Z_2$ eine OCH-Gruppe und das andere eine Gruppierung der Formel

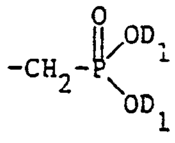 , 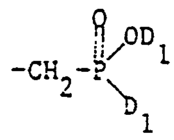

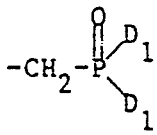 oder 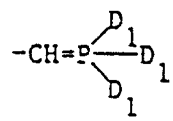

bedeutet, worin $D_1$ einen unsubstituierten oder substituierten Alkyl-, Aryl-, Cycloalkyl oder Aralkylrest darstellt und anschliessend die erhaltene Verbindung der Formel

(15) 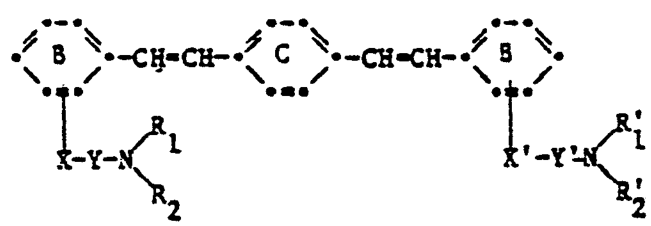

worin X, X', Y, Y', $R_1$, $R'_1$, $R_2$ und $R_2'$ die oben angegebene Bedeutung haben und die Benzolkerne B und C auch nicht-chromophor substituiert sein können, in an sich bekannter Weise mit 1 bis 2 Moläquivalanten eines Alkylierungsmittels bzw. einer Säure der Formel $R_3$-A worin $R_3$ und A die oben angegebene Bedeutung haben quaterniert bzw. protoniert. $Z_2$ ist vorzugsweise die OCH-Gruppe.

Man führt die Kondensation vorteilhaft in indifferenten Lösungsmitteln durch. Als Beispiele hierfür seien Kohlenwasserstoffe wie Toluol und Xylol oder Alkohole wie Methanol, Aethanol, Isopropanol, Butanol, Glykole, Glykoläther wie 2-Methoxyäthanol, Hexanole, Cyclohexanol und Cyclooctanol, ferner Aether wie Diisopropyläther, Tetrahydrofuran und Dioxan sowie Dimethylsulfoxid, Formamid und N-Methylpyrrolidon genannt. Besonders geeignet sind polare organische Lösungsmittel wie Dimethylformamid und Dimethylsulfoxid. Auch in wässriger Lösung lassen sich einige der Umsetzungen durchführen.

Die Temperatur, bei welcher die Umsetzung durchgeführt wird, kann in weiten Grenzen schwanken. Sie wird bestimmt:

α) durch die Beständigkeit des verwendeten Lösungsmittels gegenüber den Reaktionsteilnehmern, insbesondere gegenüber den stark basischen Alkaliverbindungen,

β) durch die Reaktivität der Kondensationspartner und

γ) durch die Wirksamkeit der Kombination Lösungsmittel-Base als Kondensationsmittel.

In der Praxis kommen hiernach im allgemeinen Temperaturen zwischen etwa 10 und 100°C in Betracht. Wenn Dimethylformamid als Lösungsmittel verwendet wird, liegt der Temperatur-Vorzugsbereich bei 20 bis 60°C.

Als stark basische Alkaliverbindungen kommen vor allem die Hydroxyde, Amide und Alkoholate (vorzugsweise 1 bis 4 Kohlenstoffatome enthaltender Alkohole) der Alkalimetalle in Betracht, wobei aus ökonomischen Gründen solche des Lithiums, Natriums und Kaliums von vorwiegendem Interesse sind. Es können jedoch grundsätzlich und in besonderen Fällen auch Alkalisulfide und -carbonate, Arylalkaliverbindungen wie z.B. Phenyl-lithium oder stark basische Amine (einschliesslich Ammoniumbasen) z.B. Trialkylammoniumhydroxide, mit Erfolg verwender werden.

Die Ausgangsprodukte der Formeln (13) und (14), worin $Z_2$ eine Aldehydgruppe bedeutet, erhält man z.B. durch Alkylierung von Hydroxybenzaldehyden mit Dialkylaminoalkylchloriden in Gegenwart basischer Alkali- oder Erdalkaliverbindungen wie z.B. Natriumalkoholate (siehe Beispiel 3), Kaliumcarbonat, Natriumcarbonat, Calciumcarbonat, Magnesiumoxid, Natriumhydrid, Kaliumhydroxid oder Natriumhydroxid in indifferenten organischen Lösungsmitteln wie Alkoholen, Dialkylamiden aliphatischer Carbonsäuren. Besonders geeignet sind

wasserfreie Lösungsmittel, welche auch in der Folgestufe, d.h. zur Herstellung der Verbindungen der Formel (15) brauchbar sind, wie z.B. Dimethylformamid, wobei die Isolierung der Verbindungen der Formeln (13) und (14) nicht mehr notwendig ist.

Distyrylbenzole der Formel

(16) 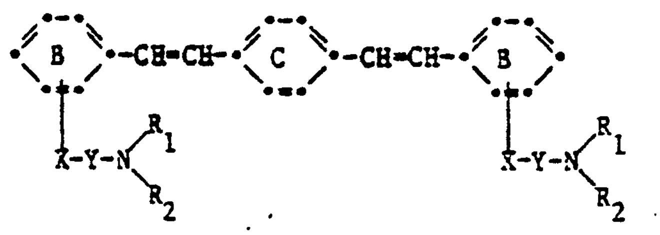

worin X -O-$C_{1-3}$-Alkylen-CON($R_4$)- oder -O-C $_3$-alkylen-COO- bedeutet, werden in an sich bekannter Weise aus den entsprechenden Säuren (vgl. z.B. DE-OS 19 23 267 und 20 39 993) hergestellt, indem man in an sich bekannter Weise die Säurehalogenide herstellt und diese mit einer Verbindung der Formel HN($R_4$)-Y-N($R_1$)($R_2$) oder HO-Y-N($R_1$)($R_2$)umsetzt.

Distyrylbenzole der Formel

(17) 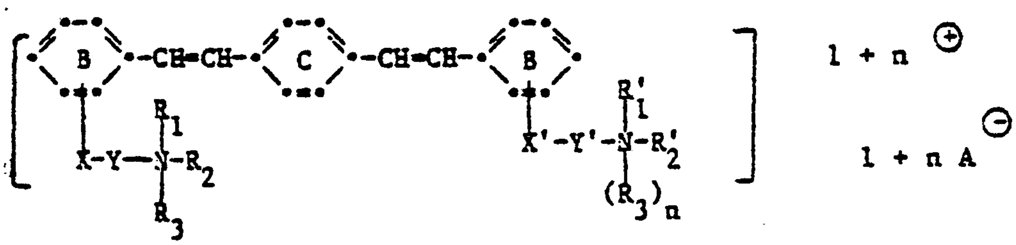

worin X und X' die direkte Bindung, Sauerstoff, Schwefel, -OCO-, -O-$C_{1-3}$-Alkylen-CON($R_4$)- oder -O-C $_{1-3}$-Alkylen-COO-, Y und Y' $C_{1-20}$-Alkyle, $R_1$ und $R'_1$ unsubstituiertes oder durch Halogen, Cyano, Hydroxy, Alkoxy, Phenyl oder Alkoxycarbonyl mit 1 bis 4 C-Atomen substituiertes $C_{1-8}$-Alkyl, $C_{3-4}$-Alkenyl oder zusammen mit $R_2$ bzw. $R_2'$ und dem N-Atom, an das sie gebunden sind, einen 5- oder 7-gliedrigen heterocyclischen Ring, $R_2$ und $R'_2$ unsubstituiertes oder durch Halogen, Cyano, Hydroxy, Alkoxy, Phenyl oder Alkoxycarbonyl mit 1 bis 4 C-Atomen substituiertes $C_{1-8}$-Alkyl, $C_{3-4}$-Alkenyl oder zusammen mit $R_1$ bzw. R' und dem N-Atom an das sie gebunden sind, einen 5- oder 7-gliedrigen heterocyclischen Ring, $R_3$ Wasserstoff oder unsubstituiertes oder substituiertes $C_{1-4}$-Alkyl oder$C_{3-4}$-Alkenyl, A⊖ ein farbloses Anion und n die Zahl 0 oder 1 bedeuten, wobei die Benzolkerne B und C auch nicht-chromophor substituiert sein können, werden dadurch hergestellt, dass man in an sich bekannter Weise ein Moläquivalent eines Distyrylbenzols der Formel (15) mit 1 bis 2 Moläquivalenten eines Alkylierungsmittels bzw. einer Säure der Formel $R_3$-A worin $R_3$ und A die oben angegebene Bedeutung haben, quaterniert bzw. protoniert.

Als Quaternierungs- bzw. Protonierungsmittel $R_3$-A sind beispielsweise folgende Verbindungen geeignet: Alkylhalogenide wie Methyljodid, Aethyljodid, Aethylbromid, Butylbromid oder Benzylchlorid, Dialkylsulfate wie Dimethyl oder Diäthylsulfat, Bulfonsäureester wie Toluoloder Benzolsulfonsäuremethyl- oder äthylester, Alkylenoxide wie Aethylen- oder Propylenoxide oder Epichlorhydrin, die Verbindungen der Formel

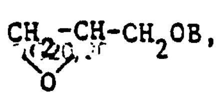

wobei B für Methyl, Aethyl, Propyl, Butyl oder Phenyl steht. Phosphite oder Phosphonate der Formel

(18) 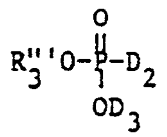

worin $R_3''$ Alkyl mit 1-4 C-Atomen, $D_2$ Wasserstoff oder unsubstituiertes oder durch Hydroxy, Cyano, Alkylcarbonyloxy oder Alkoxycarbonyl mit jeweils 1 bis 4 C-Atomen im Alkylteil substituiertes Alkyl und $D_3$ Alkyl mit 1 bis 4 C-Atomen bedeuten.

Die Quaternierung der Verbindungen der Formel (15) mit Alkylhalogeniden, Dialkylsulfaten oder Sulfosäureester zu den Verbindungen der Formel (17) wird zweckmässigerweise in einem gegenüber dem Alkylierungsmittel inerten Lösungsmittel vorgenommen. Geeignete Lösungsmittel sind z.B. Kohlenwasserstoffe wie Benzol,Toluol und Xylol, halogenierte aliphatische oder aromatische Kohlenwasserstoffe wie Chloroform, Aethylenchlorid, Chlorbenzol und Dichlorbenzol, Alkohole wie Aethanol, Butanol- Aethylenglykol und Aethylenglykolmonomethyläther, Aether wie Aethylenglykoldimethyläther und Dioxan oder Amide wie Dimethylfomamid und N-Methylpyrrolidon. Zuweilen erweist sich auch vorteilhaft, das Quaternierungsmittel als Lösungsmittel zu verwenden. Die Quaternierung mit den genannten Alkylierungsmitteln erfolgt vorteilhaft bei Temperaturen zwischen 0 und 180°C, vorzugsweise bei 30 bis 140°C.

Die Quaternierung der Verbindung der Formel(15) zu den Verbindungen der Formel 17 mit Alkylenoxiden, Epichlorhydrin sowie dessen Derivaten der Formel

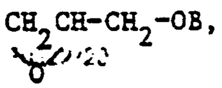

in der B die genannten Bedeutungen hat,

wird bei den genannten Temperaturen in saurem Medium vorteilhaft in Gegenwart einer organischen Säure wie Ameisensäure, Essigsäure, Propionsäure oder Milchsäure durchgeführt, jedoch können auch anorganische Säuren wie Schwefelsäure, Phosphorsäure oder Halogenwasserstoffsäuren dafür verwendet werden. Diese anorganischen Säuren könnan in konzentrierter, handelsüblicher Form als verdünnte wässrige Lösungen oder in Mischung mit den genannten organischen Lösungsmitteln, gegebenenfalls unter Zusatz von Wasser, verwendet werden. Erfolgt die Umsetzung in Gegenwart von organischen Säuren, so wird meistens die konzentrierte Form dieser Säuren angewandt, gegebenenfalls in Mischung mit den genannten organischen Lösungsmitteln.

Bevorzugte Phosphite bzw. Phosphonate sind z.B. Dimethylphosphit, Diäthylphosphit, Dimethylmethanphosphonat, Diäthylmethanphosphonat, Methyl-äthyl-methanphosphonat, Methyl-propyl-methanphosphonat, Methyl-butyl-methanphosphonat, Methyl-hexyl-methanphosphonat, Methyl-octylmethanphosphonat, Methyl-decyl-methanphosphonat, Methyldodecyl-methanphosphonat Dimethyl-β-methoxycarbonyläthan-phosphonat, Dimethyl-β-cyanäthan-phosphonat. Die Umsetzung erfolgt in Wasser und/oder organischen Lösungsmitteln wie Methanol, Aethanol, Propanol, Isopropanol, Butanol, Glykol, Glykolmethyläther, Glykol-di-methyläther, Glykolbutyläther, Diglykolmethyläther, Methyläthylketon, Methylbutylketon, Dimethylformamid Sulfolan Oxypropionitril, Toluol, Xylol, Benzylalkohol, Phenoxyäthanol, Benzyloxypropionitril bei vorzugsweise 60 bis 190°C. Bei der Verwendung von flüssigen Verbindungen der Formel (18) kann die Umsetzung auch in Abwesenheit eines zusätzlichen Lösungsmittels vorgenommen werden.

Werden protonierte Verbindungen der Formel (17) gewünscht, d.h. Säure-Additionssalze desselben, so verwendet man als Protonierungsmittel insbesondere Mineralsäuren. Geeignet sind prinzipiell alle starken bis mittels arken organischen Säuren oder Mineralsäuren.

Als Lösungsmittel, in denen die Protonierung vorgenommen werden kann, eignen sich im allgemeinen alle inerten Lösungsmittel. Bevorzugt sind solche, die das Ausgangsprodukt lösen und aus denen sich as Endprodukt sofort ausscheidet. Beispielsweise seien genannt: Aromatische Kohlenwasserstoffe wie Benzol, Toluol und Xylol; Halogenkohlenwasserstoffe wie Trichloräthan, Tetrachloräthylen, Chlorbenzol oder Dichlorbenzol, ferner auch Nitroverbindungen wie Nitromethan, Nitropropan, Nitrobenzol, Alkanole und offene oder cyclische Aether wie Butanol, Dibutyläther, Aethylenglykol, Aethylenglykolmonomethyläther, Aethylenglykolmonoäthyläther, Anisol oder Dioxan; Ketone wie Cyclohexanon oder Methyläthylketon; Fettsäureamide wie Dimethylformamid oder Dimethylacetamid; Sulfoxyde wie Dimethylsulfoxyd und Carbonssäureester wie Essigester oder Essigsäurebutylester.

Distyrylbenzole der Formel (1), worin X und X' die Gruppierung -OCO- und n und n' die Zahllbedeuten, können z.B. aus den entsprechenden Phenolen hergestellt werden, indem man diese mit einem Halogenacylhalogenid umsetzt und das erhaltene Reaktionsprodukt mit einem Trialkylamin bzw. Pyridin der Formel $N(R_1)(R_2)(R_3)$ quaterniert, wobei Y = Y', R = $R'_1$, $R_2$ = $R_2'$ und $R_3$ die vorangehend angegebene Bedeutung haben. Die Quaternierung erfolgt vorteilhaft in einem inerten Lösungsmittel mit mindestens 2 Mol des Amins bei 50 bis 160°C vorzugsweise bei 80-130°C.

Distyrylbenzole der Formel (6) werden dadurch hergestellt, dass man im Molekularverhältnis 1:2 eine

Verbindung der Formel

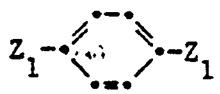

mit solchen der Formel

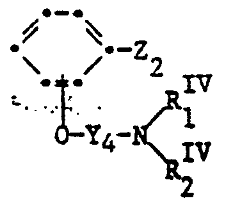

in Gegenwart einer starken Base umsetzt, in welchen Formeln $Y_4$, $R_1$ und $R^{IV}_2$ die oben angegebene Bedeutung haben und eines der Symbole $Z_1$ und $Z_2$ eine OCH-Gruppe und das andere eine Gruppierung der Formel

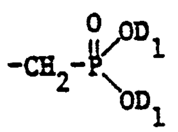

,

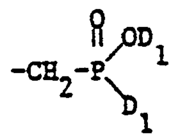

,

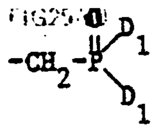

oder

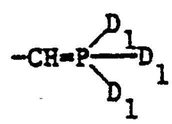

bedeutet, worin $D_1$ einen unsubstituierten oder substituierten Alkyl-, Aryl-, Cycloalkyl- oder Aralkylrest darstellt und gewünschtenfalls die erhaltene Verbindung der Formel

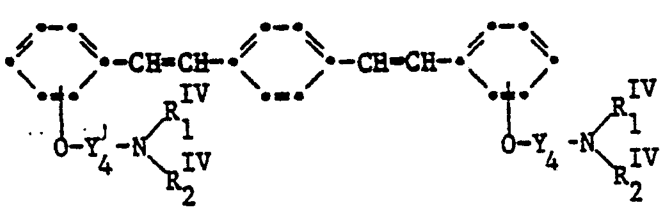

mit 2 Moläquivalenten eines Alkylierungsmittels bzw. einer Säure der Formel $R'_3$'-A quaterniert bzw. protoniert, wobei in diesen Formeln $Y_4$, $R^{IV}_1$ $R^{IV}_2$, $R''_3$ und A die oben angegebene Bedeutung haben.

Die vorstehend definierten neuen Verbindungen zeigen in gelöstem oder feinverteiltem Zustand eine mehr oder weniger ausgeprägte Fluoreszenz. Sie können zum optischen Aufhellen der verschiedensten synthetischen, halbsynthetischen oder natürlichen organischen Materialien oder Substanzen, welche solche organische Materialien enthalten, verwendet werden. Sie können diesen Materialien einverleibt oder auf deren Oberfläche aufgebracht werden. Als organische Materialien kommen vorzugsweise Polyacrylnitril und Cellulose in Betracht.

Die optisch aufzuhellenden organischen Materialien können den verschiedenartigsten

Verarbeitungszuständen (Rohstoffe, Halbfabrikate oder Fertigfabrikate) angehören.

Den erfindungsgemäss anzuwendenden Verbindungen kommt u.a. Bedeutung für die Behandlung von textilen organischen Materialien, insbesondere textilen Geweben, zu.

In Abhängigkeit vom verwendeten Aufheller-Verbindungstyp kann es sich als vorteilhaft erweisen, in neutralem oder alkalischem oder saurem Bade zu arbeiten.

Die neuen optischen Aufhellmittel gemäss vorliegender Erfindung können beispielsweise auch zur Aufhellung von Papiermassen, unter anderem auch in Gegenwart von z.B. kationischen Retentionsmitteln und anderen Zusatzstoffen eingesetzt werden.

Die neuen optischen Aufhellmittel gemäss vorliegender Erfindung können in folgenden Anwendungsformen eingesetzt werden:

a) Mischungen mit Farbstoffen (Nuancierung) oder Pigmenten (Farboder insbesondere z.B. Weisspigmenten) oder als Zusatz zu Färbebäderm,

b) in Mischungen mit Netzmitteln, Weichmachern, Quellmitteln oder Antioxydantien,

c) in Kombination mit verschiedenen Textilveredlungsverfahren, wie z.B. Flammfest-, Weichgriff-,Schmutzablöse ("anti-soiling")- oder Antistatisch-Ausrüstungen oder antimikrobiellen Ausrüstungen,

d) Einarbeiten der optischen Aufhellmittel in polymere Trägermarerialien (Polymerisations-, Polykondensations- oder Polyadditionsprodukte) in gelöster oder dispergierter Form für Amwendung z.B. in Beschichtungs-, Imprägnier- oder Bindemitteln (Lösungen, Dispersionen, Emulsionen) für Textilien, Vliese, Papier, Leder,

e) als Zusätze zu den verschiedensten industriellen Produkten, um dieselben marktfähiger zumachen (z.B. Aspektverbesserung von Seifen, Waschmitteln, Weichspül- und Textilbehandlungsmitteln, Pigmenten),

f) in Kombination mit anderen, optisch aufhellend wirkenden Substanzen,

g) in Spinnbadpräparationen, d.h. als Zusätze zu Spinnbädern, wie sie zur Gleitfähigkeitsverbesserung für die Weiterverarbeitung von Synthese-Faserm verwendet werden, oder aus einem speziellen Bad vor der Versrreckung der Faser, z.B. als Nachbehandlung von nassversponenen Polyacrylfasern im sogenannten Gelzustand.

h) für verschiedene Zwecke photographischer Art, wie z.B. für elektophotographische Reproduktion oder Supersensibilisierung,

i) je nach Substitution als Laser-Farbstoffe. Wird das Aufhellverfahren mit Textilbehandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, dass der gewünschte Aufhelleffekt erreicht wird.

Die Menge der erfindungsgemäss zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,0001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,0005 und 0,5 Gewichtsprozent von Interesse.

Aus verschiedenen Gründen ist es oft zweckmässig, die Aufheller nicht als solche, d.h. rein einzusetzen, sondern vermischt mit den verschiedensten Hilfs- und Coupiermitteln.

Die neuen optischen Aufhellmittel eignen sich besonders als Zusä ze für Waschbäder oder zu Gewerbe- und Haushaltswasch- und Wäschenachbehandlungsmitteln wobei sie in verschiedener Weise zugesetzt werden können. Zu Waschbädern werden sie zweckmässig in Form ihrer Lösungen in Wasser oder organischen Lösungsmitteln oder auch in feiner Verteilung als wässrige Dispersionen zugegeben. Zu Haushaltoder gewerblichen Waschmitteln werden sie vorteilhaft in irgendeiner Phase des Herstellungsprozesses der Waschmittel zugesetzt. Die Zugabe kann sowohl in Form einer Lösung oder Dispersion in Wasser oder anderen Lösungsmitteln als auch ohne Hilfsmittel als trockenes Aufhellerpulver erfolgen. Man kann die Aufhellmittel beispielsweise in den waschaktivensubstanzen lösen oder mit denselben vermischen, verkneten oder vermahlen und so dem fertigen Waschmittel zumischen. Sie können jedoch auch gelöst oder vordispergiert auf das fertige Waschmittel aufgesprüht werden.

Die erfindungsgemässen Verbindungen können auch im Nachspülbad, wie es zur blossen Verleihung von Weichgriff,antistatischen Eigenschaften, Antisoileffekten, Duftnoten usw. üblich ist, eingesetzt werden. Insbesondere eignen sie sich für den Einsatz in Wäschenachbehandlungsmitteln, welche kationische Weichmacher enthalten.

Die neuen optischen Aufheller eignen sich sehr gut als Aufbeller für konzentrierte flüssige Waschmittel, welche nicht-ionogene Tenside und kationische Weichmacher oder Tenside enthalten.

Die vorliegende Erfindung betrifft daher auch ein Waschmittel, welches vorzusgweise in flüssiger Form vorliegt und welches neben den neuen Distyrylbenzolen und den üblichen Zusätzen noch nicht-ionogene Tenside und kationische Textilweichmacher enthält.

Als nicht-ionogene Tenside kommen die handelsüblichen in Betracht, z.B. die wasserlöslichen Produkte die aus der Addition eines Alkylenoxides bzw. einer äquivalenten Verbindung mit einem reaktiven Wasserstoff einer hydrophoben Verbindung erhalten werden. Die hydrophoben organischen Produkte können Heterocyclen und besonders Aliphaten oder Aromaten sein. Bevorzugt sind höhere aliphatische Alkohole und Alkylphenole wobei aber auch andere wie z.B. Carbonsäuren, Carboxamide, Mercaptane, Sulfamide usw. verwendet werden können.

Bevorzugte nichtionogene Verbindungen sind die Additionsprodukte von Aethylenoxid mit höheren aliphatischen Alkoholen mit 6 bis 50 und mehr C-atomen. Die Menge Aethylenoxid kann sich in weiteren Grenzen bewegen aber in allgemeinen werden zumindest 5 Mol Aethylenoxid pro Mol hydrophober Substanz gebraucht. Anstelle von Aethylenoxid können ganz oder teilweise andere niedere Alkylenoxide, wie z.B. Propylenoxid und Butylenoxid verwendet werden. Als weitere nichtionogene verwendbare Verbindungen kommen in Betracht:

a) Polyoxyalkylenester organischer Säuren wie höherer Fettsäuren, Harzsäuren, Tallölsäuren und Säuren der Oxydationsprodukte des Erdöls, deren Ester in der Regel im Säureteil 10 bis 22 C-Atome aufweisen und ca. 12 bis ca. 30 Mole Aethylenoxid oder dessen Aequivalent enthalten:

b) Alkylenoxidaddukte höherer Fettsäureamide, wobei der Fettsäureteil in der Regel 8 bis 22 C-Atome aufweist und mit 10 bis 50 Mol Aethylenoxid kondensiert ist. Die entsprechenden Carboxamide und Sulfamide können ebenfalls als weitgehend äquivalent verwendet werden.

In der Herstellung von flüssigen konzentrierten Waschmitteln werden als nichtionogene Tenside vorzugsweise oxalkylierte höhere aliphatische Alkohole verwendet, wobei die Fettalkohole mindestens 6 und vorzugsweise mindestens 8 C-Atome aufweisen. Bevorzugte Alkohole sind Lauryl-, Myristyl-, Cetyl-, Stearyl- und Oleylalkohol, welche mit mindestens 6 Mol Aethylenoxid kondensiert werden. Als typisches nichtionogenes Produkt sei das Additionsprodukt von einem aliphatischen Alkohol mit 12-13 C-Atomen mit ca. 6,5 Mol Aethylenoxid erwähnt. Die entsprechenden Alkylmercaptane sind, nach Kondensation mit Aethylenoxid, ebenfalls als nichtionogene Tenside verwendbar.

Die alkoxylierten höheren aliphatischen Alkohole sind besonders für Haushaltswaschmittel geeignet, da sie leicht biologisch abbaubar sind und gut mit kationischen Tensiden und Textilweichmachern und den übrigen Zusätzen verträglich sind.

Von den kationischen Textilweichmachern eignen sich besonders quartäre Derivate des Ammoniaks und/oder des Imidazolins mit 2 langkettigan aliphatischen gesättigten oder ungesättigten Resten, wie z.B. 1-Methyl-1-oleylamidoäthyl-2-oleyl-imidazolinium. X⊖, 1-Methyl-1-talgamidoäthyl-2-talg-imidazolinium. X ⊖, Di-talg-dimethylammoninium.X⊖ oder eine Verbindung der Formel

(23)
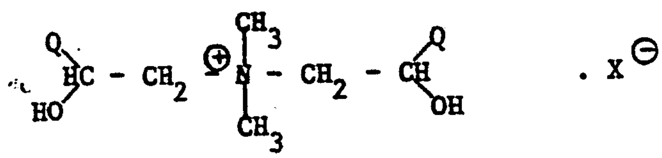

worin Q $C_{14-16}$-Alkyl bedeutet und X⊖ für Chlorid-, Bromid-, Methylsulfat-, Aethylsulfat-, Methan-, Aethan- oder Toluolsulfonatanion steht.

Die erfindungsgemäss verwendbaren quaternären Textilweichmacher und besonders die letzterwähnten verleihen dem Gewebe einen weichen und flaumigen Griff und gleichzeitig eine gute Wiederanfeuchtbarkeit. Diese Textilweichmacher sind substantiv zum Gewebe und tragen zur Verminderung der statischen Aufladung und der Neigung zum Knittern bei, so dass das Gewebe leichter gebügelt werden kann und angenehmer zu tragen ist.

Das flüssige Medium für die erfindungsgemässen Waschmittel ist wässerig und kann aus Wasser allein oder aus Wasser und zusätzlichen Lösungsmitteln für gewisse Zusätze bestehen. Die zusätzlichen Lösungsmittel können bis zu 20, vorzugsweise bis 15 % des gesamten Lösungsmittelanteils ausmachen. Als solche kommen in Betracht: niedere Alkanole oder ein niederes Diol oder Polyol wie z.B. Aethanol, Isopropanol, Aethylenglykol, Propylenglykol und Glycerin. Auch verätherte Polyole wie Diäthenglykol, Aethylenglykoldimethyläther und Aethylenglykolmonoäthyläther können als zusätzliche Lösungsmittel verwendet werden.

Das erfindungsgemässe flüssige Waschmittel kann verschiedene ausgewählte verträgliche Zusätze enthalten, wie Schmutzsuspendiermittel oder Vergrauungsinhibitoren z.B. Polyvinylalkohol, Hydroxypropylmethylcellulose; Schauminhibitoren, Konservierungsmittel, z.B. Natriumbenzoat; UV-Absorber und Parfüme. Diese werden selbstverständlich so gewählt, dass sie mit den Hauptkomponenten des Waschmittels verträglich sind.

Die nichtionogenen Tenside werden in Mengen von 10 bis 70 Gewichts-%, vorzugsweise 60 Gewichts-% eingesetzt-, Die Konzentration das Textilweichmachers beträgt 1 bis 30 Gewichts-%, vorzugsweise 2-21 Gewichts-%. Das wässrige Lösungsmittel, vorzugsweise Wasser, das noch mono- die und mehrwertige Alkohole und ähnliche Lösungsmittel enthalten kann, beträgt 5 bis 60 Gewichts-%. Das flüssige oder pulverförmige, fertige Waschmittel enthält die erfindungsgemässe Verbindung in Mengen von 0,005 bis 3 Gewichts-%. Der Gehalt an übrigen Hilfsmitteln beträgt vorzugsweise weniger als 5 Gewichts-% des Waschmittels, da die Verwendung von grösseren Mengen der Eigenschaften flüssiger Waschmittel beeinflussen kann. Obschon die bevorzugte anmeldungsgemässe waschaktive Zubereitung eine stabile, klare Flüssigkeit ist, kann man ihr ein verträgliches Trübungsmittel hinzugeben um einen opaken Aspekt hervorzurufen.

Das erfindungsgemässe Waschmittel kann in weichem oder angemessen hartem Wasser bei höherer Temperatur zur Anwendung gelangen. Dieses Waschmittel kann auch zum Waschen von Textilien in sehr hartem Wasser bei tieferer Temperatur verwendet werden. Die Wasserhärte kann deshalb von 0 bis über 300

ppm berechnet als Calciumcarbonat schwanken und die Waschtemperatur kann 4 bis 60°C betragen.

Das erfindungsgemässe Waschmittel löst sich sehr leicht in kaltem oder warmem Waschwasser, reinigt gründlich, eliminiert die statische Aufladung und macht die Wäsche weich ohne sie zu hydrophobieren. Das vevorzugte Waschmittel liegt als eine klare, stabile Flüssigkeit vor, die ihre Aktivität und Uniformität über eine längere Zeit panne behält. Zur Herstellung von klaren flüssigen Waschmitteln kann die Konzentration der Aktivsubstanzen nur innerhalb gewissen Grenzen variiert werden. So soll z.B. die Konzentration des Textilweichmachers nicht viel höher sein als 30 %, wenn man ein klar flüssiges Waschmittel erhalten will.

Die erfindungsgemässen Verbindungen werden in Mengen von 0,005 bis 1 % oder mehr bezogen auf das Gewicht des flüssigen oder pulverförmigen, fertigen Waschmittels resp. Textilbehandlungsmittels, zugesetzt. Wasch-/Behandlungsflotten, die die angegebenen Mengen der beanspruchten Aufheller enthalten, verleihen beim Waschen von Textilien aus Cellulosefasern, Polyamidfasern, hochveredelten Cellulosefasern, Polyesterfasern, Wolle etc. diesen einen brillanten Aspekt am Tageslicht.

Die Waschbehandlung wird beispielsweise wie folgt durchgeführt:

Die angegebenen Textilien werden während 1 bis 30 Minuten bei 20 bis 100°C in einem Waschbad behandelt, das 0,1 bis 10 g/kg eines aufgebauten, zusammengesetzten Waschmittels und 0,01 bis 1 %, bezogen auf das Waschmittelgewicht, der beanspruchten Aufhellmittel enthält. Das Flottenverhältnis kann 1:3 bis 1:50 betragen. Nach dem Waschen wird wie üblich gespült und getrocknet.

In den Beispielen sind Teile, soweit nicht anders angegeben, immer Gewichtsteile und Prozente immer Gewichtsprozente. Schmelz- und Siedepunkte sind, sofern nicht anders vermerkt, unkorrigiert und besonders bei quaterniedten Verbindungen unscharf.

**Beispiel 1**: 4,2 g der Verbindung der Formel

(100) 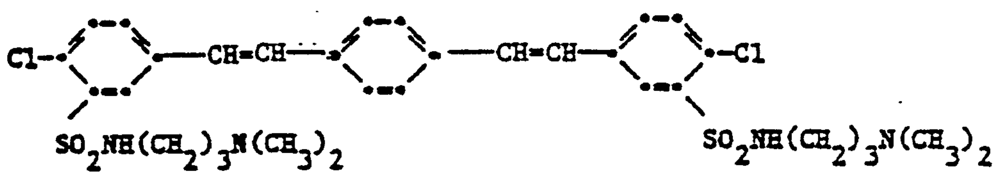

werden in 300 ml Methanol suspendiert. Nach Zugabe von 6,9 ml Dimethylsulfat wird unter Rühren auf Siedetemperatur erhitzt und während 7 Stunden unter Rückfluss gekocht. Nach dem Abkühlen auf Raumtemperatur wird des auskristallisierte Produkt abgenutscht, mit Methanol gewaschen und unter Vakuum getrocknet. Nach dem Extrahieren mit Toluol erhält man 4,7 g der Verbindung der Formel

(101) 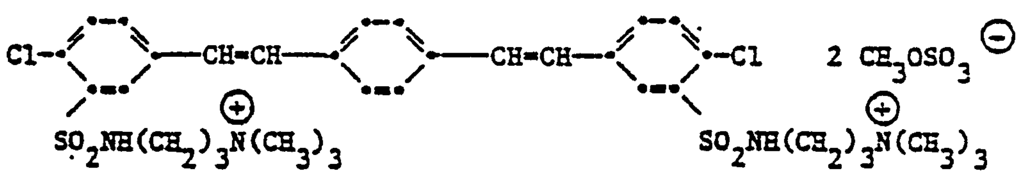

als hellgelbe Kristalle vom FP. 145 bis 147° C.

Die Verbindung der Formel (100) kann wie folgt erhalten werden:

37;3 g der Verbindung der Formel

(102) 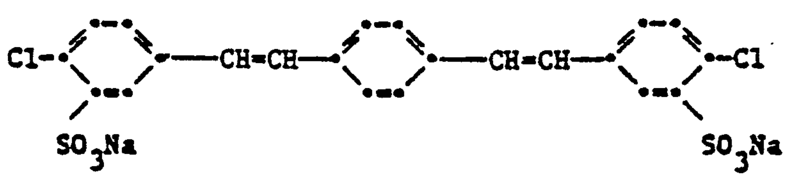

wird in 455 ml Chlorbenzol bei Raumtemperatur suspendiert. Nach Zugabe von 1,3 ml Dimethylformamid werden innert 20 Minuten 14,5 ml Thionylchlorid zugetropft. Dann wird unter Rühren auf 95 bis 105° C erhitzt und 4 Stunden bei dieser Temperatur gerührt. Dann werden 120 ml Thionylchlorid und Chlorbenzol abdestilliert, auf 5° C abgekühlt und 21,4 g calc. Soda langsam zugegeben. Zur hellgelben Suspension werden bei 5 bis 10° C innert 30 Minuten 14,4 g 3-Dimethylamino-1-propylamin in 17 ml Chlorbenzol zugetropft, auf 106° C erhitzt und nach Verdünnen mit 300 ml Chlorbenzol 7 Stunden bei dieser Temperatur gerührt. Nach Abkühlen auf 95° C wird heiss genutscht, mit Chlorbenzol gewaschen und unter Vakuum getrocknet. Nach Umkristallisation aus Methanol und

dann aus Toluol erhält man 14,7 g der Verbindung der Formel (100) als hellgelbe Kristalle vom Fp. 190 bis 192° C.

Die Verbindung der Formel (102) kann wie folgt erhalten werden: 18,9 g p-Xylylen-diphosphonsäuretetraäthylester und 25,2 g 4-Chlor-benzaldehyd-3-sulfonsäure, Natriumsalz (Gehalt: 96 %) werden in 200 ml Dimethylsulfoxid bei 40° C unter Verdrängung der Luft durch einen Stickstoffstrom, gelöst. Innert 20 Minuten werden dann 7,2 g Natriummethylat (Gehalt: 97,3 %) bei 40 bis 45° C eingetragen und noch 4 Stunden lang bei 40 bis 45° C nachgerührt. Dann werden unter Kühlung 100 ml entsalztes Wasser zugegeben. Bei 15° C wird das auskristallisierte Produkt abgenutscht, mit 100 ml entsalztem Wasser gewaschen und zweimal aus 2000 ml bzw. 1500 ml Wasser umkristallisiert. Nach Trocknung unter Vakuum bei 85° C erhält man 15,3 g der Verbindung der Formel (102). UV-Spektrum: $\lambda_{max}$: 363 nm gemessen in Dimethyliormamid/Wasser 1:1.

**Beispiel 2:** 3,6 g der Verbindung der Formel (100) werden in 25 ml Toluol mit 5 ml Methylphosphonsäuredimethylester während 5 Stunden unter Rühren unter Rückfluss gekocht. Nach dem Abkühlen auf Raumtemperatur wird das Produkt abgenutscht und unter Vakuum getrocknet. Man erhält so 4,4 g der Verbindung der Formel

(200) 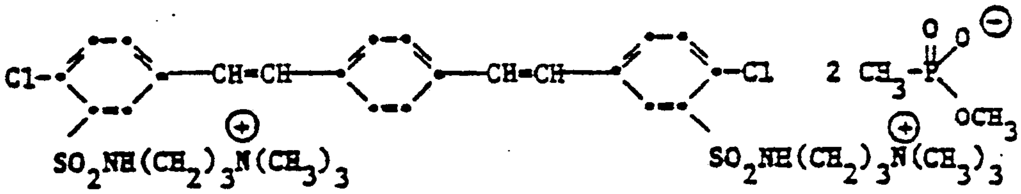

Schmelzpunkt: 201-209° C

Auf ähnliche Weise wie vorstehend beschrieben können die Verbindungen der Formel

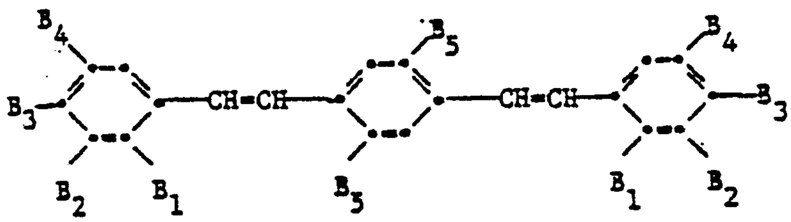

| $B_1$ | $B_2$ | $B_3$ | $B_4$ | $B_5$ |
|---|---|---|---|---|
| $-SO_2NH(CH_2)_3N(CH_3)_2$ | H | H | H | H |
| H | $-SO_2NH(CH_2)_2N(CH_3)_2$ | Cl | H | H |
| H | $-SO_2NH(CH_2)_3N(C_2H_5)_2$ | Cl | H | H |
| H | $-SO_2NH(CH_2)_2N(C_2H_5)_2$ | Cl | H | H |
| H | $-SO_2NH(CH_2)_3N(CH_3)_2$ | $CH_3$ | H | H |
| H | $-SO_2NH(CH_2)_3N(CH_3)_2$ | $OCH_3$ | H | H |

| $B_1$ | $B_2$ | $B_3$ | $B_4$ | $B_5$ |
|---|---|---|---|---|
| $OCH_3$ | H | H | $-SO_2NH(CH_2)_3N(CH_3)_2$ | H |
| Cl | H | H | $-SO_2NH(CH_2)_3N(CH_3)_2$ | H |
| $-SO_2NH(CH_2)_3N(CH_3)_2$ | H | Cl | H | H |
| $CH_3$ | H | $CH_3$ | $-SO_2NH(CH_2)_3N(CH_3)_2$ | H |
| H | $-SO_2NH(CH_2)_3N(CH_3)_2$ | $CH_3$ | H | Cl |
| $-SO_2NH(CH_2)_3N(CH_3)_2$ | H | H | H | $CH_3$ |
| $-CONH(CH_2)_3N(CH_3)_2$ | H | H | H | H |
| $-COO(CH_2)_2N(C_2H_5)_2$ | H | H | H | H |

sowie die entsprechenden quaternären Verbindungen der Formel

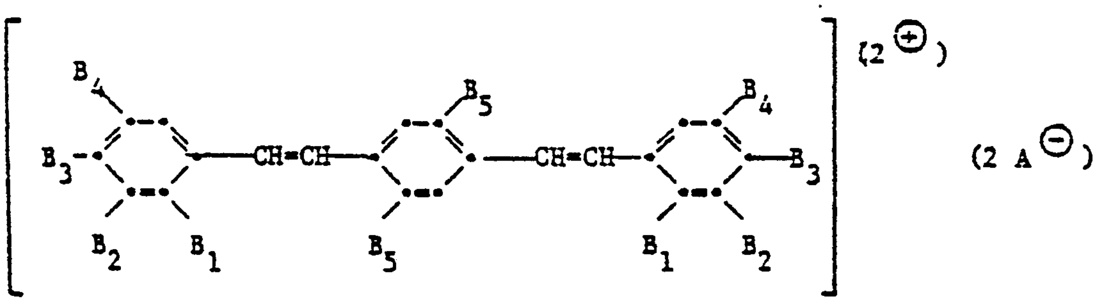

hergestellt werden.

Tabelle 1

| $B_1$ | $B_2$ | $B_3$ | $B_4$ | $B_5$ | $A^{\ominus}$ |
|---|---|---|---|---|---|
| $-SO_2NH(CH_2)_3N(CH_3)_3$ | H | H | H | H | $CH_3OSO_3^{\ominus}$ |
| $-SO_2NH(CH_2)_3N(CH_3)_3$ | H | H | H | H | 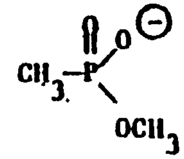 |
| H | $-SO_2NH(CH_2)_2N(CH_3)_3$ | Cl | H | H | 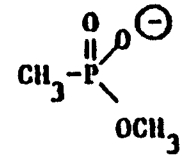 |
| H | $-SO_2NH(CH_2)_3\underset{CH_3}{\underset{\mid}{N}}(C_2H_5)_2$ | Cl | H | H | $CH_3OSO_3^{\ominus}$ |
| H | $-SO_2NH(CH_2)_2\underset{CH_3}{\underset{\mid}{N}}(C_2H_5)_2$ | Cl | H | H | 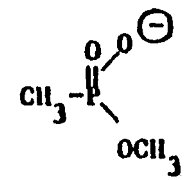 |
| H | $-SO_2NH(CH_2)_3N(CH_3)_3$ | $CH_3$ | H | H | $CH_3OSO_3^{\ominus}$ |
| H | $-SO_2NH(CH_2)_3N(CH_3)_3$ | $OCH_3$ | H | H | 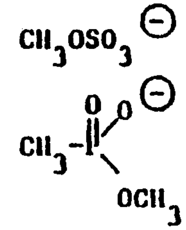 |

Tabelle 2

| $B_1$ | $B_2$ | $B_3$ | $B_4$ | $B_5$ | $A^{\ominus}$ |
|---|---|---|---|---|---|
| $OCH_3$ | H | H | $-SO_2NH(CH_2)_3N(CH_3)_3$ | H | 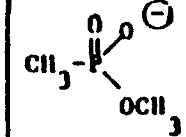 |
| Cl | H | H | $-SO_2NH(CH_2)_3N(CH_3)_3$ | H | 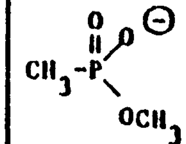 |
| $-SO_2NH(CH_2)_3N(CH_3)_3$ | H | Cl | H | H | 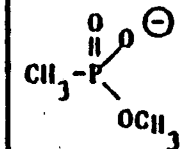 |
| $CH_3$ | H | $CH_3$ | $-SO_2NH(CH_2)_3N(CH_3)_3$ | H | 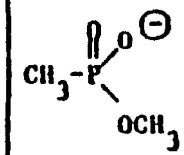 |
| H | $-SO_2NH(CH_2)_3N(CH_3)_3$ | $CH_3$ | H | Cl | 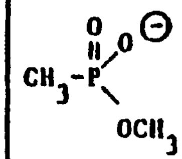 |
| $-SO_2NH(CH_2)_3N(CH_3)_3$ | H | H | H | $CH_3$ | 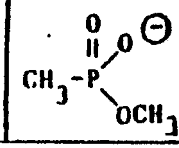 |

| $B_1$ | $B_2$ | $B_3$ | $B_4$ | $B_5$ | $A^{\ominus}$ |
|---|---|---|---|---|---|
| $-CONH(CH_2)_3N(CH_3)_3$ | H | H | H | H | $CH_3OSO_3^{\ominus}$ |
| $-COO(CH_2)_2\underset{CH_3}{\underset{\vert}{N}}(C_2H_5)_2$ | H | H | H | H | $CH_3OSO_3^{\ominus}$ |

**Beispiel 3**: Zu einer Lösung von 75,7 g 1,4-Bis-(diäthoxyphosphono-methyl)-benzol und 96,9 g der Verbindun der Formel

(300) 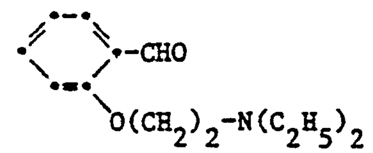

(Gehalt 96 %) in 300 ml Dimethylformamid tropft man nach Verdrängen der Luft durch Stickstoff unter Rühren 88,6 g einer methanolischen 30,5 %-igen Natriummethylatlösung, so dass die Temperatur nicht über 40' ansteigt. Man hält die Temperatur 2 Stunden bei 40-45°C, kühlt mit Eiswasser ab und versetzt die Reaktionsmischung mit 600 ml Wasser. Das ausgefallene Produkt wird abgenutscht, wiederholt mit Wasser gewaschen und im Vakuum über Calciumchlorid getrocknet. Man erhält 96,4 g der Verbindung der Formel

(301) 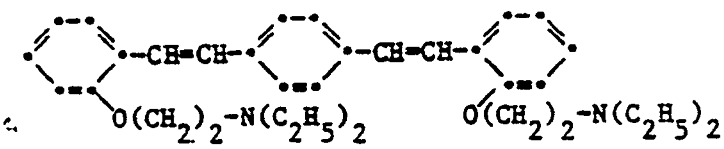

in Form leuchtend hellgelber Kristalle vom Smp. 90-91°C (nach Umkristallisation am Hexan und Isopropanol)

Die Verbindung der Formel (300) kann wie folgt hergestellt werden: Zu einer Suspension von 244 g 8alicylaldehyd und 344 g 2-Diäthylamino-äthylchlorid-hydrochlorid in 2000 ml Chlorbenzol tropft man nach Verdrängen der Luft durch Stickstoff unter Rühren 732 g einer methanolischen 30,7 %igen Natriummethylatlösung, wobei sich die Suspension gelb färbt und dick wird. Man steigert die Temperatur allmählich bis etwa 131°C und destilliert dabei das Methanol ab. Man belässt noch 4 Stunden bei dieser Temperatur, lässt abkühlen, fügt 500 ml Wasser hinzu, trennt die beiden Schichten und trocknet die organische Phase mit Natriumsulfat. Das Lösungsmittel wird alsdann im Wasserstrahlvakuum abgedampft und der Rückstand im Hochvakuum fraktioniert destilliert. Nach Abtrennung eines geringen Vorlaufes erhält man 327,5 g einer leicht rötlichen Flüssigkeit vom Kp. 105-118°C/ 0.06 mbar mit einem Gehalt von 96 %.

In analoger Weise erhält man durch Umsetzung von 1,4-Bis-(diäthoxyphosphonomethyl)-benzol mit entsprechenden Aldehyden die in Tabelle I aufgeführten Verbindungen der Formel

(302) 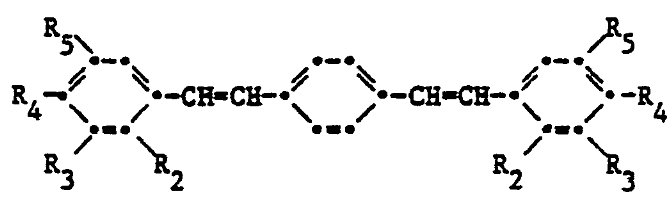

Tabelle I

| Formel | $R_2$ | $R_3$ | $R_4$ | $R_5$ | Smp. (°C) |
|---|---|---|---|---|---|
| (303) | $-O(CH_2)_3-N(CH_3)_2$ | H | H | H | 104° |
| (304) | $-O(CH_2)_2-N(CH_3)_2$ | H | H | H | 116° |
| (305) | $-O(CH_2)_2-$ 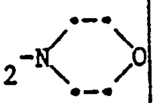 | H | H | H | 119° |
| (306) | $-O(CH_2)_2-$ 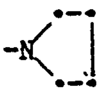 | H | H | H | 161° |
| (307) | $-O(CH_2)_2-$ 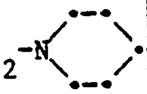 | H | H | H | 128° |
| (308) | $-O(CH_2)_3-N(CH_3)_2$ | H | H | -Cl | 110° |
| (309) | $-O(CH_2)_3-N(CH_3)_2$ | H | H | $-CH_3$ | 117° |
| (310) | $-O(CH_2)_3-N(CH_3)_2$ | $-OCH_3$ | H | H | 102° |
| (311) | H | $-O(CH_2)_2-N(C_2H_5)_2$ | H | H | 86° |

Tabelle I (Fortsetzung)

| Formel | $R_2$ | $R_3$ | $R_4$ | $R_5$ | Smp. (°C) |
|---|---|---|---|---|---|
| (312) | H | H | $-O(CH_2)_2-N(C_2H_5)_2$ | H | 211° |
| (313) | $-OCH_2-$ 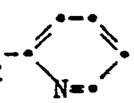 | H | H | H | 176° |
| (314) | $-S(CH_2)_2-N(CH_3)_2$ | H | H | H | 78° |
| (315) | $-CH_2N(CH_3)_2$ | H | H | H | 125° |

Der zur Herstellung der Verbindung dr Formel (314) benötigte Aldehyd der formel

(316) 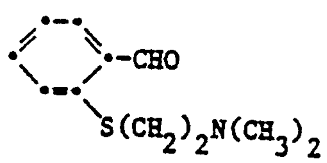

kann wie folgt hergestellt werden: zu einer Mischung von 61,7 g Kaliumhydroxid 88 %ig in 54,3 g Wasser und 60 ml Dimethylsulfoxid fügt man nach Verdrängen der Luft durch Stickstoff unter heftigem Rühren und Kühlen 100 g Dimethylamino-äthantthiol-hydrochlorid ca. 85 %ig hinzu, so dass sie Temperatur nicht über 20°C ansteigt. Alsdann lässt man 57,2 g o-Chlorbenzaldehyd eintropfen und hält die Temperatur je 2 Stunden bei 60°C und dann bei 80°C. Nach dem Abkühlen auf Raumtemperatur versetzt man mit 300 ml Methylenchlorid und 300 ml Wasser, mischt durch, lässt die beiden Schichten abtrennen und trocknet die organische Phase mit Natriumsulfat. Das Lösungsmittel wird abgedampft und der Rückstand im Hochvakuum fraktioniert destilliert. Nach Abtrennung eines Vorlaufes erhält man 18,6 g einer gelblichen Flüssigkeit vom Kp. 97-106°C/0.03 mbar mit einem Gehalt von 79,3 %.

**Beispiel 4**: Verwendet man in Beispiel 3 anstelle eines einheitlichen Aldehyds eine Mischung (400) der beiden isomeren Aldehyde

(400a) 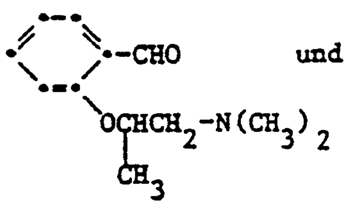 und (400b) 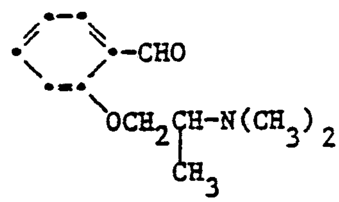

so erhält man eine Isomerenmischung (401) der beiden symmetrischen Verbindungen der Formeln (401a) und (401b) und der asymmetrischen Verbindung der Formel (401c) vom Smp. 50-70° C (nach Umkristallisation aus Hexan):

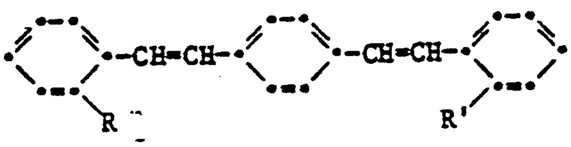

(401a) R = R' = 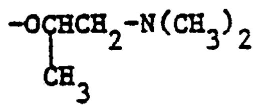

(401b) R = R' = 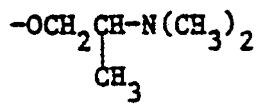

(401c) R = 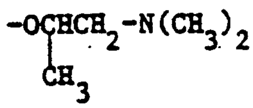 R' = 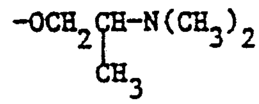

Die Isomerenmischung der Aldehyde der Formel (400) erhält man durch Umsetzung von Salicylaldehyd mit 2-Chlor-1-dimethyl-aminopropan-hydrochlorid gemäss Beispiel 3, Formel (300).

**Beispiel 5**: En ähnlicher Weise wie in Beispiel 3 beschrieben, erhält man die in Tabelle II aufgeführten Verbindungen der Formel

(500) 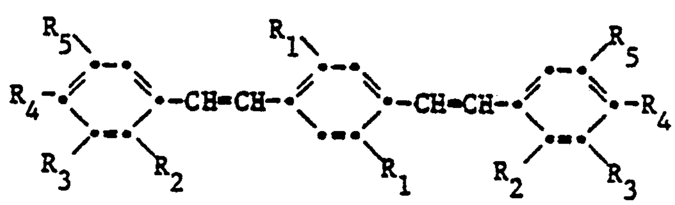

Tabelle II

| Formel | $R_1$ | $R_2$ | $R_3$ | $R_4$ | $R_5$ |
|---|---|---|---|---|---|
| (501) | $CH_3$ | $-O(CH_2)_2-N(C_2H_5)_2$ | H | H | H |
| (502) | Cl | $-O(CH_2)_2-N(C_2H_5)_2$ | H | H | H |
| (503) | H | -Cl | $-O(CH_2)_2-N(C_2H_5)_2$ | H | H |
| (504) | H | -Cl | H | $-O(CH_2)_2-N(C_2H_5)_2$ | H |
| (505) | H | $-O(CH_2)_2-N(C_2H_5)_2$ | Cl | H | Cl |
| (506) | H | $-O(CH_2)_2-N(C_2H_5)_2$ | H | $-(CH_2)_3-$ | |
| (507) | H | $-O(CH_2)_2-N(C_2H_5)_2$ | H | $-(CH_2)_4-$ | |
| (508) | H | H | H | $-O(CH_2)_3-N(CH_3)_2$ | H |
| (509) | H | H | H | $-O(CH_2)_2-N(CH_3)_2$ | H |
| (510) | H | $-O(CH_2)_2-N(C_2H_5)_2$ | $-CH_2CH=CH_2$ | H | H |
| (511) | H | $CH_3$ | H | $-O(CH_2)_2-N(C_2H_5)_2$ | $-CH(CH_3)_2$ |
| (512) | H | $-O(CH_2)_2-N(C_2H_5)_2$ | $CH_3$ | H | $CH_3$ |

**Tabelle II** (Fortsetzung)

| Formel | $R_1$ | $R_2$ | $R_3$ | $R_4$ | $R_5$ |
|---|---|---|---|---|---|
| (513) | H | $-O(CH_2)_2-N(C_2H_5)_2$ | H | $CH_3$ | Cl |
| (514) | H | $-OCH_2\overset{\underset{\|}{O}}{C}NH(CH_2)_3-N(CH_3)_2$ | H | H | H |
| (515) | H | $-OCH_2\overset{\underset{\|}{O}}{C}O(CH_2)_2-N(CH_3)_2$ | H | H | H |

Die Verbindungen der Formeln (514) und (515) erhält man durch Umsetzung von

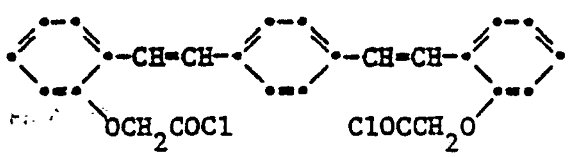

(Herstellung gemäss DE-08 2'209'126) mit 3-Dimethylamino-propylamin-(1) bzw. 2-Dimethylaminoäthanol analog Beispiel 1, Formel (100).

**Beispiel 6**: Zu einer Lösung von 4,85 g der Verbindung der Formel (303) in 50 ml Methyläthylketon tropft man unter Rühren bei ca. 70° C 2,5 ml Dimethylsulfat. Man erhitzt noch 1 Stunde bei Rückflusstemperatur, lässt abkühlen, nutscht das ausgefallene Produkt ab und wäscht wiederholt mit Methyläthylketon. Nach dem Trocknen im Vakuum bei 100° C erhält man 7,2 g (96 % der Theorie) der Verbindung der Formel

(600) 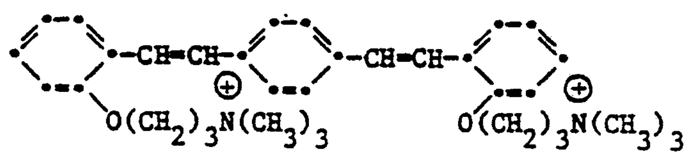 $2\ CH_3SO_4^{\ominus}$ ,

die etwa 1/2 Mol Kristallwasser enthält, in Form leuchtend hellgelber Kristalle vom Smp. 254° C(unscharf).

Anstelle von Methyläthylketon kann auch Chlorbenzol als Lösungsmittel verwendet werden.

Vermindert man in diesem Beispiel die eingesetzte Menge Dimethylsulfat von 2,5 ml auf 1,0 ml, so erhält man eine Mischung der symmetrischen Verbindung der Formel (600) mit der asymmetrischen Verbindung der Formel

(600a) 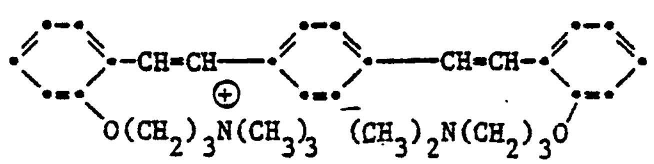 $CH_3SO_4^{\ominus}$ .

Im analoger Weise erhält man aus der Verbindung der Formel (301) sowie den in Tabelle I beschriebenen Verbindungen die in folgender Tabelle III aufgeführten Aufheller der Formel

(601) 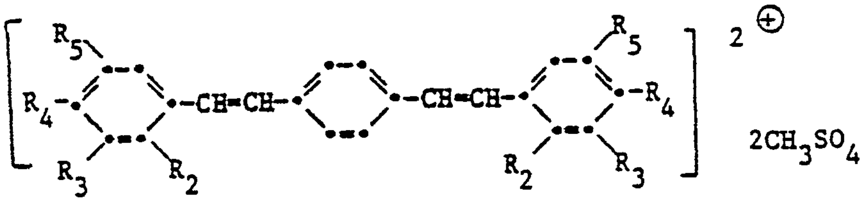 $2CH_3SO_4^{\ominus}$

Tabelle III

| Formel | $R_2$ | $R_3$ | $R_4$ | $R_5$ | Smp. (°C) |
|---|---|---|---|---|---|
| (602) | $-O(CH_2)_2-N(C_2H_5)_2$ $CH_3$ | H | H | H | 223° |
| (603) | $-O(CH_2)_2-N(CH_3)_3$ | H | H | H | 259° |
| (604) | 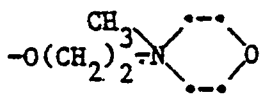 | H | H | H | 189° |
| (605) | 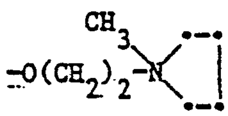 | H | H | H | 175° |

Tabelle III (Fortsetzung)

| Formel | | | | | Smp. (°C) |
|---|---|---|---|---|---|
| (606) | $-O(CH_2)_2-$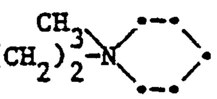 | H | H | H | 181° |
| (607) | $-O(CH_2)_3-N(CH_3)_3$ | H | H | -Cl | 284° |
| (608) | $-O(CH_2)_3-N(CH_3)_3$ | H | H | $-CH_3$ | 233° |
| (609) | $-O(CH_2)_3-N(CH_3)_3$ | $-OCH_3$ | H | H | 174° |
| (610) | H | $-O(CH_2)_2-\overset{CH_3}{N}(C_2H_5)_2$ | H | H | 169° |
| (611) | H | H | $-O(CH_2)_2-\overset{CH_3}{N}(C_2H_5)_2$ | H | 191° |
| (612) | $-OCH_2-$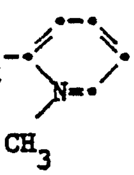 | H | H | H | 164° |
| (613) | $-S(CH_2)_2-N(CH_3)_3$ | H | H | H | 225° |
| (614) | $-CH_2N(CH_3)_3$ | H | H | H | 278° (Zers.) |

Zur Herstellung der Verbindung der Formel (611) werden anstelle von Methyläthylketon 100 ml 1,2-Dichloräthan verwendet. Bei der Herstellung der Verbindungen der Formeln (605) und (612) wird zum vollständigen Lösen der Ausgangsprodukte noch etwas Dimethylformamid zugefügt.

**Beispiel 7**: Quaterniert man die Isomerenmischung der Formel (401) gemäss Beispiel 5, so erhält man die Isomerenmischung der Formel (700), die sich aus den beiden symmetrischen Verbindungen (700a) und (700b) sowie der asymmetrischen Verbindung (700c) zusammensetzt

(700) 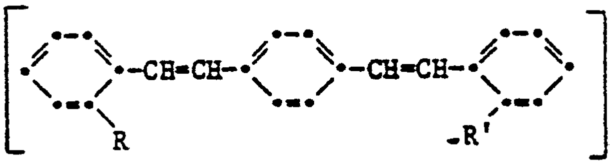 $2^{\oplus}$ $2\ CH_3SO_4^{\ominus}$

(700a) R = R' = 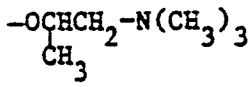

(700b) R = R' = 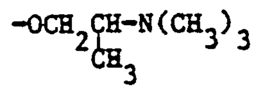

(700c) R = 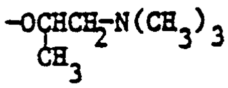 R' = 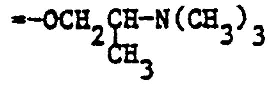

vom Smp. 240°C(unscharf) und etwa 1/2 Mol Kristallwasser enthält.

**Beispiel 8**: In eine Lösung von 4,7 g der Verbindung der Formel

(800) 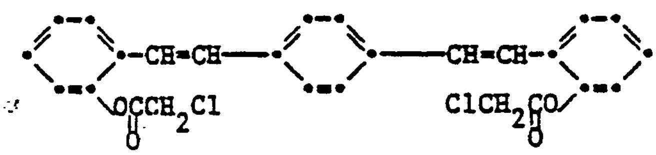

in 50 ml Chlorbenzol leitet man bei 90°C im Verlauf von 10 Minuten unter Rühren 5,5 g Trimethylamin ein. Man rührt noch weitere 15 Minuten bei dieser Temperatur, nutscht das ausgefallene Produkt bei etwa 60°C ab, wäscht wiederholt mit Methyläthylketon und trocknet im Vakuum bei 100°C. Man erhält 5,0 g der Verbindung der Formel

(801) 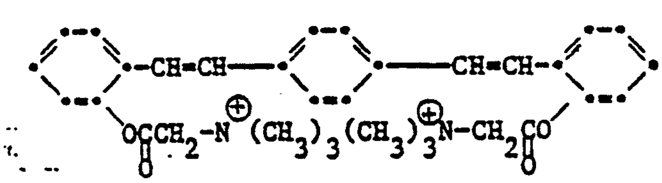 $2\ Cl^{\ominus}$

vom Smp. 235-238°C.

Verwendet man in diesem Beispiel 3,2 ml Pyridin anstelle von Trimethylamin und 50 ml Methyläthylketon als Lösungsmittel und arbeitet bei Rückflusstemperatur (1 Stunde), so erhält man 3,5 g der Verbindung der Formel

(802) 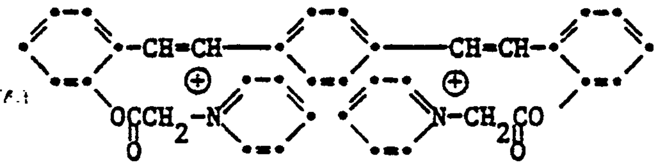 $2\ Cl^{\ominus}$

vom Smp. 227-235°C(Zers.).

Die als Ausgangsprodukt benötigte Verbindung der Formel (800) erhält man wie folgt: Zu einer Lösung von 21,5 g Terepthalaldehyd und 158,2 g der Verbindung der Formel

(803) 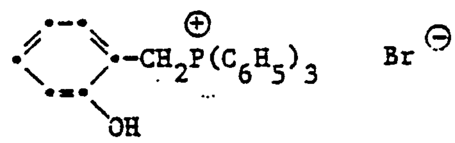

in 500 ml Dimethylformamid tropft man nach Verdrängen der Luft durch Stickstoff unter Rühren 126,6 g einer methanolischen 30,7 %igen Natriummethylatlösung, so dass die Temperatur nicht über 40°C ansteigt. Man hält die Temperatur 2 Stunden bei 40-45°C, lässt abkühlen und versetzt die Reaktionsmischung mit 150 ml Wasser. Durch Einleiten von Kohlendioxid bis zur Sättigung oder durch Zugabe von Trockeneis neutralisiert man auf pH 8, wobei das gelblich gefärbte Reaktionsprodukt ausfällt. Dieses wird abgenutscht, wiederholt mit Wasser gewaschen, im Vakuum getrocknet und aus Methyläthylketon umkristallisiert. Man erhält 13,8 g der Verbindung der Formel

(804) 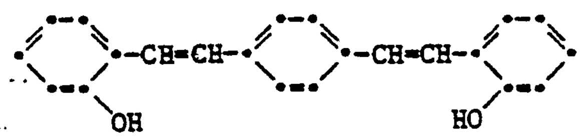

vom Smp. 275-280°C.

12,6 g der Verbindung der Formel (804) werden in 40 ml Chloracetylchlorid über Nacht unter Rückfluss verrührt, wobei zuletzt vollständige Lösung eintritt. Man versetzt mit 40 ml Cyclohexan, kühlt ab, nutscht das ausgefallene Produkt ab, wäscht es wiederholt mit Cyclohexan und trocknet es im Vakuum bei 80°C. Nach Umkristallisation aus Nonan erhält man 11,6 g der Verbindung der Formel (800) in Form blassgelber Kristalle vom Smp. 146-149°C.

**Beispiel 9**: In äthnlicher Weise wie in den Beispielen 5 oder 7 beschrieben, erhält man die in Tabelle IV aufgeführten Verbindungen der Formel

(900) 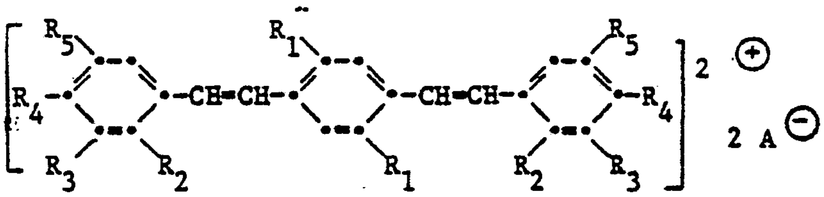

Tabelle IV

Tabelle IV

| Formel | $R_1$ | $R_2$ | $R_3$ | $R_4$ | $R_5$ | A |
|---|---|---|---|---|---|---|
| (901) | $CH_3$ | $-O(CH_2)_2-N(CH_3)(C_2H_5)_2$ | H | H | H | $CH_3OSO_3$ |
| (902) | Cl | $-O(CH_2)_2-N(CH_3)(C_2H_5)_2$ | H | H | H | " |
| (903) | H | -Cl | $-O(CH_2)_2-N(CH_3)(C_2H_5)_2$ | H | H | " |
| (904) | H | -Cl | H | $-O(CH_2)_2-N(CH_3)(C_2H_5)_2$ | H | " |
| (905) | H | $-O(CH_2)_2-N(CH_3)(C_2H_5)_2$ | -Cl | H | -Cl | " |
| (906) | H | $-O(CH_2)_2-N(CH_3)(C_2H_5)_2$ | H | $-(CH_2)_3-$ | | " |
| (907) | H | $-O(CH_2)_2-N(CH_3)(C_2H_5)_2$ | H | $-(CH_2)_4-$ | | " |
| (908) | H | H | H | $-O(CH_2)_3-N(CH_3)_3$ | H | " |
| (909) | H | H | H | $-O(CH_2)_2-N(CH_3)_3$ | H | " |
| (910) | H | $-O(CH_2)_2-N(CH_3)(C_2H_5)_2$ | $-CH_2CH=CH_2$ | H | H | " |

Tabelle IV (Fortsetzung)

| Formel | $R_1$ | $R_2$ | $R_3$ | $R_4$ | $R_5$ | A |
|---|---|---|---|---|---|---|
| (911) | H | $-CH_3$ | H | $CH_3$<br>$-O(CH_2)_2-N(C_2H_5)_2$ | 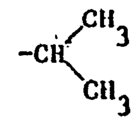 | $CH_3OSO_3$ |
| (912) | H | $CH_3$<br>$-O(CH_2)_2-N(C_2H_5)_2$ | $-CH_3$ | H | $-CH_3$ | " |
| (913) | H | $CH_3$<br>$-O(CH_2)_2-N(C_2H_5)_2$ | H | $CH_3$ | Cl | " |
| (914) | H | $-OCH_2CHCH_2N(CH_3)_2$<br>OH | H | H | H | " |
| (915) | H | H | H | $-O(CH_2)_2-N(C_2H_5)_2$<br>$CH_3$ | H | 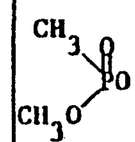 |
| (916) | H | $-O(CH_2)_2-N(C_2H_5)_3$ | H | H | H | Br |
| (917) | H | $-O(CH_2)_2-N(C_2H_5)_2$<br>$CH_2C_6H_5$ | H | H | H | Cl |
| (918) | H | H | H | $-O(CH_2)_2-N(C_2H_5)_2$<br>$CH_2COOCH_3$ | H | Cl |
| (919) | H | $-O(CH_2)_2-N(C_2H_5)_2$<br>$CH_3$ | H | H | H | 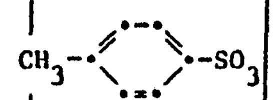 |

Tabelle IV (Fortsetzung)

| Formel | $R_1$ | $R_2$ | $R_3$ | $R_4$ | $R_5$ | A |
|---|---|---|---|---|---|---|
| (920) | H | H | H | $-O(CH_2)_2-N(C_2H_5)_2$<br>$CH_2CN$ | H | Cl |
| (921) | H | H | H | $-O(CH_2)_3-N(CH_3)_2$<br>$C_2H_5$ | H | $C_2H_5OSO_3$ |
| (922) | H | H | H | $-CH_2N(CH_3)_3$ | H | Cl |
| (923) | H | $-OCH_2\overset{O}{\overset{\|}{C}}NH(CH_2)_3N(CH_3)_3$ | H | H | H | $CH_3OSO_3$ |
| (924) | H | $-OCH_2\overset{O}{\overset{\|}{C}}O(CH_2)_2N(CH_3)_3$ | H | H | H | $CH_3OSO_3$ |
| (925) | H | $-O(CH_2)_3-N(CH_3)_2$<br>$CH_2CH=CH_2$ | H | H | H | Cl |
| (926) | H | $-O(CH_2)_3-N(CH_3)_2$<br>$CH_2CH_2OH$ | H | H | H | $CH_3COO$ |
| (927) | H | $-O(CH_3)_3-N(CH_3)_2$<br>$CH_2CHCH_3$<br>$OH$ | H | H | H | HCOO |

**Beispiel 10**: Ein gebleichtes Baumwoll-Gewebe wird auf einem Färbeapparat bei einem Flottenverhältnis von 1:20 mit einem wässrigen Bad behandelt, das 0,1 % des Aufhellers der Formel (101), (200), (600), (602) bis (606) oder (700), bezogen auf das Gewicht der Baumwolle, und 5 g/l Natriumsulfat enthält.

Die Applikation erfolgt gemäss folgendem Temperaturprogramm:
20-50°C / 15 Minuten
50°C / 15 Minuten

Anschliessend wird das Baumwoll-Gewebe während 20 Sekunden fliessendem enthärtetem Wasser gespült und bei 70°C im Trockenschrank getrocknet. Das so behandelte Baumwoll-Gewebe weist einen guten Aufhelleffekt auf.

**Beispiel 11**: Man foulardiert bei Raumtemperatur ein gebleichtes Baumwoll-Gewebe mit einer wässrigen Flotte, die 1 g/l des Aufhellers der Formel (101) oder (200) enthält. Der Abquetscheffekt beträgt 75 %.

Anschliessend wird während 30 Sekunden bei 130°C auf einem Thermofixiergerät getrocknet.

Das so behandelte Baumwollgewebe weist einen guten Aufhelleffekt auf.

**Beispiel 12**: Ein Polyacrylnitril-Gewebe (Orlon 75) wird auf einem Färbeapparat bei einem Flottenverhältnis von 1:20 mit einem wässrigen Bad, das
0,1 % des Aufhellers der Formel (101), (200),(301),(303) bis (307), (401) (600), (602 bis (606) oder (700), bezogen auf das Warengewicht,
1 g/l eines Anlagerungsproduktes von 35 Mol Aethylenoxid an 1 Mol Stearylalkohol und
1,5 ml/l Ameisensäure 85 %.
enthält, behandelt.

Die Applikation erfolgt gemäss folgendem Temperaturprogramm:
40-97°C / 30 Minuten
97°C / 30 Minuten
97-40°C / 15 Minuten.

Anschliessend wird das Polyacrylnitril-Gewebe während 20 Sekunden in fliessendem enthärtetem Wasser gespült und bei 70°C im Trockenschrank getrocknet. Das so behandelte Gewebe weist einen guten Aufhelleffekt auf.

**Beispiel 13**: Ein Polyacrylnitril-Gewebe (Courtelle ®) wird auf einem Färbeapparat bei einem Flottenverhältnis 1:20 mit einem wässrigen Bad behandelt, das
0,1 % des Aufhellers der Formel (301), (303) bis (307), (401), (600), (602) bis (606) oder (700) bezogen auf das Gewebegewicht
1 g/l Oxalsäure
0,25 g/l Natrium-Hexametaphosphat und
0,125 g/l Natrium-Metabisulfit
enthält.

Die Applikation erfolgt nach folgendem Temperaturprogramm:
40-100°C / 30 Minuten
100°C / 30 Minuten
100- 40°C / 15 Minuten

Anschliessend wird das Polyacrylnitril-Gewebe während 30 Sekunden in fliessendem, enthärtetem Wasser gespült, zentrifugiert und mit einem 150°C warmen Bügeleisen getrocknet. Das so behandelte Gewebe weist einen guten Aufhelleffekt auf.

**Beispiel 14**: Ein gebleichtes Baumwoll-Gewebe wird im Flottenverhältnis 1:20 während 15 Minuten in einer 30°C warmen, wässrigen Weichspülerflotte behandelt, die pro Liter
0,2 g quaternäres Dimethyldistearylammoniumchlorid und
0,01 g des Aufhellers der Formel (101), (200), (600) oder (602) enthält.

Anschliessend wird das Baumwoll-Gewebe während 5 Sekunden in fliessendem Trinkwasser gespült und bei 70°C im Trockenschrank getrocknet. Das so behandelte Baumwoll-Gewebe weist einen guten Aufhelleffekt auf.

**Beispiel 15**: Ein gebleichtes Baumwoll-Gewebe wird im Flottenverhältnis 1:20 während 15 Minuten in einer 40°C warmen, wässrigen Flotte gewaschen, die pro Liter
0,5 g eines Anlagerungsproduktes von 10 Mol Aethylenoxid an einem Mol Stearylalkohol und
0,01 g des Aufhellers der Formel (101), (200), (600), (602) bis (606) enthält.

Anschliessend wird das Baumwoll-Gewebe während 20 Sekunden in fliessendem Trinkwasser gespült und bei 70°C im Trockenschrank getrocknet. Das so behandelte Baumwoll-Gewebe weist einen guten Aufhelleffekt auf.

Fügt man zur vorangehend beschriebenen Flotte noch 0,2 g/l Aktivchlor in Form von Natriumhypochlorit hinzu und verfährt wie beschrieben, so erhält man mit dem Aufheller der Formel (600) ebenso gute Aufhelleffekte.

**Beispiel 16**: Ein Stück Polyamid-Texturtricot wird im Flottenverhältnis 1:20 während 15 Minuten in einer 40°C warmen, wässrigen Flotte gewaschen, die pro Liter
0,5 g eines Anlagerungsproduktes von 10 Mol Aethylenoxid an einem Mol Stearylalkohol und
0,01 g des Aufhellers der Formel (101) oder (200) enthält.

Anschliessend wird das Polyamid-Tricot während 20 Sekunden in fliessendem Trinkwasser gespült und bei 70°C im Trockenschrank getrocknet. Das so behandelte Polyamid-Tricot weist einen guten Aufhelleffekt auf.

**Beispiel 17**: Ein Polyamid-6-Gewbe wird mit einer Flotte fouldiert, welche 1 g/l des Aufhellers der Formel (301), (303) bis (306), (313) oder (401), 1 g/l eines Anlagerungsproduktes von 1 Mol Stearylalkohol an 35 Mol Aethylenoxid, 1 g/1 eines Anlagerungsproduktes von 1 Mol p-tert.-Octylphenol an 8 Mol Aethylenoxid, 90 ml

Aethanol (95 %) und 0,5 g/l Natriumphosphat Puffer enthält. Das Gewebe wird bis zu einer Flottenaufnahme von 85 % abgequetscht. Das so behandelte Gewebe wird nun in einen Färbeapparat gegeben, der so viel Wasser enthält, dass das Flottenverhältnis 1:25 beträgt. Die Applikation erfolgt nun nach folgendem Temperaturprogramm:

50-100°C / 10 Minuten
100°C / 20 Minuten
100- 50°C / 5 Minuten.

Danach wird das Gewebe in kaltem enthärtetem Wasser gespült, zentrifugiert und mit einem 180°C warmen Bügeleisen getrocknet. Das so behandelte Polyamid-6-Gewebe weist einen guten Aufhelleffekt auf.

**Beispiel 18**: Ein Polyamid-6-Gewebe wird mit einer Flotte foulardiert, welche 1 g/l des Aufhellers der Formel (600), (602) bis (606) und (700), 1 g/l eines Anlagerungsproduktes von 1 Mol Stearylalkohol an 35 Mol Aethylenoxid, 1 g/l eines Anlagerungsproduktes von 1 Mol p-tert.-Octylphenol an 8 Mol Aethylenoxid, 90 ml Aethanol (95 %) und 2 g/1 Natriumtripolyphosphat enthält. Das Gewebe wird bis zu einer Flottenaufnahme von 85 % abgequetscht. Das so behandelte Gewebe wird nun in eimen Färbeapparat gegeben, der so viel Wasser enthält, dass das Flottenverhältnis 1:25 beträgt. Die Applikation erfolgt nun nach folgendem Temperaturprogramm:

30-60°C / 10 Minuten
60°C / 20 Minuten

Danach wird das Gewebe in kaltem enthärtetem Wasser gespült, zentrifugiert und mit einem 180°C warmen Bügeleisen getrocknet. Das so behandelte Polyamid-6-Gewebe weist einen guten Aufhelleffekt auf.

**Beispiel 19**: Ein gebleichtes Baumwoll-Gewebe wird mit einer Flotte foulardiert, welche 1 g/1 des Aufhellers der Formel (600), (602) bis (606) oder (700), 1 g/l eines Anlagerungsproduktes von 1 Mol Stearylalkohol an 35 Mol Aethylenoxid, 1 g/l eines Anlagerungsproduktes von 1 Mol p-tert.-Octylphenol an 8 Mol Aethylenoxid, 90 ml Aetha-nol(95%) und 2 g/l Natriumtripolyphosphat. Das Gewebe wird bis zu einer Flottenaufnahme von 75 % abgequetscht. Das so behandelte Gewebe wird nun in einen Färbeapparat gegeben, der so viel Wasser enthält, dass das Flottenverhältnis 1:25 beträgt. Die Applikation erfolgt nun nach folgendem Temperaturprogramm:

30-70°C / 10 Minuten
70°C / 20 Minuten.

Danach wird das Gewebe in kaltem enthärtetem Wasser gespült, zentrifugiert und mit einem 155°C warmen Bügeleisen getrocknet. Das so behandelte Baumwoll-Gewebe weist einen guten Aufhelleffekt auf.

**Beispiel 20**: 5 g Faserstoff (bestehend aus gebleichter Sulfitzellulose und gebleichter Buchenzellulose 1:1) in 50 ml Wasser werden in einem Mixer mit 150 ml Aufhellerlösung, enthaltend 4 mg entsprechend einer Konzentration von 0,08 % des Aufhellers der Formel (101), (200) oder (600) während 15 Minuten gemischt. Anschliessend werden 1,5 Gew.% Leim, z.B. Bowoidleim ® und 2,5 Gew.% Aluminiumsulfat (bezogen auf das Fasertrockengewicht) zugegeben und mit Wasser von ca. 10° dH auf 1000 ml verdünnt. Mit dieser Fasersuspension wird ein Papierblatt gebildet, das einen guten Aufhelleffekt aufweist.

**Beispiel 21**: 5 g Faserstoff (bestehend aus gebleichter Sulfitzellulose und gebleichter Buchenzellulose 1:1 in 150 ml Wasser enthaltend 5 mg eines kationaktiven Polyätheramins werden in einem Mixer mit 50 ml Aufhellerlösung, enthaltend 4 mg entsprechend einer Konzentration von 0,05 % des Aufhellers der Formel (200) oder (600) während 15 Minuten gemischt, Anschliessend werden 1,5 Gew.% Leim, z.B. Bewoidleim ®, 2,5 Gew.% Aluminiumsulfat und 0,1 % eines kationaktiven Polyätheramins (bezogen auf das Fasertrockengewicht) zugegeben und mit Wasser von ca. 10° dH auf 1000 ml verdünnt. Mit dieser Fasersuspension wird ein Papierblatt gebildet, das einen guten Aufhelleffekt aufweist.

**Beispiel 22**: 5 g Faserstoff (bestehend aus gebleichter Sulfitzellulose und gebleichter Buchenzellulose 1:1) in 150 ml Wasser enthaltend 5 mg eines Polyäthylenimins, werden in einem Mixer mit 50 ml Aufhellerlösung, enthaltend 4 mg entsprechend einer Konzentration von 0,08 % des Aufhellers der Formel (200) oder (600) während 15 Minuten gemischt. Anschliessend werden 1,5 Gew. % Leim, z.B. Bewoidleim® 2,5 Gew.% Aluminiumsulfat und 0,1 % eines Polyäthylenimins (bezogen auf das Fasertrockengewicht) zugegeben und mit Wasser von ca. 10° dH auf 1000 ml verdünnt. Mit dieser Fasersuspension wird ein Papierblatt gebildet, das einen guten Aufhelleffekt aufweist.

**Beispiel 23**: Ein konzentriertes flüssiges Waschmittel wird durch Mischen folgender Komponenten hergestellt:

Gew.-%
Aethoxylierte Alkohole
($C_{12}$-$C_{13}$ Alkohol mit 6,5 Mol Aethylenoxid) 60,0
1-Methyl-1-oleylamidoäthyl-2-oleylimidazolinium:
methosulfat 26,7
Verbindung der Formel (101), (200), (600), (602)
bis (606) oder (700) 0,3
Wasser 12,0
Uebliche Zusätze 1,0

2 kg gebleichtes Baumwollgewebe werden während 10 Minuten bei 50°C in 60 Litern Wasser von 100 ppm Härte gewaschen, das 50 bis 60 g des obigen Waschmittels enthalten. Nach dem Spülen und Trocknen weist das Gewebe einen starken Aufhelleffekt und einen weichen Griff auf.

Aehnliche Resultatewurden erhalten, wenn anstelle des oben angegebenen, ein flüssiges Waschmittel folgender Zusammensetzung

Gew.-%
Aethoxylierte Alkohole
($C_{12}$-$C_{13}$ Alkohol mit 6,5 Mol Aethylenoxid) 55,0
1-Methyl-1-talgamidoäthyl-2-talgimidazolinium
methosulfat 26,0
Verbindung der Formel (101), (200), (600), (602) bis 0 3
(606) oder (700)
Wasser 13,0
Isopropanol 5,0
Uebliche Zusätze 0,7

oder ein anderes nicht-ionische Tenside und kationische Substanzen enthaltendes flüssiges Waschmittel, wie z.B. das sich im Handel befindende "Perwoll ® flüssig" oder Samtess ® verwendet wird, denen die erfindungsgemässen Aufheller beigemischt werden.

**Beispiel 24**: Ein flüssiges Waschmittel wird durch Mischen folgender Komponenten hergestellt:

Gew.-%
Aethoxylierte Alkohole ($C_{14}$-$C_{15}$ Alkohol mit
7 Mol Aethylenoxid) 12,0
Aethoxylierte Alkohole ($C_{12}$-$C_{13}$ Alkohol mit
6,5 Mol Aethylenoxid) 12,0
Nicht-gehärtetes Di-talg-dimethyl
ammoniumchlorid 6,4
Aethanol 15,0
Gew.-%
Natriumbicarbonat 0,25
Verbindung der Formel (600), (602) bis (606) oder
(700) 0,41
Uebliche Zusätze 0,41
Wasser 53,53.

Ein wie im Beispiel 23 beschrieben behandeltes Baumwollgewebe weist einen starken Aufhelleffekt und einen weichen Griff auf.

**Beispiel 25**: Eine innige Mischung aus 100 Teilen Polyvinylchlorid, 3 Teilen Stabilisator (Advastat BD 100 ®; Ba/Cd-Komplex), 2 Teilen Titandioxyd, 59 Teilen Dioctylphthalat und 0,01 bis 0,2 Teilen einer Verbindung der Formel (301), (303) bis (306) oder (401) wird auf einem Kalander bei 150 bis 155°C zu einer Folie ausgewalzt. Die so gewonnene opake Polyvinylchloridfolie besitzt einen wesentlich höheren Weissgehalt als eine Folie, welche den optischen Aufheller nicht enthält.

**Patentansprüche**

1. Distyrylbenzole der Formel

(1) 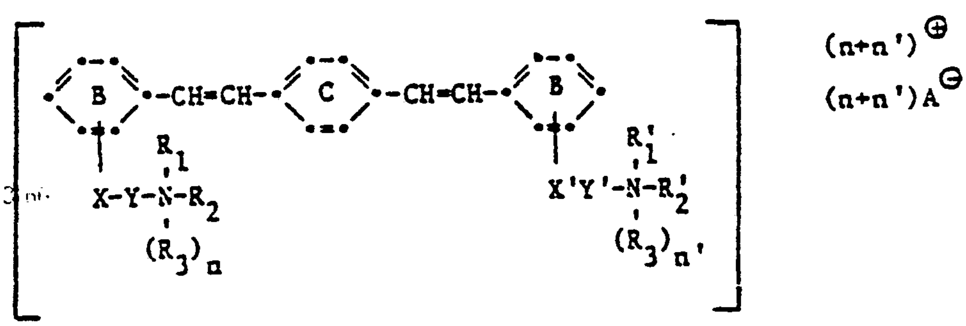

worin X und X' unabhängig voneinander die direkte Bindung, Sauerstoff, Schwefel -O-$C_{1-3}$-alkylen-CON($R_4$)-, -O-$C_{1-3}$-Alkylen-COO- oder -OCO-, Y und Y' unabhängig voneinander $C_{1-20}$-Alkylen oder durch eine Hydroxylgruppe substituiertes $C_{1-20}$-Alkylen, $R_1$ und $R'_1$ unabhängig voneinander unsubstituiertes oder durch Halogen, Cyano, Hydroxy, Alkoxy, Phenyl oder Alkoxycarbonyl mit 1 bis 4 C-Atomen substituiertes $C_{1-8}$-Alkyl, $C_{3-4}$-Alkenyl oder $R_1$ bzw. $R'_1$ zusammen mit $R_2$ bzw. $R'_2$ und dem N-Atom, an das sie gebunden sind, einen 5- bis 7-gliedrigen heterocyclischen Ring, $R_2$ und R' unabhängig voneinander unsubstituiertes oder durch Halogen, Cyano, Hydroxy, Alkoxy, Phenyl oder Alkoxycarbonyl mit 1 bis 4 C-Atomen substituiertes $C_{1-8}$-Alkyl, $C_{3-4}$-Alkenyl oder $R_2$ bzw. $R'_2$ zusammen mit $R_1$ bzw. $R_1$ und dem N-Atom, an das sie gebunden sind, einen 5- bis 7-gliedrigen

heterocyclischen Ring, $R_3$ Wasserstoff, unsubstituiertes oder substituiertes $C_{1-4}$-Alkyl oder $C_{3-4}$-Alkenyl, $R_1$, $R_2$ und $R_3$zusammen mit dem N-Atom, an das sie gebunden sind, bzw. $R'_1$, $R_2'$ und $R_3$ zusammen mit dem N-Atom, an das sie gebunden sind, auch den Pyridin- oder Picolinring, $R_4$ Wasserstoff oder unsubstituiertes oder durch Cyano, Carbamoyl, Carbalkoxy, Hydroxy, Halogen oder Alkoxy mit 1 bis 4 C-Atomen substituiertes C $_6$-Alkyl, A ⊖ ein farbloses Anion und n und n' unabhängig voneinander die Zahl 0 oder 1, bedeuten, wobei die Benzolkerne B und C auch nicht-chromophor substituiert sein können, und worin die Gruppierungen

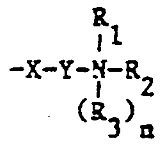 sowie 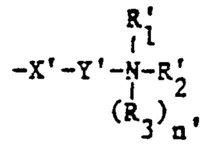

im Falle, dass die Ringe B und C keine nicht-chromophoren Substituenten tragen, auch beide jeweils

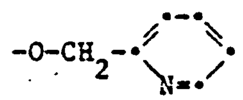

oder beide jeweils

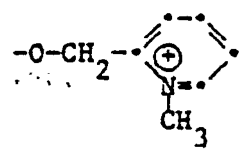

in den ortho-Stellungen zu den beiden Gruppen -CH = CH- sein können.

2. Distyrylbenzole gemäss Anspruch 1 der Formel

(2) 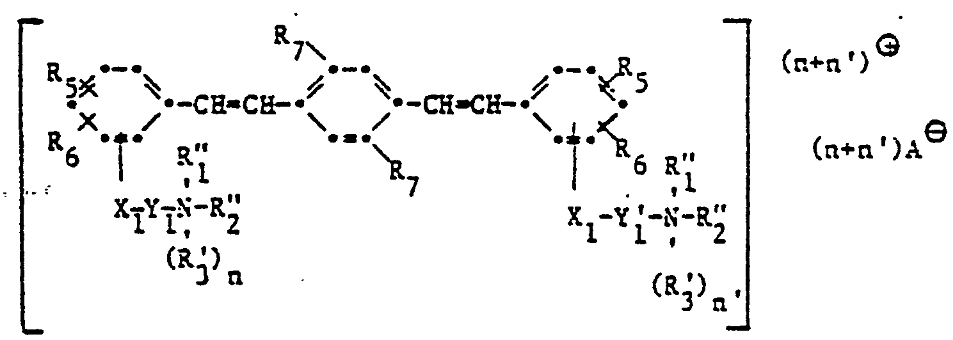

worin $X_1$ die direkte Bindung, Sauerstoff, Schwefel, -O-$C_{1-3}$-Alkylen-CONH-, -O-$C_{1-3}$-Alkylen-COO- oder -OCO-, $Y_1$ und $Y_1$ unabhängig voneinander $C_{1-4}$-Alkylen oder Hydroxypropylen, $R'_1$' und $R'_2$' unabhängig vomeinander $C_{1-4}$-Alkyl, oder $R'_1$' und $R_2''$ zusammen mit dem N-Atom, an das sie gebunden sind, einen Pyrrolidin-, Piperidin-, Hexamethylenimin- oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_{2-4}$-Hydroxyalkyl oder $C_{2-4}$-Cyanoalkyl, $R''_1$, $R''_2$ und R' zusammen mit dem N-Atom, an das sie gebunden sind, auch den Pyridin- oder Picolinring, $R_5$ Wasserstoff, Chlor, $C_{1-4}$-Alkyl, $C_{3-4}$-Alkenyl, $C_{1-3}$-Alkoxy oder zusammen mit $R_6$ eine Trimethylen- oder Tetramethylengruppe, $R_6$ Wasserstoff, Chlor, $C_{1-4}$-Alkyl, $C_{1-3}$-Alkoxy oder zusammen mit $R_5$ eineTrimethylen- oder Tetramethylengruppe, $R_7$ Wasserstoff, Chlor oder Methyl, n und n' unabhängig voneinander die Zahl 0 oder 1 und A ⊖ eim farbloses Anion bedeuten.

3. Distyrylbenzole gemäss Anspruch 1 der Formel

(3) 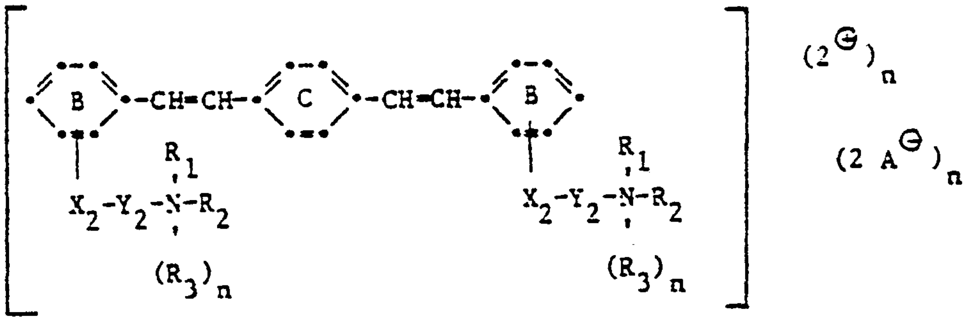

worin $X_2$ Sauerstoff, Schwefel oder -OCO-, $Y_2C_{1-20}$-Alkylen oder durch eine Hydroxylgruppe substituiertes $C_{1-20}$ -Alkylen $R_1$ unsubstituiertes oder durch Halogen, Cyano, Hydroxy, Alkoxy, Phenyl oder Alkoxycarbonyl mit 1 bis 4 C-Atomen substituiertes $C_{1-8}$-Alkyl, $C_{3-4}$-Alkenyl oder $R_1$ zusammen mit $R_2$ und dem N-Atom, an das sie gebunden sind, einen 5- bis 7-gliedrigen heterocyclischen Ring, $R_2$ unsubstituiertes oder durch Halogen,Cyano, Hydroxy, Alkoxy Phenyl oder Alkoxycarbonyl mit 1 bis 4 C-Atomen substituiertes $C_{1-8}$-Alkyl, $C_{3-4}$-Alkenyl oder $R_2$ zusammen mit $R_1$ und dem N-Atom, an das sie gebunden sind, einen 5- bis 7-gliedrigenä heterocyclischen Ring, $R_3$ Wasserstoff, unsubstituiertes oder substituiertes $C_{1-4}$-Alkyl oder $C_{3-4}$-Alkenyl, $R_1$, $R_2$ und $R_3$ zusammen mit dem N-Atom, an das sie gebunden sind, auch den Pyridin-oder Picolinring,$R_4$ Wasserstoff oder unsubstituiertes oder durch Cyano,Carbamoyl, Carbalkoxy, Hydroxy, Halogen oder Alkoxy mit 1 bis 4 C-Atomen substituiertes $C_{1-6}$-Alkyl, n die Zahl 0 oder 1 und A⊖ ein farbloses Anion bedeuten, wobei die Benzolkerne B und C auch nicht-chromophor substituiert sein können.

4. Distyrylbenzole gemäss Anspruch 2 der Formel

(4) 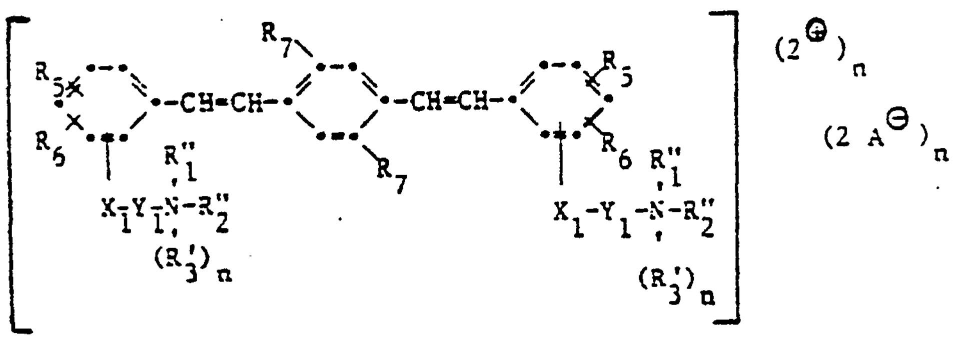

worin $X_1$ die direkte Bindung, Bauerstoff, Schwefel, -O-$C_{1-3}$-Alkylen-CONH-, -O-$C_{1-3}$-Alkylen-COO- oder -OCO-, $Y_1$ $C_{1-4}$-Alkylen oder Hydroxypropylen, $R''_1$ und $R''_2$ unabhängig voneinander $C_{1-4}$-Alkyl oder $R_1''$ und $R''_2$ mit dem N-Atom, in das sie gebunden sind, einen Pyrrolidin-, Piperidin-, Hexamethylenimin- oder Morpholinring, $R_3'$ Wasserstoff, $C_{1-4}$-Alkyl, $C_{3-4}$-Alkenyl, $C_{1-3}$-Alkoxycarbonylmethyl, Benzyl, $C_{2-4}$-Hydroxyalkyl oder $C_{2-4}$-Cyanoalkyl, $R'_1$', $R'_2$' und $R_3'$ zusammen mit dem N-Atom, an das sie gebunden sind, auch den Pyridin-oder Picolinring, $R_5$ Wasserstoff, Chlor, $C_{1-4}$-Alkyl, $C_3$ -Alkenyl, $C_{1-3}$-Alkoxy oder zusammen mit $R_6$ eime Trimethylem- oder Tetramethylengruppe, $R_6$ Wasserstoff, Chlor, $C_{1-4}$-alkyl, $C_{1-3}$-Alkoxy oder zusammen mit $R_5$ eine Trimethylen- oder Tetramethylengruppe, $R_7$ Wasserstoff, Chlor oder Methyl, n die Zahl 0 oder 1 und A ⊖ ein farbloses Anion bedeuten.

5. Distyrylbenzole gemäss Anspruch 4 der Formel

(5) 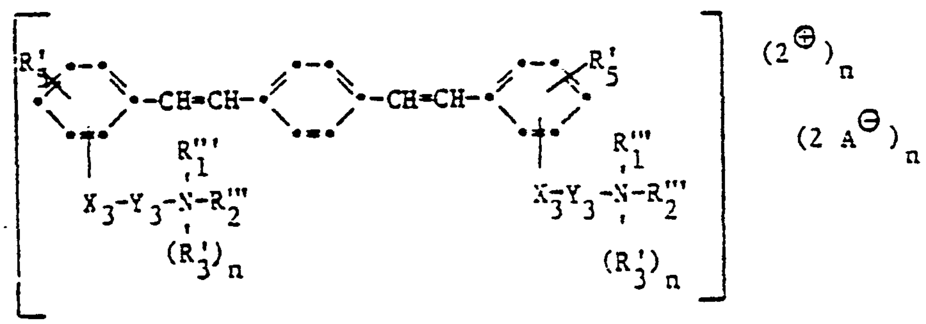

worin $X_3$ die direkte Bindung, Sauerstoff, Schwefel oder -OCO- Y $C_{1-4}$-Alkylen, $R_1''$ und $R_2''$ unabhängig voneinander $C_{1-4}$-Alkyloder $R_1''$ und $R_2''$ zusammen mit dem N-Atom, an das sie gebunden sind, einen $C_{3-4}$-Alkenyl, $C_{1-3}$-Alkoxycarbonylmethyl, Benzyl, $C_{2-4}$-Hydroxyalkyloder $C_{2-4}$-Cyanoalkyl, $R'_1''$, $R'_2''$ und $R_3'$ zusammen mit dem N-Atom,an das sie gebunden sind, auch den Pyridinring, $R_5'$ Wasserstoff, Chlor, $C_{1-4}$-Alkyl oder $C_{1-3}$-Alkoxy, n die Zahl 0 oder 1 und A ⊖ ein farbloses Anion bedeuten.

6. Distyrylbenzole gemäss Anspruch 4 der Formel

(6) 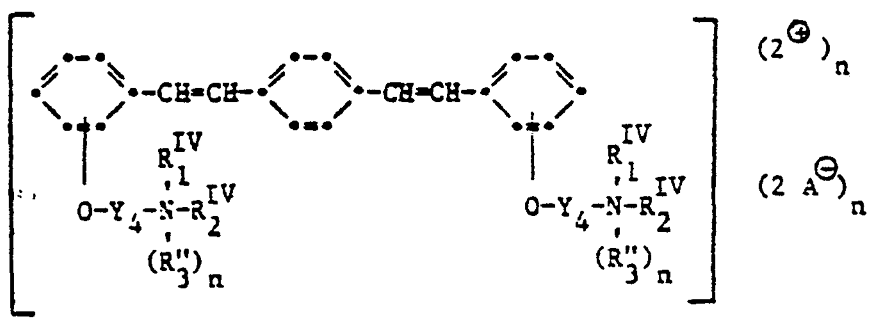

worin $Y_4$ $C_{2-4}$-Alkylen, $R_1^{IV}$ $C_{1-3}$-Alkyl oder $R_1^{IV}$ zusammen mit $R_2^{IV}$ und dem N-Atom, an das sie gebunden sind, einen Pyrrolidin-, Piperidin-, Hexanethylenimin- oder Morpholinring, $R_2^{IV}$ $C_{1-3}$ -Alkyl oder $R_2^{IV}$ zusammen mit $R_1^{IV}$ und dem N-Atom, an das sie gebunden sind, einen Pyrrolidin-, Piperidin-, Hexamethylenimin- oder Morpholinring, $R_3''$ Wasserstoff, oder $C_{1-3}$-Alkyl, n die Zahl 0 oder 1 und A ⊖ ein farbloses Anion bedeuten.

7. Distyrylbenzole gemäss Anspruch 3 der Formel

(7) 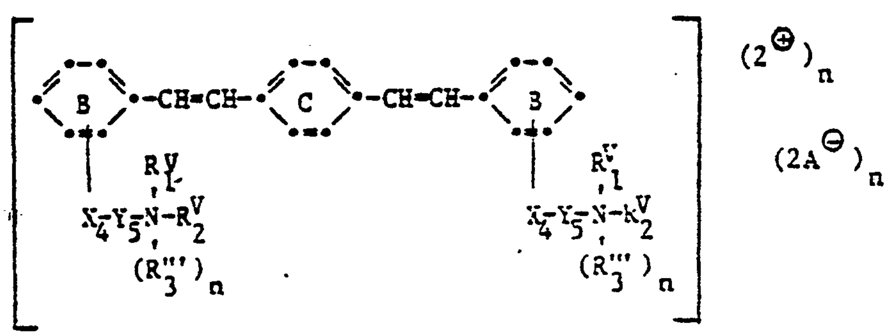

worin $X_4$ Sauerstoff, Schwefel oder -OCO, $Y_5$ $C_{2-20}$-Alkylen, $R_1^V$ unsubstituiertes oder durch Halogen, Cyano, Hydroxy, Alkoxy, Phenyl oder Alkoxycarbonyl mit 1 bis 4 C-Atomen substituiertes $C_{1-8}$-alkyl, $C_{3-4}$-Alkenyl oder $R_1^V$ zusammen mit $R_2$ und dem N-Atom, an das sie gebunden sind,einen 5- bis 7-gliedrigen heterocyclischen Ring, $R_2$ unsubstituiertes oder durch Halogen, Cyano, Hydroxy, Alkoxy, Phenyl oder Alkoxycarbonyl mit 1 bis 4, C-atomen substituiertes $C_{1-8}$-Alkyl, $C_{3-4}$-Alkenyl oder $R_2^V$ zusammen mit $R_1$ und dem N-Atom, an das sie gebunden sind, einen 5- bis 7-gliedrigen heterocyclischen Ring, $R'_3''$ Wasserstoff oder unsubstituiertes oder substituiertes

$C_{1-4}$-Alkyl, $R_4$ Wasserstoff oder unsubstituiertes oder durch Cyano, Carbamoyl, Carbalkoxy, Hydroxy, Halogen oder Alkoxy mit 1 bis 4 C-Atomen substituiertes C $_{1-6}$-Alkyl, A⊖ ein farbloses Anion und n die Zahl 0 oder 1 bedeuten, wobei die Benzolkerne B und C auch nicht-chromophor substituiert sein können.

8. Distyrylbenzole nach Anspruch 7 der Formel

(3) 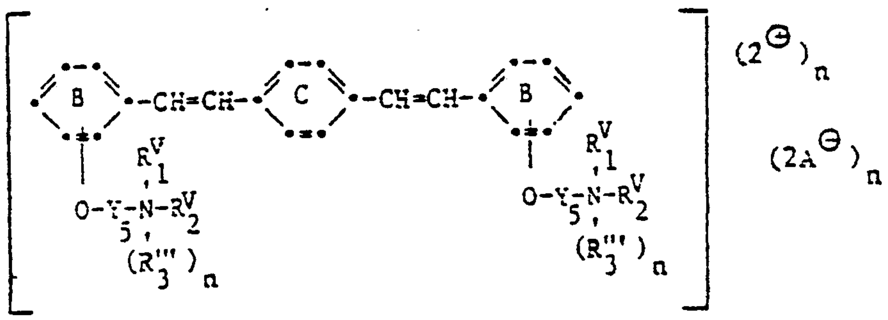

worin $Y_5$ $C_{2-20}$-Alkylen, $R_1$ unsubstituiertes oder durch Halogen Cyano Hydroxy, Alkoxy, Phenyl oder Alkoxycarbonyl mit 1 bis 4 C-Atomen substituiertes $C_{1-8}$-Alkyl, $C_{3-4}$-Alkenyl oder zusammen mit $R_2$ und dem N-Atom, an das sie gebunden sind, einen 5- bis 7-gliedrigen heterocyclischen Ring, $R_2$ unsubstituiertes oder durch Halogen Cyano, Hydroxy, Alkoxy, Phenyl oder Alkoxycarbonyl mit 1 bis 4 C-Atomen substituiertes $C_{1-4}$-Alkyl, $C_{3-4}$-Alkenyl oder zusammen mit $R_1^V$ und dem N-Atom, an das sie gebunden sind, einen 5- bis 7-gliedrigen heterocyclischen Ring, $R_3''$ Wasserstoff oder unsubstituiertes oder substituiertes $C_{1-4}$-Alkyl, A ⊖ ein farbloses Anion und n die Zahl 0 oder 1 bedeuten, wobei die Benzolkerne B und C auch nicht-chromophor substituiert sein können.

9. Distyrylbenzol nach Anspruch 8 der Formel

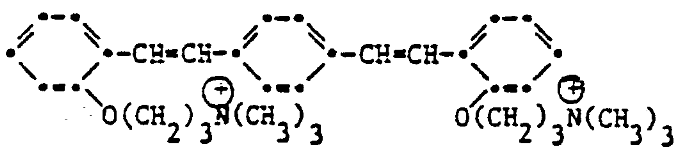

$2\ CH_3OSO_3^{\ominus}$

10. Distyrylbenzol nach Anspruch 8 der Formel

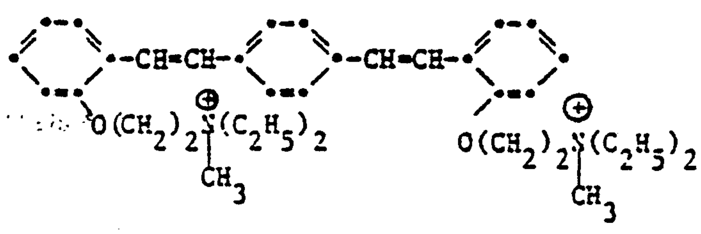

$2\ CH_3OSO_3^{\ominus}$

11. Distyrylbenzol nach Anspruch 8 der Formel

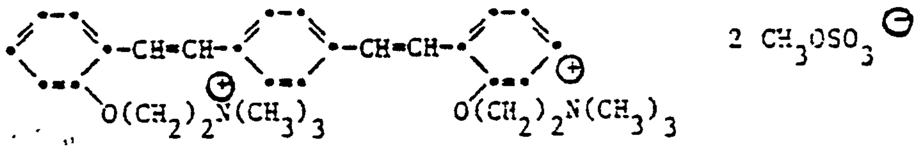

12. Verfahren zur Herstellung von Distyrylbenzolen der Formel

(1) 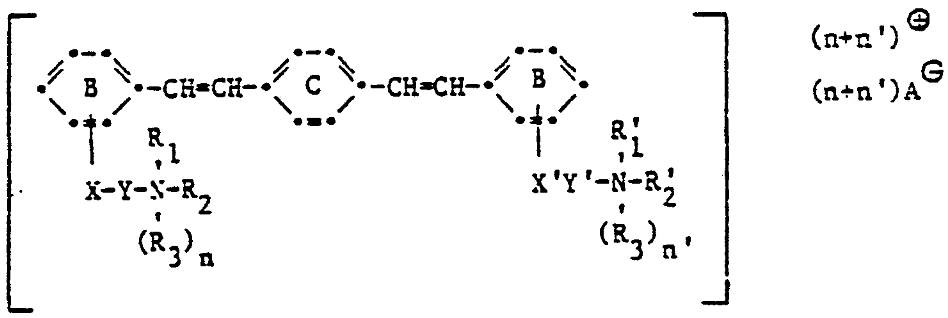

worin X und X' unabhängig voneinander die direkte Bindung, Sauerstoff, Schwefel, -O-$C_{1-3}$-Alkylen-CON($R_4$)-, -O-$C_{1-3}$-Alkylen-COO- oder -OCO-, Y und Y' unabhängig voneinander $C_{1-20}$-Alkylen oder durch eine Hydroxylgruppe substituiertes C,$_{20}$-Alkylen, $R_1$ und $R'_1$ unabhängig voneinander unsubstituiertes oder durch Halogen, Cyano, Hydroxy, Alkoxy, Phenyl oder Alkoxvcarbonyl mit 1 bis 4 C-Atomem substituiertes $C_{1-8}$-Alkyl, $C_{3-4}$-Alkenyl oder $R_1$ bzw. $R'_1$ zusammen mit $R_2$ bzw. $R_2'$ und dem N-Atom, an das sie gebunden sind, einen 5- bis 7-gliedrigen heterocyclischen Ring, $R_2$ und $R'_2$ unabhängig voneinander unsubstituiertes oder durch Halogen, Cyano, Hydroxy, Alkoxy, Phenyl oder Alkoxycarbonyl mit 1 bis 4 C-Atomen substituiertes $C_{1-8}$-Alkyl. $C_{3-4}$-Alkenyl oder $R_2$ bzw. R') zusammen mit $R_1$ bzw. $R'_1$ und de N-Atom, an das sie gebunden sind, einen 5- bis 7-gliedrigen heterocyclischen Ring, $R_3$ Wasserstoff, unsubstituiertes oder substituiertes $C_{1-4}$-Alkyl oder $C_{3-4}$-Alkenyl,$R_1$, $R_2$ und $R_3$ zusammen mit dem N-Atom,an das sie gebunden sind, bzw. $R_1'$, $R_2'$ und $R_3$ zusammenmit dem N-Atom, an das sie gebunden sind, auch den Pyridin- oder Picolinring, $R_4$ Wasserstoff oder unsubstituiertes oder durch Cyano, Carbamoyl, Carbalkoxy, Hydroxy, Halogen oder Alkoxy mit 1 bis 4 C-Atomen substituiertes $C_{1-6}$-Alkyl, $A^{\ominus}$ ein farbloses Anion und n und n' unabhängig voneinander die Zahl 0 oder 1 bedeuten, wobei die Benzolkerne B und C auch nicht-chromophor substituiert sein können und worin die Gruppierungen

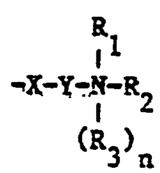

sowie

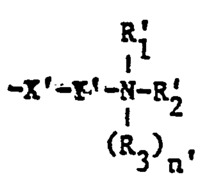

im Falle dass die Ringe B und C keine nicht-chromophoren Substituenten tragen, auch beide jeweils

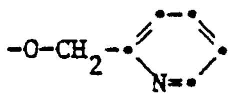

oder beide jeweils

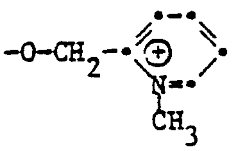

in den ortho-Stellungen zu den beiden Gruppen -CH=CH- sein können, dadurch gekennzeichnet, dass man 1 Moläquivalent eines Distyrylbenzols der Formel

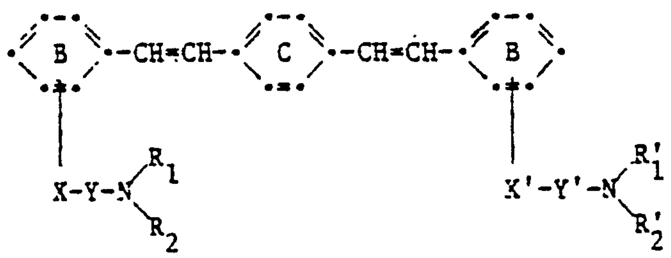

mit 1 oder 2 Moläquivalenten eines Alkylierungsmittels bzw. einer Säure der Formel $R_3$-A quaterniert bzw. protoniert, wobei in dieser Formel die Benzolkerne B und C und X, X', Y, Y', $R_1$, $R'_1$, $R_2$, $R'_2$, $R_3$ und A die oben angegebene Bedeutung haben.

13. Verfahren zur Herstellung von Distyrylbenzolen der Formel

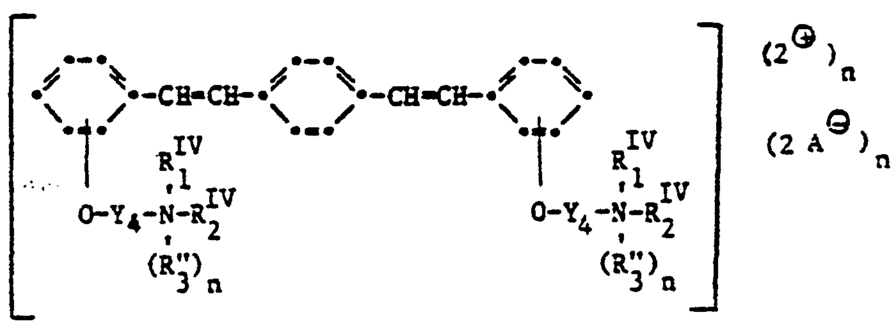

worin $Y_4$ $C_{2-4}$-Alkylen, $R_1^{IV}$ $C_{1-3}$-Alkyl oder $R_1^{IV}$ zusammem mit $R_2^{IV}$ und dem N-Atom, an das sie gebunden sind, einen Pyrrolidin-, Piperidin-, Hexamethylenimin- oder Morpholinring $R^{IV}_2$ $C_{1-3}$-Alkyl oder $R_2^{IV}$ zusammen mit $R_1^{IV}$ und dem N-Atom, an das sie gebunden sind, einen Pyrrolidin-, Piperidin-, Hexamethylenimin- oder Morpholinring, $R_3''$ Wasserstoff, oder $C_{1-3}$-Alkyl, n die Zahl 0 oder 1 und A ⊖ ein farbloses Anion bedeuten dadurch gekennzeichnet, dass man im Molekularverhältnis 1:2 eine Verbindung der Formel

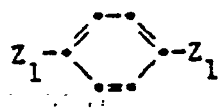

mit solchen der Formel

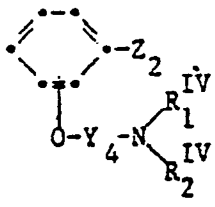

in Gegenwart einer starken Base umsetzt, in welchen Formeln $Y_4$, $R_1^{IV}$ und $R_2^{IV}$ die oben angegebene Bedeutung haben und eines der Symbole $Z_1$ und $Z_2$ eine OCH-Gruppe und das andere eine Gruppierung der Formel

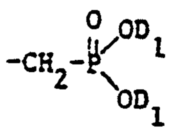
,
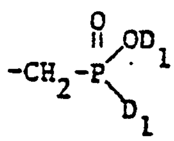
,

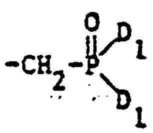
oder
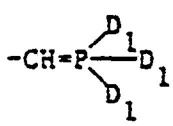

bedeutet, worin $D_1$ einen unsubstituierten oder substituierten Alkyl-, Aryl-, Cycloalkyl- oder Aralkylrest darstellt und gewünschtenfalls die erhaltene Verbindung der Formel

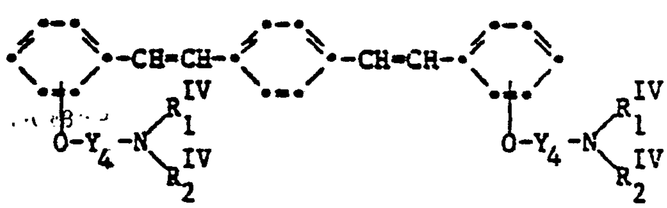

mit 2 Moläquivalenten eines Alkylierungsmittels bzw. einer Säure der Formel $R_3''$-A quaterniert bzw. protoniert, wobei in diesen Formeln $Y_4$, $R_1^{IV}$, $R_2^{IV}$, $R_3''$ und A die oben angegebene Bedeutung haben.

14. Verwendung von den in einem der Ansprüche 1 bis 11 definierten Verbindungen zum optischen Aufhellen von organischen Materialien.

15. Verwendung nach Anspruch 14 zum optischen Aufhellen von Polyacrylnitril und Cellulose als organischem Material.

16. Waschmittel, enthaltend ein oder mehrere Verbindungen der im Anspruch 1 definierten Formel 1.

17. Waschmittel gemäss Anspruch 16, welches 10 bis 70 Gew.-% eines nichtionogenen Tensides und 1 bis 30 Gew.% eines kationischen Textilweichmachers enthält.

18. Waschmittel gemäss Anspruch 17 welches 10 bis 70 Gew.% eines nichtionogenen Tensides und 1 bis 30 Gew.% eines quaternären Derivates des Ammoniaks und/oder des Imidizolins mit 2 langkettigen, aliphatischen gesättigten oder ungesättigten Resten als kationischen Textilweichmacher und, zur Verleihung der flüssigen Form, noch ein Lösungsmittel enthält.

19. Waschmittel nach Anspruch 18, welches 1-Methyl-1-oleylamidoäthyl-2-oleyl-imidazolinium.X' ⊖ 1-Methyl-1-talgamidoäthyl-2-talgimidazol-inium.X ⊖, Di-talg-dimethyl-ammonium-X⊖ oder ein Verbindungen der Formel

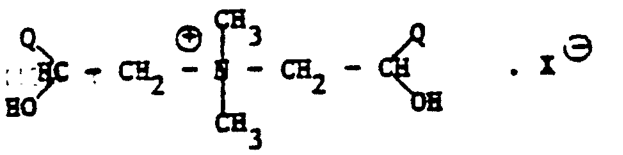

worin Q $C_{14\text{-}16}$-Alkyl bedeutet und X⊖ für ein Chlorid-, Bromid-, Methylsulfat-, Aethylsulfat-, Methansulfonat, Aethansulfonat-oder Toluolsulfonat-Anion steht, als kationischen Textilweichmacher enthält.

20. Textilbehandlungsmittel, enthaltend eine oder mehrere Verbindungen der im Anspruch 1 definierten Formel 1.

21. Wäschenachbehandlungsmittel, enthaltend eine oder mehrere Verbindungen der im Anspruch 1 definierten Formel 1 und einen kationischen Textilweichmacher.

**Claims**

1. A distyrylbenzene of the formula

(1) 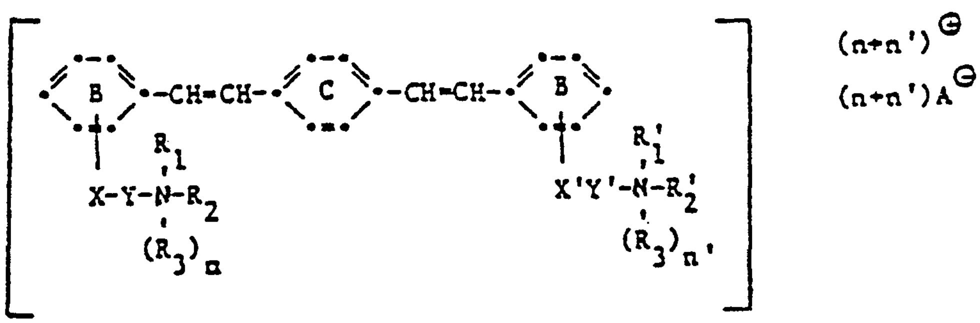

in which X and X' independently of each other are a direct bond, oxygen, sulfur, - O-$C_{1\text{-}3}$alkylene- CON ($R_4$) -, -O-$C_{1\text{-}3}$alkylene-COO- or -OCO-, Y and Y' independently of each other are $C_{1\text{-}20}$alkylene or $C_{1\text{-}20}$alkylene which is substituted by a hydroxyl group, $R_1$ and $R_1$' independently of each other are $C_{1\text{-}8}$alkyl which is unsubstituted or substituted by halogen, cyano, hydroxy, alkoxy, phenyl or $C_{1\text{-}4}$alkoxycarbonyl, or are $C_{3\text{-}4}$alkenyl, or $R_1$ together with $R_2$ and the nitrogen atom to which they are attached is a 5- to 7-membered heterocyclic ring and/or $R_1$' together with $R_2$' and the nitrogen atom to which they are attached is a 5- to 7-membered heterocyclic ring, $R_2$ and $R_2$' independently of each other are $C_{1\text{-}8}$alkyl which is unsubstituted or substituted by halogen, cyano, hydroxy, alkoxy, phenyl or $C_{1\text{-}4}$alkoxycarbonyl, or are $C_{3\text{-}4}$alkenyl, or $R_2$ together with $R_1$ and the nitrogen atom to which they are attached is a 5- to 7-membered heterocyclic ring and/or $R_2$' together with $R_1$' and the nitrogen atom to which they are attached is a 5- to 7-membered heterocyclic ring, $R_3$ is hydrogen, unsubstituted or substituted $C_{1\text{-}4}$alkyl, or is $C_{3\text{-}4}$alkenyl, or $R_1$, $R_2$ and $R_3$ together with the nitrogen atom to which they are attached are a pyridine or picoline ring and/or $R_1$', $R_2$' and $R_3$ together with the nitrogen atom to which they are attached are a pyridine or picoline ring $R_4$ is hydrogen or $C_{1\text{-}6}$alkyl which is unsubstituted or substituted by cyano, carbamoyl, carbalkoxy, hydroxy, halogen or $C_{1\text{-}4}$alkoxy, A⊖ is a colourless anion and n and n' independently of each other are 0 or 1, and the benzene nuclei B and C may also be substituted by non-chromophoric substituents, and in which, if the nuclei B and C do not carry non-chromophoric substituents, the substituents, the

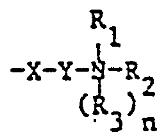

and

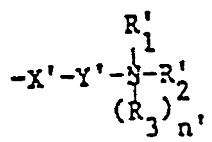

groups,
as well as each of the two

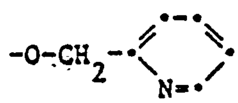

groups or
each of the two

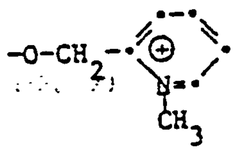

groups, may be ortho to the two -CH=CH- groups.

2. A dystyrylbenzene according to claim 1 of the formula

(2) 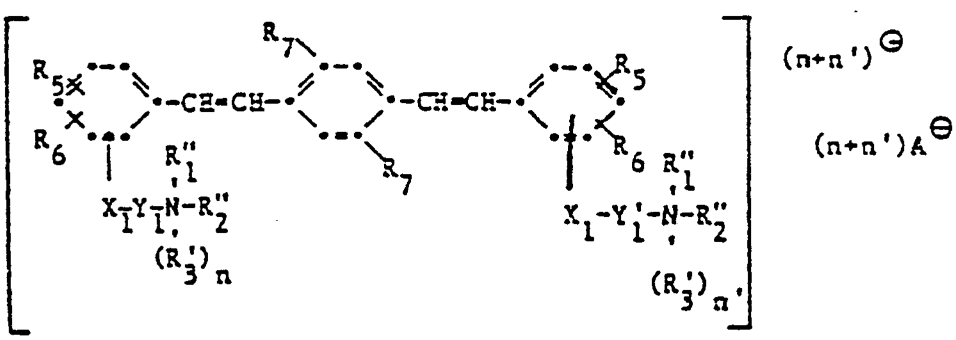

in which $X_1$ is a direct bond, oxygen, sulfur, -O-$C_{1-3}$ alkylene-CONH-, -O-$C_{1-3}$alkylene-COO- or -OCO-, $Y_1$ and $Y_1'$ independently of each other are $C_{1-4}$ alkylene or hydroxypropylene, $R_1''$ and $R_2''$ independently of each other are $C_{1-4}$alkyl, or $R_1''$ and $R_2''$ together with the nitrogen atom to which they are attached are a pyrrolidine, piperidine, hexamethyleneimine or morpholine ring, $R_3'$ is hydrogen, $C_{1-4}$alkyl, $C_{3-4}$ alkenyl, $C_{1-3}$ alkoxycarbonylmethyl benzyl, $C_{2-4}$hydroxy-alkyl or $C_{2-4}$cyanoalkyl, or $R_1''$ $R_2''$ and $R_3'$ together with the nitrogen atom to which they are attached are a pyridine or picoline ring, $R_5$ is hydrogen, chlorine, $C_{1-4}$alkyl, $C_{3-4}$alkenyl or $C_{1-3}$alkoxy, or $R_5$ together with $R_6$ is a trimethylene or tetramethylene group, $R_6$ is hydrogen, chlorine, $C_{1-4}$alkyl or $C_{1-3}$alkoxy, or $R_6$ together with $R_5$ is a trimethylene or tetraethylene group, $R_7$ is hydrogen, chlorine or methyl, n and n' independently of each other are 0 or 1 and A ⊖ is a colourless anion.

3. A dystyrylbenzene according to claim 1 of the formula

(3) 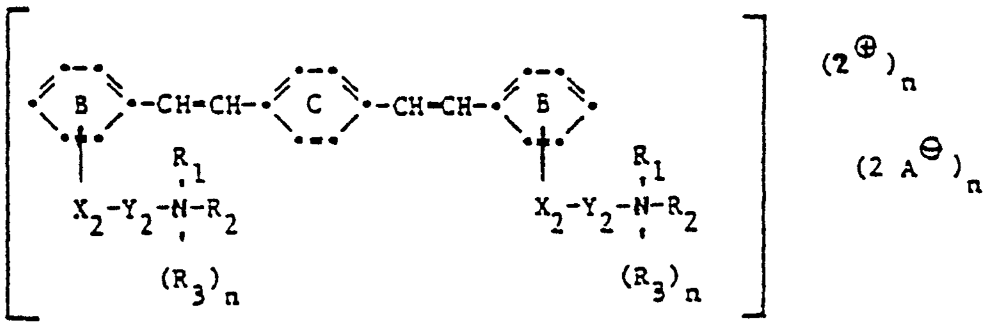

in which $X_2$ is oxygen, sulfur or -OCO-, $Y_2$ is $C_{1-20}$alkylene or $C_{1-20}$alkylene which is substituted by a hydroxyl group, $R_1$ is $C_{1-8}$alkyl which is unsubstituted or substituted by halogen, cyano, hydroxy, alkoxy, phenyl or $C_{1-4}$alkoxy-carbonyl, or is $C_{3-4}$alkenyl, or $R_1$ together with $R_2$ and the nitrogen atom to which they are attached is a 5- to 7-membered heterocyclic ring, $R_2$ is $C_{1-8}$alkyl which is unsubstituted or substituted by halogen, cyano, hydroxy, alkoxy, phenyl or $C_{1-4}$alkoxycarbonyl, or is $C_{3-4}$alkenyl, or $R_2$ together with $R_1$ and the nitrogen atom to which they are attached is a 5- to 7-membered heterocyclic ring, $R_3$ is hydrogen, unsubstituted or substituted $C_1$ $^4$ alkyl, or is $C_{3-4}$alkenyl, or $R_1$, $R_2$ and R together with the nitrogen to which they are attached are a pyridine or picoline ring, $R_4$ is hydrogen or $C_{1-6}$alkyl which is unsubstituted or substituted by cyano, carbamoyl, carbalkoxy, hydroxy, halogen or $C_{1-4}$alkoxy, n is 0 or 1 and A⊖ is a colourless anion, and the benzene nuclei B and C may also be substituted by non-chromophoric substituents.

4. A distyrylbenzene according to claim 2 of the formula

(4) 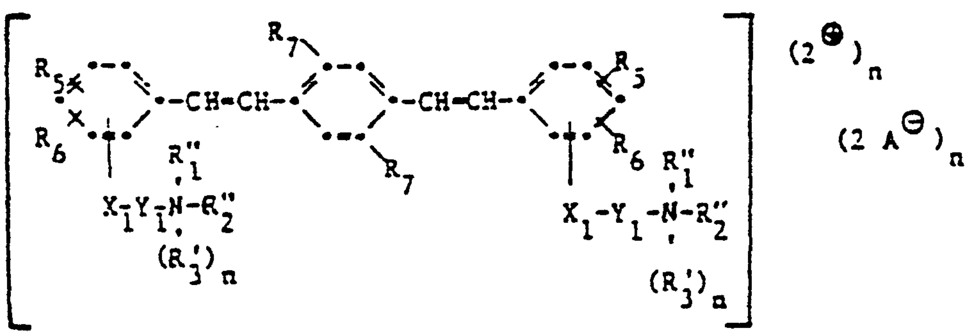

in which $X_1$ is a direct bond, oxygen, sulfur, -O-$C_{13}$-alkylene CONH-, -O-$C_{1-3}$alkylene-COO- or -OCO-, $Y_1$ is $C_{1-4}$alkylene or hydroxypropylene, $R_1''$ and $R_2''$ independently of each other are $C_{1-4}$alkyl, or $R_1''$ and $R_2''$ together with the nitrogen atom to which they are attached are a pyrrolidine, piperidine, hexamethyleneimine or morpholine ring, $R_3'$ is hydrogen, $C_{1-4}$alkyl, $C_{3-4}$alkenyl, $C_{1-3}$ alkoxycarbonylmethyl, benzyl, $C_{2-4}$hydroxy-alkyl or $C_{2-4}$cyanoalkyl, or $R_1''$, $R_2''$ and $R_3'$ together with the nitrogen atom to which they are attached are a pyridine or picoline ring, $R_5$ is hydrogen, chlorine, $C_{1-4}$-alkyl, $C_{3-4}$alkenyl or $C_{1-3}$alkoxy, or $R_5$ together with $R_6$ is a trimethylene or tetramethylene group, $R_6$ is hydrogen, chlorine, $C_{1-4}$alkyl or $C_{1-3}$alkoxy, or $R_6$ together with $R_5$ is a trimethylene or tetramethylene group, $R_7$ is hydrogen chlorine or methyl, n is 0 or 1 and A⊖ is a colourless anion.

5. A distyrylbenzene according to claim 4 of the formula

(5) 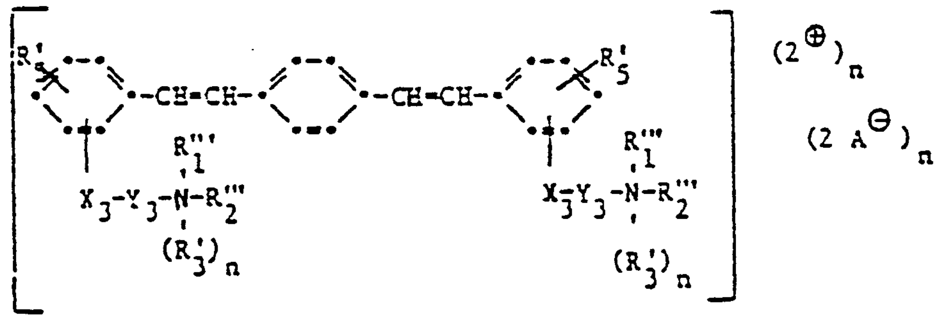

in which $X_3$ is a direct bond, oxygen, sulfur or -OCO-, $Y_3$ is $C_1$-alkylene, $R'_1$'' and $R'_2$'' independently of each other are $C_{1\text{-}4}$alkyl, or $R'_1$'' and $R'_2$'' together with the nitrogen atom to which they are attached are a pyrrolidine, piperidine or morpholine ring, $R_3$' is hydrogen, $C_{1\text{-}4}$alkyl, $C_{3\text{-}4}$alkenyl, $C_{1\text{-}3}$alkoxycarbonylmethyl, benzyl, $C_{2\text{-}4}$hydroxyalkyl or $C_{2\text{-}4}$ cyanoalkyl,or $R'_1$'',$R'_2$'' and $R_3$' together with the nitrogen atom to which they are attached are a pyridine ring, $R_5$' is hydrogen, chlorine, $C_{1\text{-}4}$alkyl or $C_{1\text{-}3}$alkoxy, n is 0 or 1 and $A^{\ominus}$ is a colourless anion.

6. A dystyrylbenzene according to claim 4 of the formula

(6) 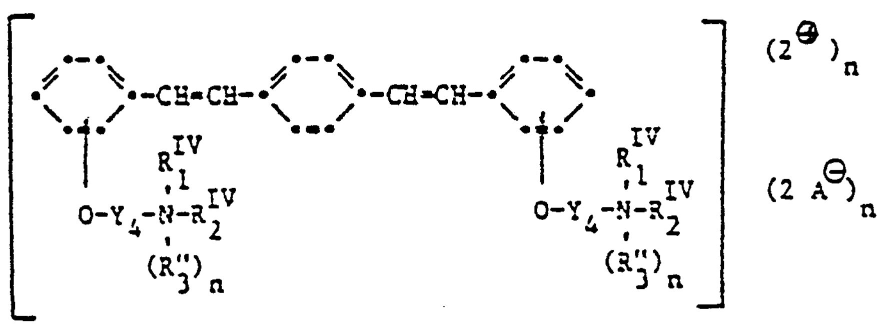

in which $Y_4$ is $C_{2\text{-}4}$alkylene, $R^{IV}_1$ is $C_{1\text{-}3}$alkyl or $R^{IV}$together with $R_2^{IV}$ and the nitrogen atom to which they are attached is a pyrrolidine, piperidine, hexamethyleneimine or morpholine ring, $R^{IV}_2$ is $C_{1\text{-}3}$alkyl, or $R^{IV}_2$ together with $R^{IV}_1$ and the nitrogen atom to which they are attached is a pyrrolidine, piperidine, hexamethyleneimine or morpholine ring, $R_3$'' is hydrogen or $C_{1\text{-}3}$alkyl, n is 0 or 1 and A $\ominus$ is a colourless anion.

7. A distyrylbenzene according to claim 3 of the formula

(7) 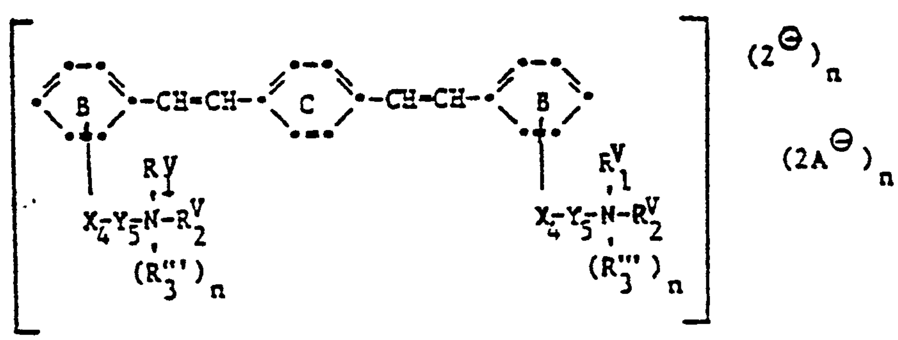

in which $X_4$ is oxygen, sulfur or -OCO-, $Y_5$ is $C_{2\text{-}20}$alkylene, $R^V_1$ is $C_{1\text{-}8}$alkyl which is unsubstituted or substituted by halogen, cyano, hydroxy, alkoxy, phenyl or $C_{1\text{-}4}$ alkoxycarbonyl, or is $C_{3\text{-}4}$alkenyl, or $R^V_1$ together with $R^V_2$ and the nitrogen atom to which they are attached is a 5- to 7-membered heterocyclic ring, $R_2^V$ is $C_{1\text{-}8}$ alkyl which is unsubstituted or substituted by halogen, cyano, hydroxy, alkoxy, phenyl or $C_{1\text{-}4}$ alkoxycarbonyl, or is $C_{3\text{-}4}$alkenyl, or $R^V_2$ together with $R^V_1$ and the nitrogen atom to which they are attached is a 5- to 7-membered

heterocyclic ring, R'3'' is hydrogen or unsubstituted or substituted $C_{1-4}$alkyl, $R_4$ is hydrogen or $C_{1-6}$alkyl which is unsubstituted or substituted by cyano, carbamoyl, carbalkoxy, hydroxy, halogen or $C_{1-4}$alkoxy, $A^{\ominus}$ is a colourless anion and n is 0 or 1, and the benzene nuclei 8 and C may also be substituted by non-chromophoric substituents.

8. A distyrylbenzene according to claim 7 of the formula

(8) 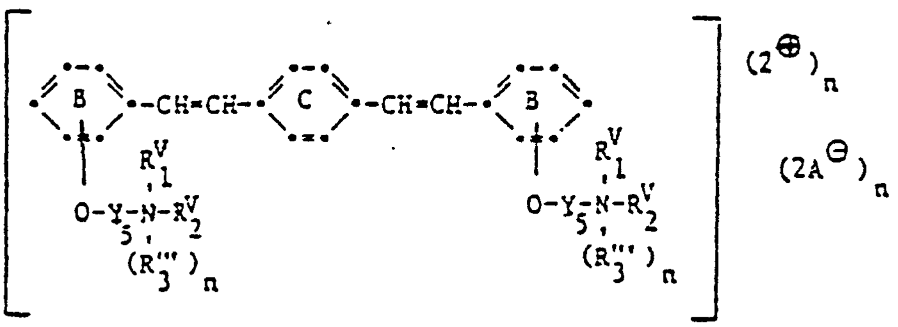

in which $Y_5$ is $C_{2-20}$alkylene, $R^V{}_1$ is $C_{1-8}$alkyl which is unsubstituted or substituted by halogen, cyano, hydroxy, alkoxy, phenyl or $C_{1-4}$alkoxycarbonyl, or is $C_{3-4}$alkenyl, or $R^V{}_1$ together with $R^V{}_2$ and the nitrogen atom to which they are attached is a 5- to 7-membered heterocyclic ring, $R^V{}_2$ is $C_{1-8}$alkyl which is unsubstituted or substituted by halogen, cyano, hydroxy, alkoxy, phenyl or $C_{1-4}$alkoxycarbonyl, or is $C_{3-4}$alkenyl, or $R^V{}_2$ together with $R^V{}_1$ and the nitrogen atom to which they are attached is a 5- to 7-membered heterocyclic ring, R'3'' is hydrogen or unsubstituted or substituted $C_{1-4}$alkyl, $A\ominus$ is a colourless anion and n is 0 or 1, and the benzene nuclei B and C may also be substituted by nonchromophoric substituents.

9. A distyrylbenzene according to claim 8 of the formula

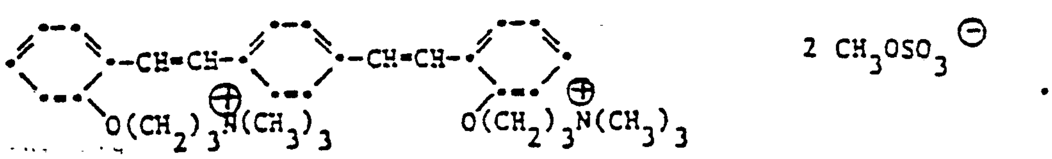

10. A distyrylbenzene according to claim 8 of the formula

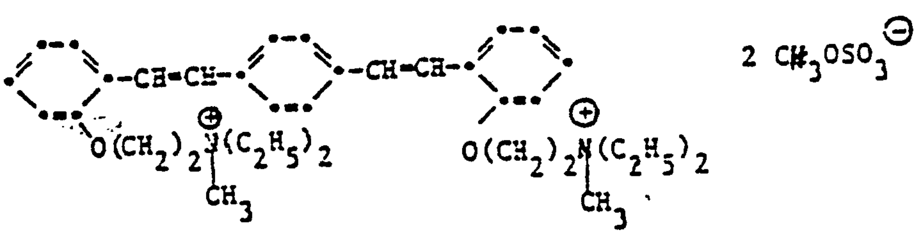

11. A distyrylbenzene according to claim 8 of the formula

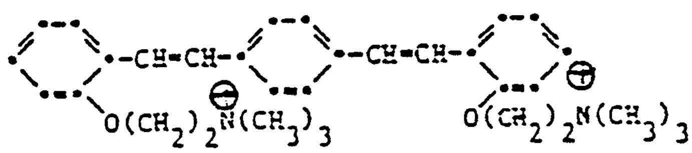

$2\ CH_3OSO_3^{\ominus}$

12. A process for the preparation of a distyrylbenzene of the formula

(1) 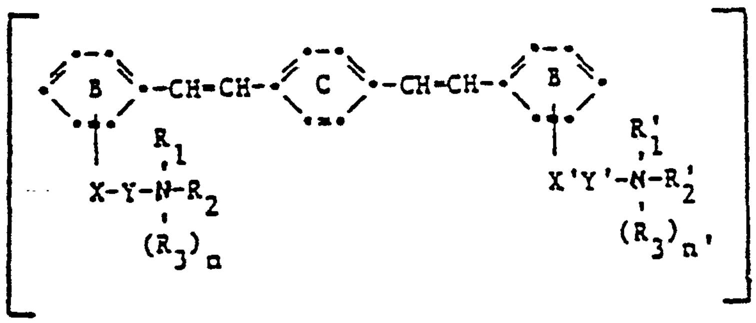

$(n+n')^{\oplus}$
$(n+n')A^{\ominus}$

in which X and X' independently of each other are a direct bond, oxygen, sulfur, - O-$C_{1\text{-}3}$alkyl ene-CON ($R_4$) -, - O-$C_{1\text{-}3}$alkylene-COO- or -OCO-, Y and Y' independently of each other are $C_{1\text{-}20}$alkylene or $C_{1\text{-}20}$alkylene which is substituted by a hydroxyl group, $R_1$ and $R_1'$ independently of each other are $C_{1\text{-}8}$alkyl which is unsubstituted or substituted by halogen, cyano, hydroxy, alkoxy, phenyl or $C_{1\text{-}4}$alkoxycarbonyl, or are $C_{3\text{-}4}$alkenyl, or $R_1$ together with $R_2$ and the nitrogen atom to which they are attached is a 5- to 7-membered heterocyclic ring and/or $R_1'$ together with $R_2'$ and the nitrogen atom to which they are attached is a 5- to 7-membered heterocyclic ring, $R_2$ and $R_2'$ independently of each other are $C_{1\text{-}8}$alkyl which is unsubstituted or substituted by halogen, cyano, hydroxy, alkoxy, phenyl or $C_{1\text{-}4}$alkoxycarbonyl, or are $C_{3\text{-}4}$alkenyl, or $R_2$ together with $R_1$ and the nitrogen atom to which they are attached is a 5- to 7-membered heterocyclic ring and/or $R_2'$ together with $R_1'$ and the nitrogen atom to which they are attached is a 5- to 7-membered heterocyclic ring, $R_3$ is hydrogen, unsubstituted or substituted $C_{1\text{-}4}$alkyl, or is $C_{3\text{-}4}$alkenyl, or $R_1$, $R_2$ and $R_3$ together with the nitrogen atom to which they are attached are a pyridine or picoline ring and/or $R_1'$, $R_2'$ and $R_3$ together with the nitrogen atom to which they are attached are a pyridine or picoline ring, $R_4$ is hydrogen or $C_{1\text{-}6}$alkyl which is unsubstituted or substituted by cyano, carbamoyl, carbalkoxy, hydroxy, halogen or $C_{1\text{-}4}$alkoxy, $A^{\ominus}$ is a colourless anion and n and n' independently of each other are 0 or 1, and the benzene nuclei B and C may also be substituted by non-chromophoric substituents, and in which, if the nuclei B and C do not carry non-chromophoric substituents, the

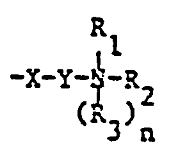

and

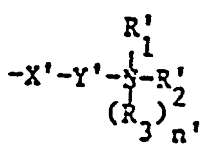

groups,
as well as each of the two

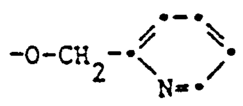

groups or each of the two

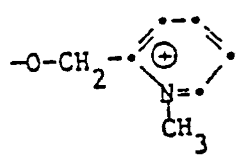

groups, may be ortho to the two -CH=CH- groups, which process comprises quaternising or protonising 1 mol equivalent of a dystyrylbenzene of the formula

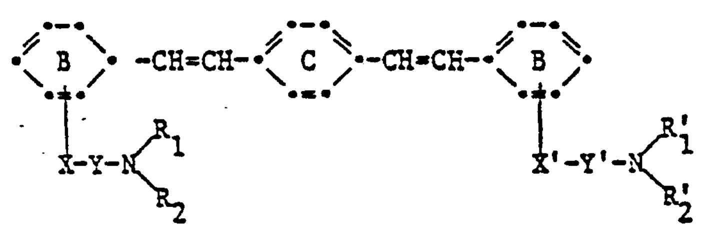

in which the benzenenuclei B and C and X, X', Y, Y', $R_1$, $R_1'$, $R_2$ and $R_2'$ are as defined above, with 1 mol equivalent or with 2 mol equivalents of an alkylating agent or, respectively, of an acid of the formula $R_3$-A, in which $R_3$ and A are as defined above.

13. A process for the preparation of a dystyrylbenzene of the formula

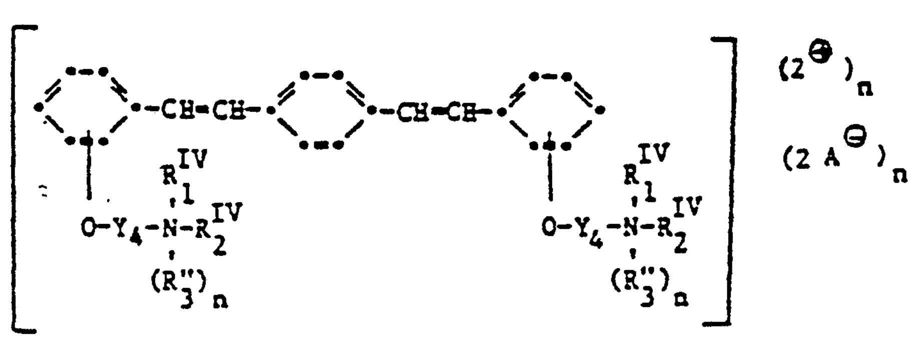

in which $Y_4$ is $C_{2-4}$alkylene, $R_1^{IV}$ is $C_{1-3}$alkyl or $R_1^{IV}$ together with $R^{IV}_2$ and the nitrogen atom to which they are attached is a pyrrolidine, piperidine, hexamethyleneimine or morpholine ring, $R_2^{IV}$ is $C_{1-3}$alkyl, or $R^{IV}_2$ together with $R^{IV}_1$ and the nitrogen atom to which they are attached is a pyrrolidine, piperidine, hexamethyleneimine or morpholine ring, $R_3''$ is hydrogen or $C_{1-3}$alkyl, n is 0 or 1 and $A^{\ominus}$ is a colourless anion, which process comprises reacting a compound of the formula

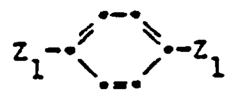

in a molecular ratio of 1:2 with a compound of the formula

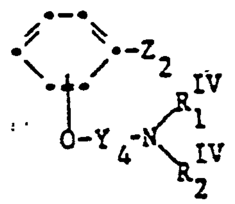

in the presence of a strong base, in which formulae $Y_4$ $R_1^{IV}$ and $R_2^{IV}$ are as defined above and one of the symbols $Z_1$ and $Z_2$ is an OCH group and the other is a grouping of the formula

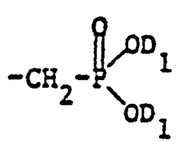

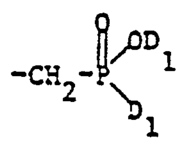

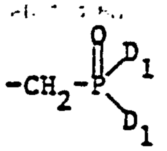

or

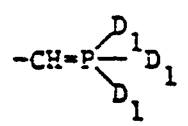

in which formulae $D_1$ is an unsubstituted or substituted alkyl, aryl, cycloalkyl or aralkyl radical, and, if desired, quaternising or protonising the resultant compound of the formula

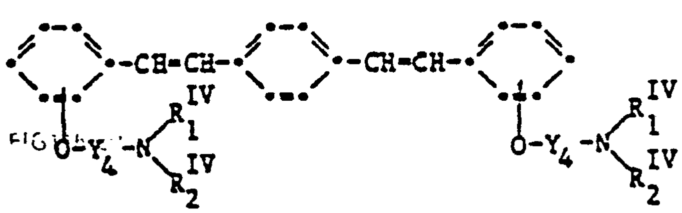

in which $Y_4$, $R^{IV}_1$ and $R^{IV}_2$ are as defined above, with 2 mol equivalents of an alkylating agent or respectively of an acid of the formula $R_3''$-A, in which $R_3''$ and A are as defined above.

14. Use of a compound as defined in any one of claims 1 to 11 for brightening organic material.

15. Use according to claim 14, wherein the organic material to be brightened is polyacrylonitrile or cellulose.

16. A detergent containing one or more compounds of the formula (1) as defined in claim 1.

17. A detergent according to claim 16, which contains 10 to 70 % by weight of a nonionic surfactant and 1 to 30 % of a cationic textile softener.

18. A detergent according to claim 17, which contains 10 to 70 % by weight of a nonionic surfactant and 1 to 30 % by weight of a quaternary derivative of ammonia and/or of imidazoline, each containing 2 long chain aliphatic radicals, as cationic textile softener, and a solvent to render it in the liquid form.

19. A detergent according to claim 18, which contains 1-methyl-1oleylamidoethyl-2-oleyl-imidazolinium.X⊖, 1-methyl-1-tallow-amidoethyl-2-tallow-imidazolinium.X⊖, di-tallow-dimethyl-ammonium.X⊖ or a compound of the formula

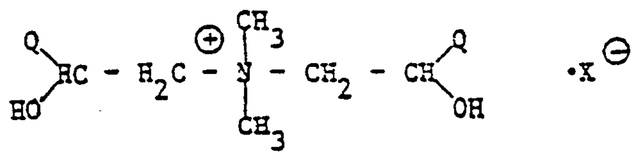

in which Q is $C_{14-16}$ alkyl and X ⊖ is a chloride, bromide, methylsulfate, ethylsulfate, methanesulfonate, ethanesulfonate or toluenesulfonate anion, as cationic textile softener.

20. A textile treatment agent containing one or more compounds of the formula (1) as defined in claim 1.

21. A laundry after-treatment agent containing one or more compounds of the formula (1) as defined in claim 1 and a cationic textile softener.

**Revendications**

1. Distyrylbenzènes de formule

(1) 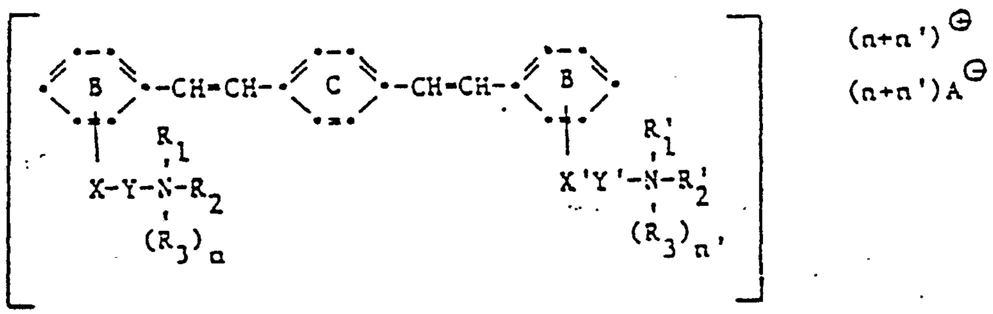

dans laquelle X et X', indépendamment l'un de l'autre, sont chacun la liaison directe, un atome d'oxygène, de soufre, un radical -O-(alkylène en $C_{1-3}$)-CON($R_4$)-, -O-(alkylène en $C_{1-3}$)-COO- ou -OCO-, Y et Y', indépendamment l'un de l'autre, sont chacun un radical alkylène en $C_{1-20}$ ou alkylène en $C_{1-20}$ substitué par un groupe hydroxyle, $R_1$ et $R_1'$, indépendamment l'un de l'autre, sont chacun un radical alkyle en $C_{1-8}$ alcényle en $C_{34}$ non-substitué ou substitué par un halogène ou par un radical cyano, hydroxy, alcoxy, phényle ou alcoxycarbonyle ayant 1 à 4 atomes de carbone, ou bien $R_1$ ou $R_1'$, respectivement avec $R_2$ ou $R_2'$, et avec l'atome N auquel ils sont fixés forment un noyau hétérocyclique pentagonal à heptagonal, $R_2$ et $R_2'$, indépendamment l'un de l'autre, sont chacun un radical alkyle en $C_{1-8}$ alcényle en $C_{3-4}$ non-substitué ou substitué par un halogène un radical cyano, hydroxy, alcoxy, phényle ou alcoxycarbonyle ayant de 1 à 4 atomes de carbone, ou bien $R_2$ ou $R_2'$, respectivement avec R ou $R_1'$, et avec l'atome N auquel ils sont liés forment un noyau hétérocyclique pentagonal à heptagonal, $R_3$ est l'hydrogène, un radical alkyle en $C_{1-4}$ ou alcényle en $C_{3-4}$, non-substitue ou substituer $R_1$, $R_2$ et $R_3$, avec l'atome N auauel ils sont liés, ou encore $R_1'$, $R_2'$ et $R_3$, avec l'atome N auquel ils sont liés, forment aussi le noyau pyridine ou picoline, $R_4$ est l'hydrogène ou un radical alkyle en $C_{1-6}$ substitué par un radical cyano, carbamoyle, carbalcoxy, halogène ou alcoxy ayant de 1 à 4 atomes de carbone, A ⊖ est un anion incolore, et n et n', indépendamment l'un de l'autre, sont chacun le nombre 0 ou 1, les noyaux benzéniques B et C pouvant aussi être substitués par des substituants non-chromophores, et où les groupements

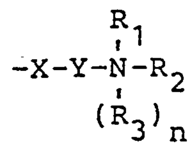

et

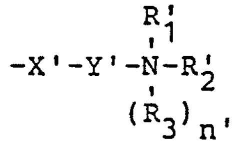

peuvent tous les deux, dans le cas où les noyaux B et C ne portent pas de substituants non-chromophores, être chacun le radical

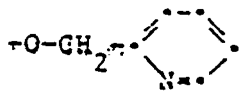

ou chacun le radical

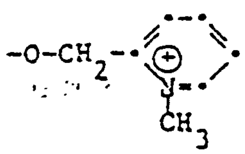

sur les positions ortho par rapport aux deux groupes -CH=CH-.

2. Distyrylbenzènes selon la revendication 1, de formule

(2) 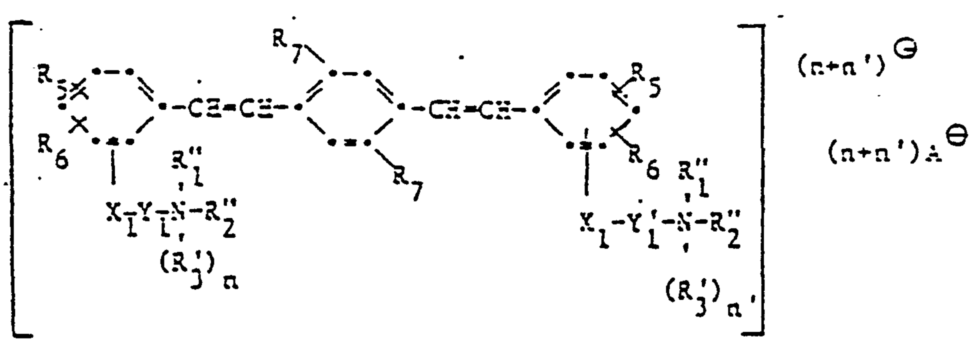

dans laquelle $X_1$ est la liaison directe, un atome d'oxygène, de soufre, un radical -O-(alkylene en $C_{1-3}$)-CONH-, -O-(alkylene en $C_{1-3}$)-COO- ou -OCO-, $Y_1$ et $Y_1'$, indépendamment l'un de l'autre, sont chacun un radical alkylène en $C_{1-4}$ ou hydroxypropylene, $R'_1$' et $R_2''$, indépendamment l'un de l'autre, sont chacun un radical alkyle en $C_{1-4}$, ou bien $R''_1$ et $R''_2$, avec l'atome N auquel ils sont liés, forment un noyau pyrrolidine, pipéridine, hexaméthylenimine ou morpholine, $R_3'$ est l'hydrogène, un radical alkyle en $C_{1-4}$, alcényle en $C_{3-4}$, (alcoxy en $C_{1-3}$)-carbonylméthyle, benzyle, hydroxyalkyle en $C_{2-4}$ ou cyanoalkyle en $C_{2-4}$, $R_1'$, $R_2''$ et $R'_3$, avec l'atome N auquel ils sont liés, forment aussi le noyau pyridine ou picoline, $R_5$ est l'hydrogène, le chlore, un radical alkyle en $C_{1-4}$, alcényle en $C_{3-4}$, alcoxy en $C_{1-3}$ ou bien, avec $R_6$, forme un enchaînement triméthylene ou tétraméthylène, $R_6$ est l'hydrogène, le chlore, un radical alkyle en $C_{1-4}$, alcoxy en $C_{1-3}$ ou forme avec $R_5$ un enchainement triméthylene ou tétraméthylene, $R_7$ est l'hydrogène, le chlore ou le radical méthyle, n et n', indépendamment l'un de l'autre, sont chacun le nombre 0 ou 1 et A⊖ est un anion incolore.

3. Distyrylbenzènes selon la revendication 1 de formule

(3) 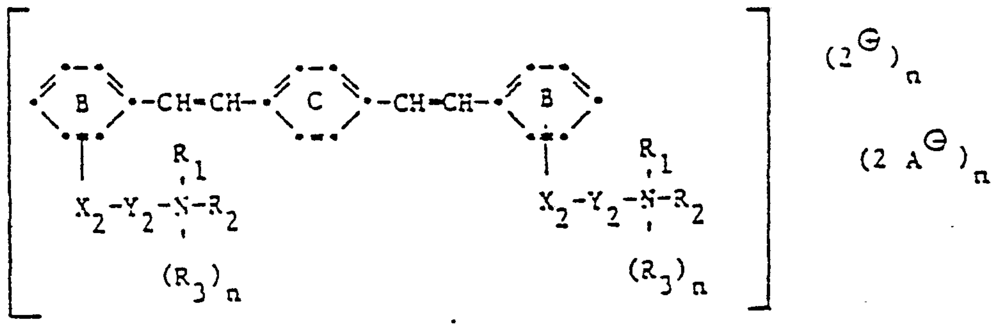

dans laquelle $X_2$ est l'oxygène, le soufre ou un radical -OCO-, $Y_2$ est un radical alkylene en $C_{1-20}$ ou alkylène en $C_{1-20}$ substitué par un groupe hydroxyle, $R_1$ est un radical alkyle en $C_{1-8}$ alcényle en $C_{3-4}$ non-substitué ou substitué par un halogène, un groupe cyano, hydroxy, alcoxy, phényle ou alcoxycarbonyle ayant 1 à 4 atomes de carbone, ou bien $R_1$, avec $R_2$ et l'atome N auquel ils sont liés, forme un noyau hétérocyclique pentagonal à heptagonal, $R_2$ est un radical alkyle en $C_{1-8}$ alcényle en $C_{3-4}$,non-substitué ou substitué par un halogène, un groupe cyano, hydroxy, alcoxy, phényle ou alcoxycarbonyle ayant de 1 à 4 atomes de carbone, ou bien $R_2$, avec $R_1$ et l'atome N auquel ils sont liés, forme un noyau hétérocyclique pentagonal à heptagonal, $R_3$ est l'hydrogène, un radical alkyle en $C_{1-4}$ ou alcényle en $C_{3-4}$, non-substitué ou substitué, ou $R_1$, $R_2$ et $R_3$, avec l'atome N auquel ils sont liés, forment aussi le noyau pyridine ou picoline, $R_4$ est l'hydrogène ou un radical alkyle en $C_{1-6}$ non-substitué ou substitué par un groupe cyano, carbamoyle, carbalcoxy, hydroxy, halogène ou alcoxy ayant de 1 à 4 atomes de carbone, n est le nombre 0 ou 1 et A⊖ est un anion incolore, les noyaux benzéniques B et C pouvant aussi être substitués par des substituants non-chromophores.

4. Distyrylbenzènes selon la revendication 2, de formule

(4) 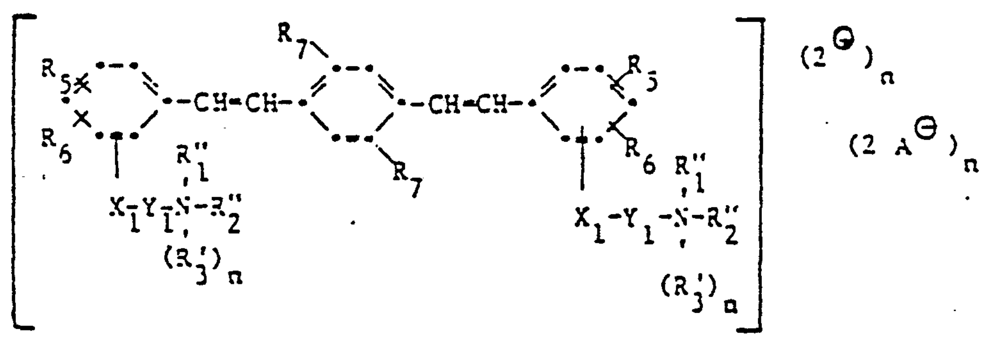

dans laquelle $X_1$ est la liaison directe, l'oxygène, le soufre, un radical -O-(alkylène en $C_{1-3}$)-CONH-, -O-(alkylène en $C_{1-3}$)-COO- ou -OCO-, $Y_1$ est un radical alkylène en $C_{1-4}$ ou hydroxypropylène, $R_1$' et $R_2$'', indépendamment l'un de l'autre, sont chacun un radical alkyle en $C_{1-4}$, ou bien R'' et R''$_2$, avec l'atome N auquel ils sont liés, forment un noyau pyrrolidine, pipéridine, hexaméthylènimine ou morpholine, $R_3$' est l'hydrogène, un radical alkyle en $C_{1-4}$, alcenyle en $C_{3-4}$, (alcoxy en $C_{1-3}$)-carbonylmethyle, benzyle, hydroxyalkyle en $C_{2-4}$ ou cyanoalkyle en $C_{2-4}$, R''$_1$, R''$_2$ et $R_3$', avec l'atome N auquel ils sont liés, forment aussi le noyau pyridine ou picoline, $R_5$ est l'hydrogène, le chlore, un radical alkyle en $C_{1-4}$, alcényle en $C_{3-4}$, alcoxy en $C_{1-3}$ ou forme avec $R_6$ un enchaînement triméthylène ou tétraméthylène, $R_6$ est l'hydrogène, le chlore, un radical alkyle en $C_{1-4}$, alcoxy en $C_{1-3}$ ou forme avec $R_5$ un enchainement triméthylène ou tétraméthylène, $R_7$ est l'hydrogène, le chlore ou le radical méthyle, n est le nombre 0 ou 1, et A⊖ est un anion incolore.

5. Distyrylbenzènes selon la revendication 4, de formule

(5) 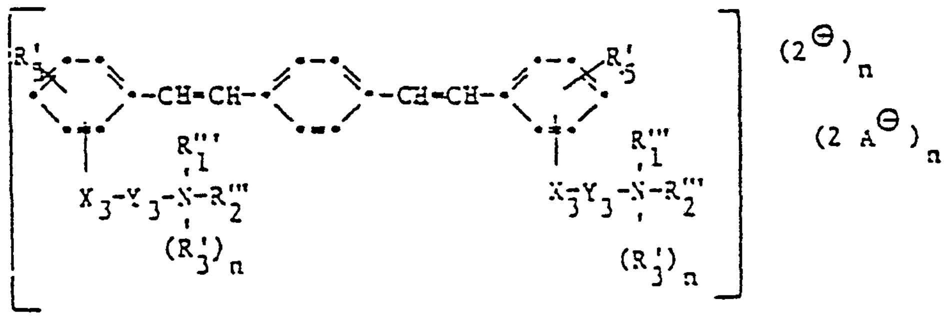

dans laquelle $X_3$ est la liaison directe, l'oxygène, le soufre ou -OCO-, $Y_3$ est-un radical alkylène en $C_{1-4}$, $R'''_1$ et $R'_2''$, indépendamment l'un de l'autre, sont chacun un radical alkyle en $C_{1-4}$, ou bien $R'_1''$ et $R_2'''$, avec l'atome N auquel ils sont liés, forment un noyau pyrrolidine, pipéridine ou morpholine, $R_3'$ est l'hydrogène, un radical alkyle en $C_{1-4}$, alcényle en $C_{3-4}$, (alcoxy en $C_{1-3}$)-carbonylméthyle, benzyle, hydroxyalkyle en $C_{2-4}$ ou cyanoalkyle en $C_{2-4}$, $R'_1''$ $R_2'''$ et $R_3'$ forment aussi avec l'atome N auquel ils sont liés le noyau pyridine, $R_5'$ est l'hydrogène, le chlore, un radical alkyle en $C_{1-4}$ ou alcoxy en $C_{1-3}$, n est le nombre 0 ou 1, et A⊖ est un anion incolore.

6. Distyrylbenzènes selon la revendication 4, de formule

(6) 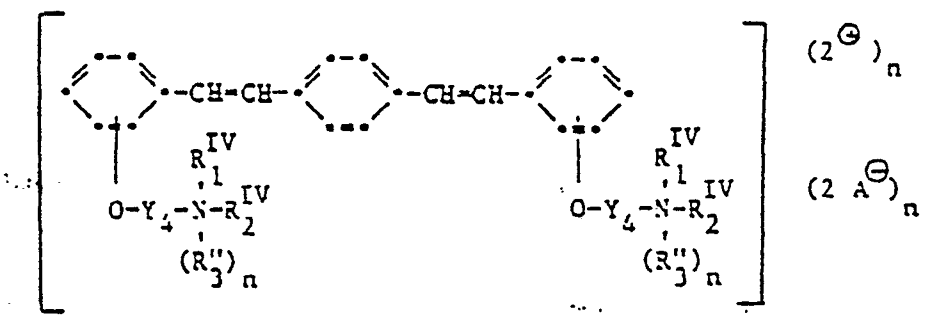

dans laquelle $Y_4$ est un radical alkylène en $C_{2-4}$, $R_1^{IV}$ est un radical alkyle en $C_{1-3}$, ou bien $R_1^{IV}$,-avec $R_2^{IV}$ et l'atomeN auquel ils sont liés, forme un noyau pyrrolidine, pipéridine, hexaméthylènimine ou morpholine, $R_2^{IV}$ est un radical alkyle en $C_{1-3}$, ou bien $R_2^{IV}$, avec $R_1^{IV}$ et l'atome N auquel ils sont liés, forme un noyau pyrrolidine, pipéridine, hexaméthylènimine ou morpholine, $R'''_3$ est l'hydrogène ou un radcal alkyle en $C_{1-3}$, n est le nombre 0 ou 1, et A ⊖ est un anion incolore.

7. Distyrylbenzènes selon la revendication 3, de formule

(7) 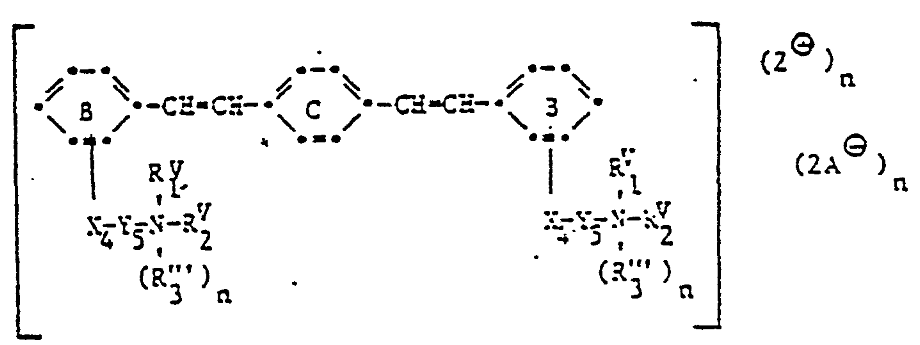

dans laquelle $X_4$ est l'oxygène, le soufre ou -OCO-, $Y_5$ est un radical alkylène en $C_{2-20}$, $R_1^V$ est un radical alkyle en $C_{1-8}$ ou alcényle en $C_{3-4}$,non-substitué ou substitué par un halogène, un groupe cyano, hydroxyle, alcoxy, phényle ou alcoxycarbonyle ayant 1 à 4 atomes de carbone, ou bien $R_1^V$ forme, avec $R_2^V$ et l'atome N auquel ils sont liés, un noyau hétérocyclique pentagonal ou heptagonal, $R_2^V$ est un radical alkyle en $C_{1-8}$ ou alcényle en

$C_{3-4}$,non-substitué ou substitué par un halogène ou par un groupe cyano, hydroxyle, alcoxy, phényle ou alcoxycarbonyle ayant de 1 à 4 atomes de carbone, ou bien $R_2^V$ forme,avec $R_1^V$ et l'atome N auquel ils sont liés, un noyau hétérocyclique pentagonal à heptagonal, $R'''_3$ est l'hydrogène ou un radical alkyle en $C_{1-4}$ substitué ou non-substitué, $R_4$ est l'hydrogène ou un radical alkyle en $C_{1-6}$ nonsubstitué ou substitué par un groupe cyano, carbamoyle, carbalcoxy, hydroxyle, halogène ou alcoxy ayant de 1 à 4 atomes de carbone, $A^{\ominus}$ est un anion incolore, et n est le nombre 0 ou 1, les noyaux benzéniques B et C pouvant aussi être substitués par des substituants non-chromophores.

8. Distyrylbenzènes selon la revendication 7, de formule

(3) 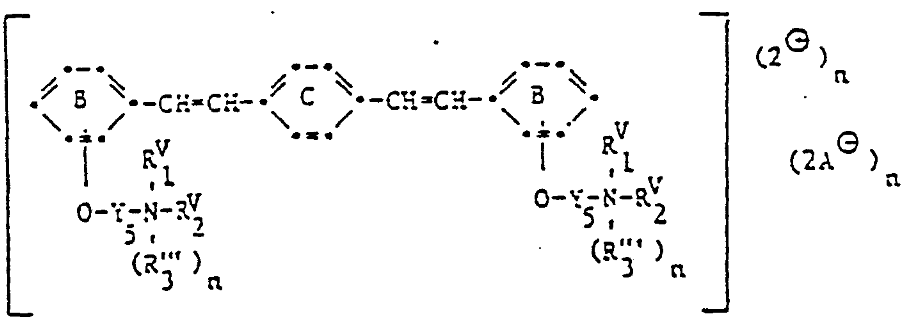

dans laquelle $Y_5$ est un radical alkylène en $C_{2-20}$, $R_1^V$ est un radical alkyle en $C_{1-8}$ ou alcényle en $C_{3-4}$, non-substitué ou substitué par un halogène un groupe cyano, hydroxyle, alcoxy, phényle ou alcoxycarbonyle ayant de 1 à 4 atomes de carbone, ou forme, avec $R_2^V$ et l'atome N auquel ils sont liés, un noyau hétérocyclique pentagonal à heptagonal, $R_2^V$ est un radical alkyle en $C_{1-8}$ alcényle en $C_{3\ 4}$,non-substitué ou substitué par un halogène, un groupe cyano, hydroxyle, alcoxy, phényle ou alcoxycarbonyle ayant de 1 à 4 atomes de carbone, ou forme, avec $R_1^V$ et l'atome N auquel ils sont liés, un noyau hétérocyclique pentagonal à heptagonal, $R'''_3$ est l'hydrogène ou un radical alkyle en $C_{1-4}$ non-substitué ou substitué, $A^{\ominus}$ est un anion incolore, et n est le nombre 0 ou 1, les noyaux benzéniques B et C pouvant aussi être substitués par des substituants non-chromophores.

9. Distyrylbenzène selon la revendication 8, de formule

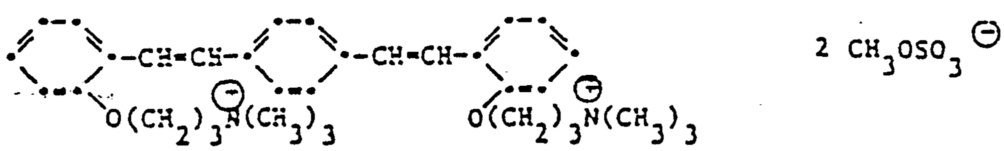

10. Distyrylbenzène selon la revendication 8, de formule

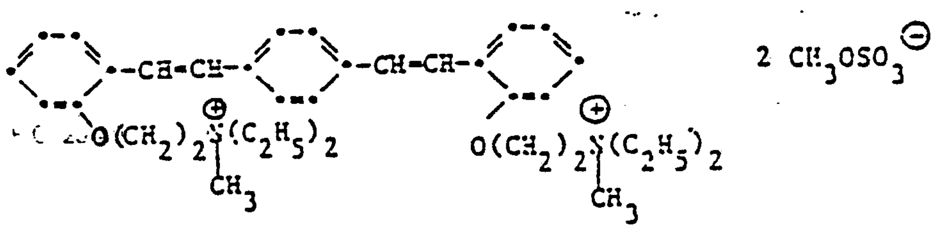

11. Distyrylbenzène selon la revendication 8, de formule

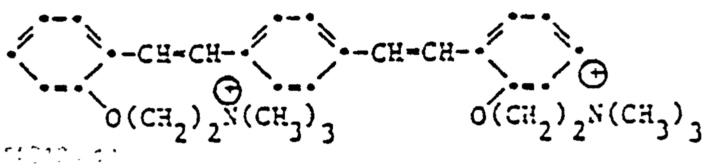

$2\ CH_3OSO_3^{\ominus}$

12. Procédé pour la préparation de distyrylbenzènes de formule

(1)

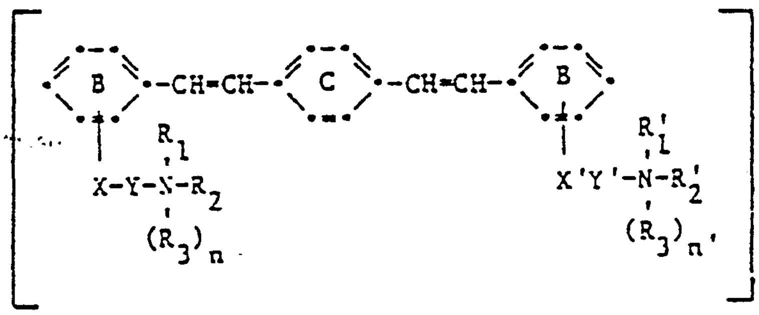

$(n+n')^{\ominus}$

$(n+n')A^{\ominus}$

dans laquelle X et X', indépendamment l'un de l'autre, sont chacun la liaison directe, l'oxygène, le soufre, un radical -O-(alkylène en $C_{1-3}$)-CON($R_4$)-, -O-(alkylène en $C_{1-3}$)-COO-ou -OCO-, Y et Y', indépendamment l'un de l'autre, sont chacun un radical alkylène en $C_{1-20}$ ou alkylène en $C_{1-20}$ substitué par un groupe hydroxyle, $R_1$ et $R_1'$, indépendamment l'un de l'autre, sont chacun un radical alkyle en $C_{1-8}$, ou alcényle en $C_{3-4}$,non-substitué ou substitué par un halogène, ou par un groupe cyano, hydroxyle, alcoxy, phényle ou alcoxycarbonyle ayant de 1 à 4 atomes de carbone, ou bien $R_1$ ou $R_1'$, respectivement avec $R_2$ et $R_2'$, et avec l'atome N auquel ils sont liés, forme un noyau hétérocyclique pentagonal à heptagonal, $R_2$ et $R_2'$, indépendamment l'un de l'autre, sont chacun un radical alkyle en $C_{1-8}$ ou alcényle en $C_{3-4}$, non-substitué ou substitué par un halogène ou par un groupe cyano, hydroxyle, alcoxy, phényle ou alcoxycarbonyle ayant de 1 à 4 atomes de carbone, ou bien $R_2$ ou $R_2'$, respectivement avec $R_1$ et $R_1'$ et avec l'atome N auquel ils sont liés, forme un noyau hétérocyclique pentagonal à heptagonal, $R_3$ est l'hydrogène, un radical alkyle en $C_{1-4}$ ou alcényle en $C_{3-4}$, non-substitué ou substitué, ou $R_1$, $R_2$ et $R_3$, avec l'atome N auquel ils sont liés, ou encore $R_1'$, $R_2'$ et $R_3$, avec l'atome N auquel ils sont liés, forment aussi le noyau pyridine ou picoline, $R_4$ est l'hydrogène ou un radical alkyle en $C_{1-6}$ non-substitué ou substitué par un groupe cyano, carbamoyle, carbalcoxy, hydroxyle, halogène ou alcoxy ayant de 1 à 4 atomes de carbone, A⊖ est un anion incolore,et n et n', indépendamment l'un de l'autre, sont chacun le nombre 0 ou 1, les noyaux benzéniques B et C pouvant aussi être substitués par des substituants non-chromophores, et où les groupements

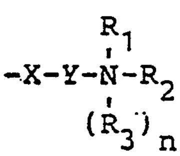

et

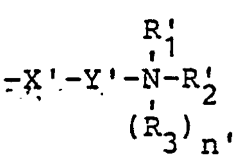

,

dans le cas où les noyaux B et C ne portent pas de substituants non-chromophores, peuvent aussi être tous les deux chacun

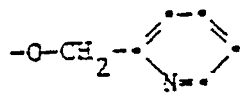

ou tous les deux chacun

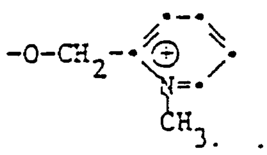

sur les positions ortho par rapport aux deux groupes -CH=CH-, caractérisé en ce qu'on quaternise ou protone un équivalent molaire d'un distyrylbenzène de formule

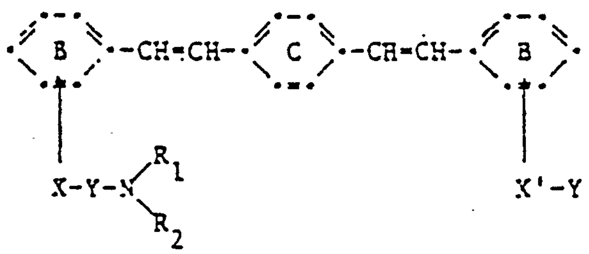

avec 1 ou 2 équivalents molaires d'un agent d'alkylation ou d'un acide de formule $R_3$-A où, dans ces formules, les noyaux benzéniques B et C et X, X', Y, Y', $R_1$, $R_{1'}$, $R_{2'}$, $R_{2'}$, $R_3$ et A ont les significations données ci-dessus.

13. Procédé pour la préparation de distyrylbenzènes de formule

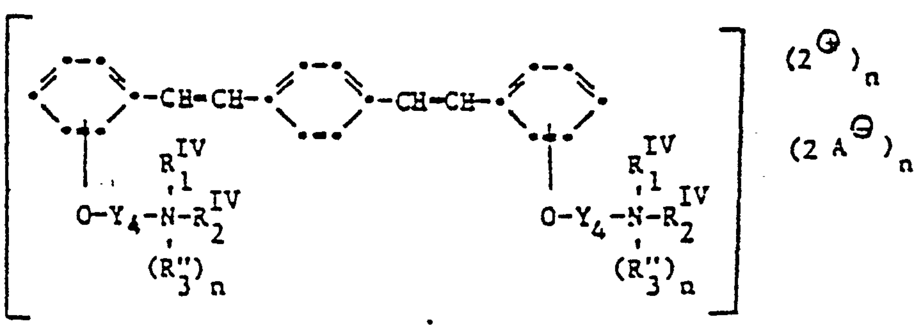

dans laquelle $Y_4$ est un radical alkylène en $C_{2\,4}$, $R_1^{IV}$ est un radical alkyle en $C_{1-3}$,ou bien $R_1^{IV}$ forme avec $R_2^{IV}$ et l'atome N auquel ils sont liés un noyau pyrrolidine, pipéridine, hexaméthylènimine ou morpholine, $R_2^{IV}$ est un radical alkyle en $C_{1-3}$, ou bien $R_2^{IV}$ forme avec $R_1^{I}$ et l'atome N auquel ils sont liés un noyau pyrrolidine, pipéridine, hexaméthylènimine ou morpholine, $R'''_3$ est l'hydrogène ou un radical alkyle en $C_{1-3}$, n est le nombre 0 ou 1, et A⊖ est un anion incolore, caractérisé en ce qu'on fait réagir selon un rapport moléculaire de 1:2 un composé de formule

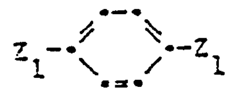

sur des composés de formule

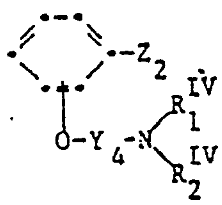

en présence d'une base forte, formules dans lesquelles $Y_4$, $R_1^{IV}$ et $R_2^{IV}$ ont les significations données ci-dessus et l'un des symboles $Z_1$ et $Z_2$ est un groupe OCH et l'autre est un groupement de formule

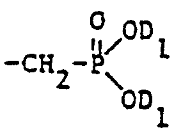 , 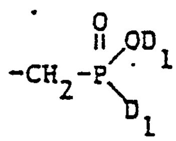 ,

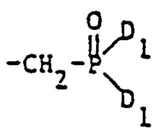 ou 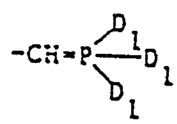

où $D_1$ est un radical alkyle, aryle, cycloalkyle ou aralkyle, substitué ou non-substitué, et en ce que, si on le souhaite, on quaternise ou protone le composé obtenu de formule

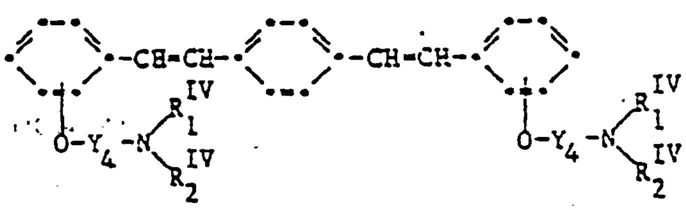

avec 2 équivalents molaires d'un agent d'alkylation ou d'un acide de formule $R'_3$'-A, formules dans lesquelles $Y_4$, $R_1^{IV}$, $R_2^{IV}$, $R''_3$ et A ont les significations données ci-dessus.

14. Utilisation des composés définis dans l'une des revendications 1 à 11 pour le blanchiment optique de matières organiques.

15. Utilisation selon la revendication 14, pour le blanchiment optique des matières organiques polyacrylonitrile et cellulose.

16. Détergent contenant un ou plusieurs composés ayant la formule I définie dans la revendication 1.

17. Détergent selon la revendication 16, qui contient 10 à 70 % en poids d'un tensioactif non-ionique et 1 à 30 % en poids d'un assouplissant textile cationique.

18. Détergent selon la revendication 17, qui contient 10 à 70 % en poids d'un tensioactif non-ionique et 1 à 30 % en poids d'un dérivé quaternaire de l'ammoniac et/ou de l'imidazoline, avec 2 radicaux aliphatiques à longue chaîne, saturés ou insaturés, en tant qu'assouplissants textiles cationiques, et encore, pour conférer la forme liquide, un solvant.

19. Détergent selon la revendication 18, qui contient en tant qu'assouplissant textile cationique un sel 1-méthyl-1-oléylamidoéthyl-2-oléyl-imidazolinium.X⊖, 1-méthyl-1-suif-amidoéthyl-2-suif-imidazolinium.X⊖, di-suifdiméthyl-ammonium.X⊖, ou un composé de formule

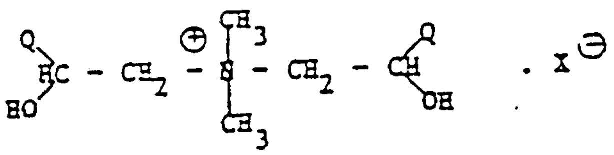

O étant un radical alkyle en $C_{14\text{-}16}$, et X⊖ étant un anion chlorure, bromure, méthylsulfate, éthylsulfate, méthanesulfonate, éthanesulfonate ou toluènesulfonate.

20. Produit de traitement de textiles contenant un ou plusieurs composés ayant la formule 1 définie dans la revendication 1.

21. Produit de post-traitement du linge blanc contenant un ou plusieurs composés ayant la formule 1 définie dans la revendication 1 et un assouplissant textile cationique.